# EUROPEAN PATENT SPECIFICATION

(11) **EP 3 126 837 B1**
(45) Date of publication and mention of the grant of the patent: **15.09.2021**
(21) Application number: 15774062.2
(22) Date of filing: 20.03.2015
(51) Int. Cl.: G01N 33/68

(54) **PEPTIDES AND METHODS FOR DETECTING FOOD ALLERGY**
PEPTIDE UND VERFAHREN FÜR DEN NACHWEIS VON LEBENSMITTELALLERGIEN
PEPTIDES ET MÉTHODES PERMETTANT DE DÉTECTER UNE ALLERGIE ALIMENTAIRE

(30) Priority: 03.04.2014 US 201461974675 P
(43) Date of publication of application: 08.02.2017
(73) Proprietor: AllerGenis, LLC, Hatfield, PA 19440 (US); Icahn School of Medicine at Mount Sinai, New York, NY 10029 (US)
(72) Inventor: GETTS, Robert C., Collegeville, Pennsylvania 19426 (US); KADUSHIN, James, Gilbertsville, Pennsylvania 19525 (US); SAMPSON, Hugh A., Greenwich, Connecticut 06831 (US); BARDINA, Luda, Red Hook, New York 12571 (US); GRISHINA, Galina, Tarrytown, New York 10591 (US); GIMENEZ, Gustavo, New York, NY 10009 (US); LIN, Jing, Guangzhou 510275 (CN)
(74) Representative: FRKelly
(86) International application number: PCT/US2015/021715
(87) International publication number: WO 2015/153151

(56) References cited:
- EP-A1- 3 054 300
- WO-A1-99/39211
- WO-A1-2010/052939
- WO-A1-2010/110454
- WO-A1-2015/015043
- WO-A2-2005/100995
- US-A1- 2011 071 043
- SHREFFLER WAYNE G ET AL: "Microarray immunoassay: Association of clinical history, in vitro IgE function, and heterogeneity of allergenic peanut epitopes", JOURNAL OF ALLERGY AND CLINICAL IMMUNOLOGY, ELSEVIER, AMSTERDAM, NL, vol. 113, no. 4, 17 April 2004 (2004-04-17), pages 776-782, XP002449104, ISSN: 0091-6749, DOI: 10.1016/J.JACI.2003.12.588
- FLINTERMAN ET AL: "Peanut epitopes for IgE and IgG4 in peanut-sensitized children in relation to severity of peanut allergy", JOURNAL OF ALLERGY AND CLINICAL IMMUNOLOGY, ELSEVIER, AMSTERDAM, NL, vol. 121, no. 3, 30 January 2008 (2008-01-30), pages 737-743.e10, XP022506340, ISSN: 0091-6749, DOI: 10.1016/J.JACI.2007.11.039
- MATSUMOTO ET AL.: 'Peptide array-based analysis of the specific IgE and IgG4 in cow's milk allergens and its use in allergy evaluation' PEPTIDES vol. 30, no. 10, 2009, pages 1840 - 1847, XP026653308
- POMPONI ET AL.: 'Allergen micro-bead array for IgE detection: a feasibility study using allergenic molecules tested on a flexible multiplex flow cytometric immunoassay' PLOS ONE vol. 7, no. 4, 2012, pages 1 - 16, XP055033628
- LIN ET AL.: 'Development of a novel peptide microarray for large-scale epitope mapping of food allergens' JOURNAL OF ALLERGY AND CLINICAL IMMUNOLOGY vol. 124, no. 2, 2009, pages 315 - 322, XP026390934
- WANG ET AL.: 'Correlation of IgE/IgG4 milk epitopes and affinity of milk-specific IgE antibodies with different phenotypes of clinical milk allergy' JOURNAL OF ALLERGY AND CLINICAL IMMUNOLOGY vol. 125, no. 3, 2010, pages 695 - 702, XP026942667

### SEQUENCE LISTING

<110> Genisphere, LLC
   ICAHN School of Medicine at Mount Sinai
   Getts, Robert C.
   Kadushin, James
   Sampson, Hugh A.
   Bardina, Luda
   Grishina, Galina
   Gimenez, Gustavo
   Lin, Jing
<120> Peptides, Reagents and Methods for Detecting Food Allergy
<130> DSC0056-00WO
<160> 683
<170> PatentIn version 3.5
<210> 1
   <211> 20
   <212> PRT
   <213> Cow's milk peptide
<400> 1
   Lys His Gln Gly Leu Pro Gln Glu Val Leu Asn Glu Asn Leu Leu Arg
   1               5                   10                  15
   Phe Phe Val Ala
               20
<210> 2
   <211> 20
   <212> PRT
   <213> Cow's milk peptide
<400> 2
   Val Ala Pro Phe Pro Glu Val Phe Gly Lys Glu Lys Val Asn Glu Leu
   1               5                   10                  15
   Ser Lys Asp Ile
               20
<210> 3
   <211> 20
   <212> PRT
   <213> Cow's milk peptide
<400> 3
   Ser Ile Ser Ser Ser Glu Glu Ile Val Pro Asn Ser Val Glu Gln Lys
   1               5                   10                  15
   His Ile Gln Lys
               20
<210> 4
   <211> 20
   <212> PRT
   <213> Cow's milk peptide
<400> 4
   Lys His Ile Gln Lys Glu Asp Val Pro Ser Glu Arg Tyr Leu Gly Tyr
   1               5                   10                  15
   Leu Glu Gln Leu
               20
<210> 5
   <211> 20
   <212> PRT
   <213> Cow's milk peptide
<400> 5
   Ser Glu Arg Tyr Leu Gly Tyr Leu Glu Gln Leu Leu Arg Leu Lys Lys
   1               5                   10                  15
   Tyr Lys Val Pro
               20
<210> 6
   <211> 20
   <212> PRT
   <213> Cow's milk peptide
<400> 6
   Lys Tyr Lys Val Pro Gln Leu Glu Ile Val Pro Asn Ser Ala Glu Glu
   1 5 10 15
   Arg Leu His Ser
   20
<210> 7
   <211> 20
   <212> PRT
   <213> Cow's milk peptide
<400> 7
   Gln Gln Lys Glu Pro Met Ile Gly Val Asn Gln Glu Leu Ala Tyr Phe
   1 5 10 15
   Tyr Pro Glu Leu
   20
<210> 8
   <211> 20
   <212> PRT
   <213> Cow's milk peptide
<400> 8
   Leu Gly Thr Gln Tyr Thr Asp Ala Pro Ser Phe Ser Asp Ile Pro Asn
   1 5 10 15
   Pro Ile Gly Ser
   20
<210> 9
   <211> 20
   <212> PRT
   <213> Cow's milk peptide
<400> 9
   Ser Asp Ile Pro Asn Pro Ile Gly Ser Glu Asn Ser Glu Lys Thr Thr
   1 5 10 15
   Met Pro Leu Trp
   20
<210> 10
   <211> 20
   <212> PRT
   <213> Cow's milk peptide
<400> 10
   Gln Glu Lys Asn Met Ala Ile Asn Pro Ser Lys Glu Asn Leu Cys Ser
   1               5                   10                  15
   Thr Phe Cys Lys
               20
<210> 11
   <211> 20
   <212> PRT
   <213> Cow's milk peptide
<400> 11
   Ser Thr Phe Cys Lys Glu Val Val Arg Asn Ala Asn Glu Glu Glu Tyr
   1               5                   10                  15
   Ser Ile Gly Ser
               20
<210> 12
   <211> 20
   <212> PRT
   <213> Cow's milk peptide
<400> 12
   Lys His Tyr Gln Lys Ala Leu Asn Glu Ile Asn Gln Phe Tyr Gln Lys
   1               5                   10                  15
   Phe Pro Gln Tyr
               20
<210> 13
   <211> 20
   <212> PRT
   <213> Cow's milk peptide
<400> 13
   Gln Tyr Leu Tyr Gln Gly Pro Ile Val Leu Asn Pro Trp Asp Gln Val
   1               5                   10                  15
   Lys Arg Asn Ala
               20
<210> 14
   <211> 20
   <212> PRT
   <213> Cow's milk peptide
<400> 14
   Lys Ile Ser Gln Arg Tyr Gln Lys Phe Ala Leu Pro Gln Tyr Leu Lys
   1               5                   10                  15
   Thr Val Tyr Gln
               20
<210> 15
   <211> 20
   <212> PRT
   <213> Cow's milk peptide
<400> 15
   Gln Tyr Leu Lys Thr Val Tyr Gln His Gln Lys Ala Met Lys Pro Trp
   1               5                   10                  15
   Ile Gln Pro Lys
               20
<210> 16
   <211> 20
   <212> PRT
   <213> Cow's milk peptide
<400> 16
   Arg Glu Leu Glu Glu Leu Asn Val Pro Gly Glu Ile Val Glu Ser Leu
   1               5                   10                  15
   Ser Ser Ser Glu
               20
<210> 17
   <211> 20
   <212> PRT
   <213> Cow's milk peptide
<400> 17
   Gln Asp Lys Ile His Pro Phe Ala Gln Thr Gln Ser Leu Val Tyr Pro
   1               5                   10                  15
   Phe Pro Gly Pro
               20
<210> 18
   <211> 20
   <212> PRT
   <213> Cow's milk peptide
<400> 18
   Phe Ala Gln Thr Gln Ser Leu Val Tyr Pro Phe Pro Gly Pro Ile Pro
   1 5 10 15
   Asn Ser Leu Pro
   20
<210> 19
   <211> 20
   <212> PRT
   <213> Cow's milk peptide
<400> 19
   Asn Ile Pro Pro Leu Thr Gln Thr Pro Val Val Val Pro Pro Phe Leu
   1 5 10 15
   Gln Pro Glu Val
   20
<210> 20
   <211> 20
   <212> PRT
   <213> Cow's milk peptide
<400> 20
   Lys Val Lys Glu Ala Met Ala Pro Lys His Lys Glu Met Pro Phe Pro
   1 5 10 15
   Lys Tyr Pro Val
   20
<210> 21
   <211> 20
   <212> PRT
   <213> Cow's milk peptide
<400> 21
   Ser Leu Thr Leu Thr Asp Val Glu Asn Leu His Leu Pro Leu Pro Leu
   1 5 10 15
   Leu Gln Ser Trp
   20
<210> 22
   <211> 20
   <212> PRT
   <213> Cow's milk peptide
<400> 22
   Phe Pro Pro Gln Ser Val Leu Ser Leu Ser Gln Ser Lys Val Leu Pro
   1 5 10 15
   Val Pro Gln Lys
   20
<210> 23
   <211> 20
   <212> PRT
   <213> Cow's milk peptide
<400> 23
   Pro Val Pro Gln Lys Ala Val Pro Tyr Pro Gln Arg Asp Met Pro Ile
   1 5 10 15
   Gln Ala Phe Leu
   20
<210> 24
   <211> 20
   <212> PRT
   <213> Cow's milk peptide
<400> 24
   Arg Val Tyr Val Glu Glu Leu Lys Pro Thr Pro Glu Gly Asp Leu Glu
   1 5 10 15
   Ile Leu Leu Gln
   20
<210> 25
   <211> 20
   <212> PRT
   <213> Cow's milk peptide
<400> 25
   Asp Glu Cys Ala Gln Lys Lys Ile Ile Ala Glu Lys Thr Lys Ile Pro
   1 5 10 15
   Ala Val Phe Lys
   20
<210> 26
   <211> 20
   <212> PRT
   <213> Cow's milk peptide
<400> 26
   Cys Leu Val Arg Thr Pro Glu Val Asp Asp Glu Ala Leu Glu Lys Phe
   1 5 10 15
   Asp Lys Ala Leu
   20
<210> 27
   <211> 20
   <212> PRT
   <213> Cow's milk peptide
<400> 27
   Glu Val Asp Asp Glu Ala Leu Glu Lys Phe Asp Lys Ala Leu Lys Ala
   1 5 10 15
   Leu Pro Met His
   20
<210> 28
   <211> 20
   <212> PRT
   <213> Cow's milk peptide
<400> 28
   Arg Cys Glu Lys Asp Glu Arg Phe Phe Ser Asp Lys Ile Ala Lys Tyr
   1 5 10 15
   Ile Pro Ile Gln
   20
<210> 29
   <211> 20
   <212> PRT
   <213> Cow's milk peptide
<400> 29
   Lys Pro Val Ala Leu Ile Asn Asn Gln Phe Leu Pro Tyr Pro Tyr Tyr
   1 5 10 15
   Ala Lys Pro Ala
   20
<210> 30
   <211> 20
   <212> PRT
   <213> Cow's milk peptide
<400> 30
   Met Ala Ile Pro Pro Lys Lys Asn Gln Asp Lys Thr Glu Ile Pro Thr
   1               5                   10                  15
   Ile Asn Thr Ile
               20
<210> 31
   <211> 20
   <212> PRT
   <213> Cow's milk peptide
<400> 31
   Pro Thr Ser Thr Pro Thr Thr Glu Ala Val Glu Ser Thr Val Ala Thr
   1               5                   10                  15
   Leu Glu Asp Ser
               20
<210> 32
   <211> 20
   <212> PRT
   <213> Cow's milk peptide
<400> 32
   Thr Leu Glu Asp Ser Pro Glu Val Ile Glu Ser Pro Pro Glu Ile Asn
   1               5                   10                  15
   Thr Val Gln Val
               20
<210> 33
   <211> 20
   <212> PRT
   <213> Cow's milk peptide
<400> 33
   Tyr Ala Lys Pro Ala Ala Val Arg Ser Pro Ala Gln Ile Leu Gln Trp
   1               5                   10                  15
   Gln Val Leu Ser
               20
<210> 34
   <211> 20
   <212> PRT
   <213> Cow's milk peptide
<400> 34
   Asn Arg Glu Gln Leu Ser Thr Ser Glu Glu Asn Ser Lys Lys Thr Val
   1               5                   10                  15
   Asp Met Glu Ser
               20
<210> 35
   <211> 20
   <212> PRT
   <213> Cow's milk peptide
<400> 35
   Arg Ile Asn Lys Lys Ile Glu Lys Phe Gln Ser Glu Glu Gln Gln Gln
   1               5                   10                  15
   Thr Glu Asp Glu
               20
<210> 36
   <211> 20
   <212> PRT
   <213> Cow's milk peptide
<400> 36
   Lys Val Leu Val Leu Asp Thr Asp Tyr Lys Lys Tyr Leu Leu Val Cys
   1               5                   10                  15
   Met Glu Asn Ser
               20
<210> 37
   <211> 20
   <212> PRT
   <213> Cow's milk peptide
<400> 37
   Asn Leu Leu Arg Phe Phe Val Ala Pro Phe Pro Glu Val Phe Gly Lys
   1               5                   10                  15
   Glu Lys Val Asn
               20
<210> 38
   <211> 20
   <212> PRT
   <213> Cow's milk peptide
<400> 38
   Pro Asn Ser Val Glu Gln Lys His Ile Gln Lys Glu Asp Val Pro Ser
   1               5                   10                  15
   Glu Arg Tyr Leu
               20
<210> 39
   <211> 20
   <212> PRT
   <213> Cow's milk peptide
<400> 39
   Gln Lys Glu Asp Val Pro Ser Glu Arg Tyr Leu Gly Tyr Leu Glu Gln
   1               5                   10                  15
   Leu Leu Arg Leu
               20
<210> 40
   <211> 20
   <212> PRT
   <213> Cow's milk peptide
<400> 40
   Leu Glu Ile Val Pro Asn Ser Ala Glu Glu Arg Leu His Ser Met Lys
   1               5                   10                  15
   Glu Gly Ile His
               20
<210> 41
   <211> 20
   <212> PRT
   <213> Cow's milk peptide
<400> 41
   Glu Arg Leu His Ser Met Lys Glu Gly Ile His Ala Gln Gln Lys Glu
   1               5                   10                  15
   Pro Met Ile Gly
               20
<210> 42
   <211> 20
   <212> PRT
   <213> Cow's milk peptide
<400> 42
   Ile His Ala Gln Gln Lys Glu Pro Met Ile Gly Val Asn Gln Glu Leu
   1               5                   10                  15
   Ala Tyr Phe Tyr
               20
<210> 43
   <211> 20
   <212> PRT
   <213> Cow's milk peptide
<400> 43
   Ile Gly Val Asn Gln Glu Leu Ala Tyr Phe Tyr Pro Glu Leu Phe Arg
   1               5                   10                  15
   Gln Phe Tyr Gln
               20
<210> 44
   <211> 20
   <212> PRT
   <213> Cow's milk peptide
<400> 44
   Pro Ser Gly Ala Trp Tyr Tyr Val Pro Leu Gly Thr Gln Tyr Thr Asp
   1               5                   10                  15
   Ala Pro Ser Phe
               20
<210> 45
   <211> 20
   <212> PRT
   <213> Cow's milk peptide
<400> 45
   Ala Trp Tyr Tyr Val Pro Leu Gly Thr Gln Tyr Thr Asp Ala Pro Ser
   1               5                   10                  15
   Phe Ser Asp Ile
               20
<210> 46
   <211> 20
   <212> PRT
   <213> Cow's milk peptide
<400> 46
   Ser Ile Ile Ser Gln Glu Thr Tyr Lys Gln Glu Lys Asn Met Ala Ile
   1               5                   10                  15
   Asn Pro Ser Lys
               20
<210> 47
   <211> 20
   <212> PRT
   <213> Cow's milk peptide
<400> 47
   Ile Asn Pro Ser Lys Glu Asn Leu Cys Ser Thr Phe Cys Lys Glu Val
   1               5                   10                  15
   Val Arg Asn Ala
               20
<210> 48
   <211> 20
   <212> PRT
   <213> Cow's milk peptide
<400> 48
   Ser Ala Glu Val Ala Thr Glu Glu Val Lys Ile Thr Val Asp Asp Lys
   1               5                   10                  15
   His Tyr Gln Lys
               20
<210> 49
   <211> 20
   <212> PRT
   <213> Cow's milk peptide
<400> 49
   Thr Ser Glu Glu Asn Ser Lys Lys Thr Val Asp Met Glu Ser Thr Glu
   1               5                   10                  15
   Val Phe Thr Lys
               20
<210> 50
   <211> 20
   <212> PRT
   <213> Cow's milk peptide
<400> 50
   Thr Lys Leu Thr Glu Glu Glu Lys Asn Arg Leu Asn Phe Leu Lys Lys
   1 5 10 15
   Ile Ser Gln Arg
   20
<210> 51
   <211> 20
   <212> PRT
   <213> Cow's milk peptide
<400> 51
   Tyr Gln His Gln Lys Ala Met Lys Pro Trp Ile Gln Pro Lys Thr Lys
   1 5 10 15
   Val Ile Pro Tyr
   20
<210> 52
   <211> 20
   <212> PRT
   <213> Cow's milk peptide
<400> 52
   Pro Phe Pro Gly Pro Ile Pro Asn Ser Leu Pro Gln Asn Ile Pro Pro
   1 5 10 15
   Leu Thr Gln Thr
   20
<210> 53
   <211> 20
   <212> PRT
   <213> Cow's milk peptide
<400> 53
   Gln Thr Pro Val Val Val Pro Pro Phe Leu Gln Pro Glu Val Met Gly
   1 5 10 15
   Val Ser Lys Val
   20
<210> 54
   <211> 20
   <212> PRT
   <213> Cow's milk peptide
<400> 54
   Val Glu Asn Leu His Leu Pro Leu Pro Leu Leu Gln Ser Trp Met His
   1 5 10 15
   Gln Pro His Gln
   20
<210> 55
   <211> 20
   <212> PRT
   <213> Cow's milk peptide
<400> 55
   Ser Trp Met His Gln Pro His Gln Pro Leu Pro Pro Thr Val Met Phe
   1 5 10 15
   Pro Pro Gln Ser
   20
<210> 56
   <211> 20
   <212> PRT
   <213> Cow's milk peptide
<400> 56
   Ser Gln Ser Lys Val Leu Pro Val Pro Gln Lys Ala Val Pro Tyr Pro
   1 5 10 15
   Gln Arg Asp Met
   20
<210> 57
   <211> 20
   <212> PRT
   <213> Cow's milk peptide
<400> 57
   Gly Asp Leu Glu Ile Leu Leu Gln Lys Trp Glu Asn Asp Glu Cys Ala
   1 5 10 15
   Gln Lys Lys Ile
   20
<210> 58
   <211> 20
   <212> PRT
   <213> Cow's milk peptide
<400> 58
   Asp Glu Ala Leu Glu Lys Phe Asp Lys Ala Leu Lys Ala Leu Pro Met
   1 5 10 15
   His Ile Arg Leu
   20
<210> 59
   <211> 20
   <212> PRT
   <213> Cow's milk peptide
<400> 59
   Arg Phe Phe Ser Asp Lys Ile Ala Lys Tyr Ile Pro Ile Gln Tyr Val
   1 5 10 15
   Leu Ser Arg Tyr
   20
<210> 60
   <211> 20
   <212> PRT
   <213> Cow's milk peptide
<400> 60
   Lys Pro Val Ala Leu Ile Asn Asn Gln Phe Leu Pro Tyr Pro Tyr Tyr
   1 5 10 15
   Ala Lys Pro Ala
   20
<210> 61
   <211> 20
   <212> PRT
   <213> Cow's milk peptide
<400> 61
   Tyr Ala Lys Pro Ala Ala Val Arg Ser Pro Ala Gln Ile Leu Gln Trp
   1 5 10 15
   Gln Val Leu Ser
   20
<210> 62
   <211> 20
   <212> PRT
   <213> Cow's milk peptide
<400> 62
   Pro Ala Gln Ile Leu Gln Trp Gln Val Leu Ser Asn Thr Val Pro Ala
   1 5 10 15
   Lys Ser Cys Gln
   20
<210> 63
   <211> 20
   <212> PRT
   <213> Cow's milk peptide
<400> 63
   Cys Gln Ala Gln Pro Thr Thr Met Ala Arg His Pro His Pro His Leu
   1 5 10 15
   Ser Phe Met Ala
   20
<210> 64
   <211> 20
   <212> PRT
   <213> Cow's milk peptide
<400> 64
   Arg His Pro His Pro His Leu Ser Phe Met Ala Ile Pro Pro Lys Lys
   1 5 10 15
   Asn Gln Asp Lys
   20
<210> 65
   <211> 20
   <212> PRT
   <213> Cow's milk peptide
<400> 65
   Thr Ile Asn Thr Ile Ala Ser Gly Glu Pro Thr Ser Thr Pro Thr Thr
   1 5 10 15
   Glu Ala Val Glu
   20
<210> 66
   <211> 20
   <212> PRT
   <213> Cow's milk peptide
<400> 66
   Asp Ser Pro Glu Val Ile Glu Ser Pro Pro Glu Ile Asn Thr Val Gln
   1 5 10 15
   Val Thr Ser Thr
   20
<210> 67
   <211> 20
   <212> PRT
   <213> Cow's milk peptide
<400> 67
   Pro Glu Val Ile Glu Ser Pro Pro Glu Ile Asn Thr Val Gln Val Thr
   1               5                   10                  15
   Ser Thr Ala Val
               20
<210> 68
   <211> 20
   <212> PRT
   <213> Cow's milk peptide
<400> 68
   Gln Ser Leu Val Cys Gln Cys Leu Val Arg Thr Pro Glu Val Asp Asp
   1               5                   10                  15
   Glu Ala Leu Glu
               20
<210> 69
   <211> 15
   <212> PRT
   <213> Peanut peptide
<400> 69
   Val Leu Ala Ser Val Ser Ala Thr His Ala Lys Ser Ser Pro Tyr
   1               5                   10                  15
<210> 70
   <211> 15
   <212> PRT
   <213> Peanut peptide
<400> 70
   Ser Val Ser Ala Thr His Ala Lys Ser Ser Pro Tyr Gln Lys Lys
   1               5                   10                  15
<210> 71
   <211> 15
   <212> PRT
   <213> Peanut peptide
<400> 71
   Ala Thr His Ala Lys Ser Ser Pro Tyr Gln Lys Lys Thr Glu Asn
   1               5                   10                  15
<210> 72
   <211> 15
   <212> PRT
   <213> Peanut peptide
<400> 72
   Thr Glu Asn Pro Cys Ala Gln Arg Cys Leu Gln Ser Cys Gln Gln
   1 5 10 15
<210> 73
   <211> 15
   <212> PRT
   <213> Peanut peptide
<400> 73
   Pro Cys Ala Gln Arg Cys Leu Gln Ser Cys Gln Gln Glu Pro Asp
   1 5 10 15
<210> 74
   <211> 15
   <212> PRT
   <213> Peanut peptide
<400> 74
   Leu Gln Ser Cys Gln Gln Glu Pro Asp Asp Leu Lys Gln Lys Ala
   1 5 10 15
<210> 75
   <211> 15
   <212> PRT
   <213> Peanut peptide
<400> 75
   Cys Gln Gln Glu Pro Asp Asp Leu Lys Gln Lys Ala Cys Glu Ser
   1 5 10 15
<210> 76
   <211> 15
   <212> PRT
   <213> Peanut peptide
<400> 76
   Glu Pro Asp Asp Leu Lys Gln Lys Ala Cys Glu Ser Arg Cys Thr
   1 5 10 15
<210> 77
   <211> 15
   <212> PRT
   <213> Peanut peptide
<400> 77
   Gln Lys Ala Cys Glu Ser Arg Cys Thr Lys Leu Glu Tyr Asp Pro
   1 5 10 15
<210> 78
   <211> 15
   <212> PRT
   <213> Peanut peptide
<400> 78
   Cys Glu Ser Arg Cys Thr Lys Leu Glu Tyr Asp Pro Arg Cys Val
   1 5 10 15
<210> 79
   <211> 15
   <212> PRT
   <213> Peanut peptide
<400> 79
   Arg Cys Thr Lys Leu Glu Tyr Asp Pro Arg Cys Val Tyr Asp Pro
   1 5 10 15
<210> 80
   <211> 15
   <212> PRT
   <213> Peanut peptide
<400> 80
   Lys Leu Glu Tyr Asp Pro Arg Cys Val Tyr Asp Pro Arg Gly His
   1 5 10 15
<210> 81
   <211> 15
   <212> PRT
   <213> Peanut peptide
<400> 81
   Tyr Asp Pro Arg Cys Val Tyr Asp Pro Arg Gly His Thr Gly Thr
   1 5 10 15
<210> 82
   <211> 15
   <212> PRT
   <213> Peanut peptide
<400> 82
   Tyr Asp Pro Arg Gly His Thr Gly Thr Thr Asn Gln Arg Ser Pro
   1 5 10 15
<210> 83
   <211> 15
   <212> PRT
   <213> Peanut peptide
<400> 83
   Arg Gly His Thr Gly Thr Thr Asn Gln Arg Ser Pro Pro Gly Glu
   1               5                   10                  15
<210> 84
   <211> 15
   <212> PRT
   <213> Peanut peptide
<400> 84
   Thr Asn Gln Arg Ser Pro Pro Gly Glu Arg Thr Arg Gly Arg Gln
   1               5                   10                  15
<210> 85
   <211> 15
   <212> PRT
   <213> Peanut peptide
<400> 85
   Arg Ser Pro Pro Gly Glu Arg Thr Arg Gly Arg Gln Pro Gly Asp
   1               5                   10                  15
<210> 86
   <211> 15
   <212> PRT
   <213> Peanut peptide
<400> 86
   Pro Gly Glu Arg Thr Arg Gly Arg Gln Pro Gly Asp Tyr Asp Asp
   1               5                   10                  15
<210> 87
   <211> 15
   <212> PRT
   <213> Peanut peptide
<400> 87
   Arg Thr Arg Gly Arg Gln Pro Gly Asp Tyr Asp Asp Asp Arg Arg
   1               5                   10                  15
<210> 88
   <211> 15
   <212> PRT
   <213> Peanut peptide
<400> 88
   Gly Arg Gln Pro Gly Asp Tyr Asp Asp Asp Arg Arg Gln Pro Arg
   1               5                   10                  15
<210> 89
   <211> 15
   <212> PRT
   <213> Peanut peptide
<400> 89
   Pro Gly Asp Tyr Asp Asp Asp Arg Arg Gln Pro Arg Arg Glu Glu
   1               5                   10                  15
<210> 90
   <211> 15
   <212> PRT
   <213> Peanut peptide
<400> 90
   Tyr Asp Asp Asp Arg Arg Gln Pro Arg Arg Glu Glu Gly Gly Arg
   1               5                   10                  15
<210> 91
   <211> 15
   <212> PRT
   <213> Peanut peptide
<400> 91
   Asp Arg Arg Gln Pro Arg Arg Glu Glu Gly Gly Arg Trp Gly Pro
   1               5                   10                  15
<210> 92
   <211> 15
   <212> PRT
   <213> Peanut peptide
<400> 92
   Gln Pro Arg Arg Glu Glu Gly Gly Arg Trp Gly Pro Ala Gly Pro
   1               5                   10                  15
<210> 93
   <211> 15
   <212> PRT
   <213> Peanut peptide
<400> 93
   Arg Glu Glu Gly Gly Arg Trp Gly Pro Ala Gly Pro Arg Glu Arg
   1               5                   10                  15
<210> 94
   <211> 15
   <212> PRT
   <213> Peanut peptide
<400> 94
   Gly Gly Arg Trp Gly Pro Ala Gly Pro Arg Glu Arg Glu Arg Glu
   1 5 10 15
<210> 95
   <211> 15
   <212> PRT
   <213> Peanut peptide
<400> 95
   Trp Gly Pro Ala Gly Pro Arg Glu Arg Glu Arg Glu Glu Asp Trp
   1 5 10 15
<210> 96
   <211> 15
   <212> PRT
   <213> Peanut peptide
<400> 96
   Ala Gly Pro Arg Glu Arg Glu Arg Glu Glu Asp Trp Arg Gln Pro
   1 5 10 15
<210> 97
   <211> 15
   <212> PRT
   <213> Peanut peptide
<400> 97
   Arg Glu Arg Glu Arg Glu Glu Asp Trp Arg Gln Pro Arg Glu Asp
   1 5 10 15
<210> 98
   <211> 15
   <212> PRT
   <213> Peanut peptide
<400> 98
   Glu Arg Glu Glu Asp Trp Arg Gln Pro Arg Glu Asp Trp Arg Arg
   1 5 10 15
<210> 99
   <211> 15
   <212> PRT
   <213> Peanut peptide
<400> 99
   Glu Asp Trp Arg Gln Pro Arg Glu Asp Trp Arg Arg Pro Ser His
   1 5 10 15
<210> 100
   <211> 15
   <212> PRT
   <213> Peanut peptide
<400> 100
   Arg Gln Pro Arg Glu Asp Trp Arg Arg Pro Ser His Gln Gln Pro
   1 5 10 15
<210> 101
   <211> 15
   <212> PRT
   <213> Peanut peptide
<400> 101
   Arg Glu Asp Trp Arg Arg Pro Ser His Gln Gln Pro Arg Lys Ile
   1 5 10 15
<210> 102
   <211> 15
   <212> PRT
   <213> Peanut peptide
<400> 102
   Trp Arg Arg Pro Ser His Gln Gln Pro Arg Lys Ile Arg Pro Glu
   1 5 10 15
<210> 103
   <211> 15
   <212> PRT
   <213> Peanut peptide
<400> 103
   Pro Ser His Gln Gln Pro Arg Lys Ile Arg Pro Glu Gly Arg Glu
   1 5 10 15
<210> 104
   <211> 15
   <212> PRT
   <213> Peanut peptide
<400> 104
   Gln Gln Pro Arg Lys Ile Arg Pro Glu Gly Arg Glu Gly Glu Gln
   1 5 10 15
<210> 105
   <211> 15
   <212> PRT
   <213> Peanut peptide
<400> 105
   Arg Lys Ile Arg Pro Glu Gly Arg Glu Gly Glu Gln Glu Trp Gly
   1 5 10 15
<210> 106
   <211> 15
   <212> PRT
   <213> Peanut peptide
<400> 106
   Arg Pro Glu Gly Arg Glu Gly Glu Gln Glu Trp Gly Thr Pro Gly
   1 5 10 15
<210> 107
   <211> 15
   <212> PRT
   <213> Peanut peptide
<400> 107
   Gly Arg Glu Gly Glu Gln Glu Trp Gly Thr Pro Gly Ser His Val
   1 5 10 15
<210> 108
   <211> 15
   <212> PRT
   <213> Peanut peptide
<400> 108
   Gly Glu Gln Glu Trp Gly Thr Pro Gly Ser His Val Arg Glu Glu
   1 5 10 15
<210> 109
   <211> 15
   <212> PRT
   <213> Peanut peptide
<400> 109
   Glu Trp Gly Thr Pro Gly Ser His Val Arg Glu Glu Thr Ser Arg
   1 5 10 15
<210> 110
   <211> 15
   <212> PRT
   <213> Peanut peptide
<400> 110
   Arg Glu Glu Thr Ser Arg Asn Asn Pro Phe Tyr Phe Pro Ser Arg
   1 5 10 15
<210> 111
   <211> 15
   <212> PRT
   <213> Peanut peptide
<400> 111
   Thr Ser Arg Asn Asn Pro Phe Tyr Phe Pro Ser Arg Arg Phe Ser
   1 5 10 15
<210> 112
   <211> 15
   <212> PRT
   <213> Peanut peptide
<400> 112
   Asn Asn Pro Phe Tyr Phe Pro Ser Arg Arg Phe Ser Thr Arg Tyr
   1               5                   10                  15
<210> 113
   <211> 15
   <212> PRT
   <213> Peanut peptide
<400> 113
   Arg Ile Pro Ser Gly Phe Ile Ser Tyr Ile Leu Asn Arg His Asp
   1               5                   10                  15
<210> 114
   <211> 15
   <212> PRT
   <213> Peanut peptide
<400> 114
   Ser Gly Phe Ile Ser Tyr Ile Leu Asn Arg His Asp Asn Gln Asn
   1               5                   10                  15
<210> 115
   <211> 15
   <212> PRT
   <213> Peanut peptide
<400> 115
   Asn Gln Asn Leu Arg Val Ala Lys Ile Ser Met Pro Val Asn Thr
   1               5                   10                  15
<210> 116
   <211> 15
   <212> PRT
   <213> Peanut peptide
<400> 116
   Leu Arg Val Ala Lys Ile Ser Met Pro Val Asn Thr Pro Gly Gln
   1               5                   10                  15
<210> 117
   <211> 15
   <212> PRT
   <213> Peanut peptide
<400> 117
   Ala Lys Ile Ser Met Pro Val Asn Thr Pro Gly Gln Phe Glu Asp
   1               5                   10                  15
<210> 118
   <211> 15
   <212> PRT
   <213> Peanut peptide
<400> 118
   Ser Met Pro Val Asn Thr Pro Gly Gln Phe Glu Asp Phe Phe Pro
   1 5 10 15
<210> 119
   <211> 15
   <212> PRT
   <213> Peanut peptide
<400> 119
   Val Asn Thr Pro Gly Gln Phe Glu Asp Phe Phe Pro Ala Ser Ser
   1 5 10 15
<210> 120
   <211> 15
   <212> PRT
   <213> Peanut peptide
<400> 120
   Pro Gly Gln Phe Glu Asp Phe Phe Pro Ala Ser Ser Arg Asp Gln
   1 5 10 15
<210> 121
   <211> 15
   <212> PRT
   <213> Peanut peptide
<400> 121
   Phe Glu Asp Phe Phe Pro Ala Ser Ser Arg Asp Gln Ser Ser Tyr
   1 5 10 15
<210> 122
   <211> 15
   <212> PRT
   <213> Peanut peptide
<400> 122
   Ala Ser Ser Arg Asp Gln Ser Ser Tyr Leu Gln Gly Phe Ser Arg
   1 5 10 15
<210> 123
   <211> 15
   <212> PRT
   <213> Peanut peptide
<400> 123
   Arg Asp Gln Ser Ser Tyr Leu Gln Gly Phe Ser Arg Asn Thr Leu
   1 5 10 15
<210> 124
   <211> 15
   <212> PRT
   <213> Peanut peptide
<400> 124
   Ser Ser Tyr Leu Gln Gly Phe Ser Arg Asn Thr Leu Glu Ala Ala
   1 5 10 15
<210> 125
   <211> 15
   <212> PRT
   <213> Peanut peptide
<400> 125
   Leu Gln Gly Phe Ser Arg Asn Thr Leu Glu Ala Ala Phe Asn Ala
   1 5 10 15
<210> 126
   <211> 15
   <212> PRT
   <213> Peanut peptide
<400> 126
   Arg Val Leu Leu Glu Glu Asn Ala Gly Gly Glu Gln Glu Glu Arg
   1 5 10 15
<210> 127
   <211> 15
   <212> PRT
   <213> Peanut peptide
<400> 127
   Leu Glu Glu Asn Ala Gly Gly Glu Gln Glu Glu Arg Gly Gln Arg
   1 5 10 15
<210> 128
   <211> 15
   <212> PRT
   <213> Peanut peptide
<400> 128
   Asn Ala Gly Gly Glu Gln Glu Glu Arg Gly Gln Arg Arg Trp Ser
   1 5 10 15
<210> 129
   <211> 15
   <212> PRT
   <213> Peanut peptide
<400> 129
   Gly Glu Gln Glu Glu Arg Gly Gln Arg Arg Trp Ser Thr Arg Ser
   1               5                   10                  15
<210> 130
   <211> 15
   <212> PRT
   <213> Peanut peptide
<400> 130
   Glu Glu Arg Gly Gln Arg Arg Trp Ser Thr Arg Ser Ser Glu Asn
   1               5                   10                  15
<210> 131
   <211> 15
   <212> PRT
   <213> Peanut peptide
<400> 131
   Lys Lys Gly Ser Glu Glu Glu Gly Asp Ile Thr Asn Pro Ile Asn
   1               5                   10                  15
<210> 132
   <211> 15
   <212> PRT
   <213> Peanut peptide
<400> 132
   Ser Glu Glu Glu Gly Asp Ile Thr Asn Pro Ile Asn Leu Arg Glu
   1               5                   10                  15
<210> 133
   <211> 15
   <212> PRT
   <213> Peanut peptide
<400> 133
   Glu Gly Asp Ile Thr Asn Pro Ile Asn Leu Arg Glu Gly Glu Pro
   1               5                   10                  15
<210> 134
   <211> 15
   <212> PRT
   <213> Peanut peptide
<400> 134
   Ile Thr Asn Pro Ile Asn Leu Arg Glu Gly Glu Pro Asp Leu Ser
   1               5                   10                  15
<210> 135
   <211> 15
   <212> PRT
   <213> Peanut peptide
<400> 135
   Pro Ile Asn Leu Arg Glu Gly Glu Pro Asp Leu Ser Asn Asn Phe
   1 5 10 15
<210> 136
   <211> 15
   <212> PRT
   <213> Peanut peptide
<400> 136
   Leu Arg Glu Gly Glu Pro Asp Leu Ser Asn Asn Phe Gly Lys Leu
   1 5 10 15
<210> 137
   <211> 15
   <212> PRT
   <213> Peanut peptide
<400> 137
   Gly Glu Pro Asp Leu Ser Asn Asn Phe Gly Lys Leu Phe Glu Val
   1 5 10 15
<210> 138
   <211> 15
   <212> PRT
   <213> Peanut peptide
<400> 138
   Asp Leu Ser Asn Asn Phe Gly Lys Leu Phe Glu Val Lys Pro Asp
   1 5 10 15
<210> 139
   <211> 15
   <212> PRT
   <213> Peanut peptide
<400> 139
   Asn Asn Phe Gly Lys Leu Phe Glu Val Lys Pro Asp Lys Lys Asn
   1 5 10 15
<210> 140
   <211> 15
   <212> PRT
   <213> Peanut peptide
<400> 140
   Gly Lys Leu Phe Glu Val Lys Pro Asp Lys Lys Asn Pro Gln Leu
   1 5 10 15
<210> 141
   <211> 15
   <212> PRT
   <213> Peanut peptide
<400> 141
   Lys Lys Asn Pro Gln Leu Gln Asp Leu Asp Met Met Leu Thr Cys
   1 5 10 15
<210> 142
   <211> 15
   <212> PRT
   <213> Peanut peptide
<400> 142
   Pro Gln Leu Gln Asp Leu Asp Met Met Leu Thr Cys Val Glu Ile
   1 5 10 15
<210> 143
   <211> 15
   <212> PRT
   <213> Peanut peptide
<400> 143
   Gln Asp Leu Asp Met Met Leu Thr Cys Val Glu Ile Lys Glu Gly
   1 5 10 15
<210> 144
   <211> 15
   <212> PRT
   <213> Peanut peptide
<400> 144
   Asp Met Met Leu Thr Cys Val Glu Ile Lys Glu Gly Ala Leu Met
   1 5 10 15
<210> 145
   <211> 15
   <212> PRT
   <213> Peanut peptide
<400> 145
   Leu Thr Cys Val Glu Ile Lys Glu Gly Ala Leu Met Leu Pro His
   1 5 10 15
<210> 146
   <211> 15
   <212> PRT
   <213> Peanut peptide
<400> 146
   Val Glu Ile Lys Glu Gly Ala Leu Met Leu Pro His Phe Asn Ser
   1 5 10 15
<210> 147
   <211> 15
   <212> PRT
   <213> Peanut peptide
<400> 147
   Lys Glu Gly Ala Leu Met Leu Pro His Phe Asn Ser Lys Ala Met
   1 5 10 15
<210> 148
   <211> 15
   <212> PRT
   <213> Peanut peptide
<400> 148
   Ser Asn Arg Glu Val Arg Arg Tyr Thr Ala Arg Leu Lys Glu Gly
   1 5 10 15
<210> 149
   <211> 15
   <212> PRT
   <213> Peanut peptide
<400> 149
   Glu Val Arg Arg Tyr Thr Ala Arg Leu Lys Glu Gly Asp Val Phe
   1 5 10 15
<210> 150
   <211> 15
   <212> PRT
   <213> Peanut peptide
<400> 150
   Arg Tyr Thr Ala Arg Leu Lys Glu Gly Asp Val Phe Ile Met Pro
   1 5 10 15
<210> 151
   <211> 15
   <212> PRT
   <213> Peanut peptide
<400> 151
   Ala Arg Leu Lys Glu Gly Asp Val Phe Ile Met Pro Ala Ala His
   1 5 10 15
<210> 152
   <211> 15
   <212> PRT
   <213> Peanut peptide
<400> 152
   Lys Glu Gly Asp Val Phe Ile Met Pro Ala Ala His Pro Val Ala
   1 5 10 15
<210> 153
   <211> 15
   <212> PRT
   <213> Peanut peptide
<400> 153
   Asp Val Phe Ile Met Pro Ala Ala His Pro Val Ala Ile Asn Ala
   1 5 10 15
<210> 154
   <211> 15
   <212> PRT
   <213> Peanut peptide
<400> 154
   Pro Val Ala Ile Asn Ala Ser Ser Glu Leu His Leu Leu Gly Phe
   1 5 10 15
<210> 155
   <211> 15
   <212> PRT
   <213> Peanut peptide
<400> 155
   Ile Asn Ala Ser Ser Glu Leu His Leu Leu Gly Phe Gly Ile Asn
   1 5 10 15
<210> 156
   <211> 15
   <212> PRT
   <213> Peanut peptide
<400> 156
   Ser Ser Glu Leu His Leu Leu Gly Phe Gly Ile Asn Ala Glu Asn
   1 5 10 15
<210> 157
   <211> 15
   <212> PRT
   <213> Peanut peptide
<400> 157
   Leu His Leu Leu Gly Phe Gly Ile Asn Ala Glu Asn Asn His Arg
   1 5 10 15
<210> 158
   <211> 15
   <212> PRT
   <213> Peanut peptide
<400> 158
   Leu Gly Phe Gly Ile Asn Ala Glu Asn Asn His Arg Ile Phe Leu
   1               5                   10                  15
<210> 159
   <211> 15
   <212> PRT
   <213> Peanut peptide
<400> 159
   Gly Ile Asn Ala Glu Asn Asn His Arg Ile Phe Leu Ala Gly Asp
   1               5                   10                  15
<210> 160
   <211> 15
   <212> PRT
   <213> Peanut peptide
<400> 160
   Ala Glu Asn Asn His Arg Ile Phe Leu Ala Gly Asp Lys Asp Asn
   1               5                   10                  15
<210> 161
   <211> 15
   <212> PRT
   <213> Peanut peptide
<400> 161
   Asn His Arg Ile Phe Leu Ala Gly Asp Lys Asp Asn Val Ile Asp
   1               5                   10                  15
<210> 162
   <211> 15
   <212> PRT
   <213> Peanut peptide
<400> 162
   Ile Phe Leu Ala Gly Asp Lys Asp Asn Val Ile Asp Gln Ile Glu
   1               5                   10                  15
<210> 163
   <211> 15
   <212> PRT
   <213> Peanut peptide
<400> 163
   Ala Gly Asp Lys Asp Asn Val Ile Asp Gln Ile Glu Lys Gln Ala
   1               5                   10                  15
<210> 164
   <211> 15
   <212> PRT
   <213> Peanut peptide
<400> 164
   Lys Asp Asn Val Ile Asp Gln Ile Glu Lys Gln Ala Lys Asp Leu
   1               5                   10                  15
<210> 165
   <211> 15
   <212> PRT
   <213> Peanut peptide
<400> 165
   Val Ile Asp Gln Ile Glu Lys Gln Ala Lys Asp Leu Ala Phe Pro
   1               5                   10                  15
<210> 166
   <211> 15
   <212> PRT
   <213> Peanut peptide
<400> 166
   Gln Ile Glu Lys Gln Ala Lys Asp Leu Ala Phe Pro Gly Ser Gly
   1               5                   10                  15
<210> 167
   <211> 15
   <212> PRT
   <213> Peanut peptide
<400> 167
   Lys Gln Ala Lys Asp Leu Ala Phe Pro Gly Ser Gly Glu Gln Val
   1               5                   10                  15
<210> 168
   <211> 15
   <212> PRT
   <213> Peanut peptide
<400> 168
   Lys Asp Leu Ala Phe Pro Gly Ser Gly Glu Gln Val Glu Lys Leu
   1               5                   10                  15
<210> 169
   <211> 15
   <212> PRT
   <213> Peanut peptide
<400> 169
   Ala Phe Pro Gly Ser Gly Glu Gln Val Glu Lys Leu Ile Lys Asn
   1               5                   10                  15
<210> 170
   <211> 15
   <212> PRT
   <213> Peanut peptide
<400> 170
   Gly Ser Gly Glu Gln Val Glu Lys Leu Ile Lys Asn Gln Lys Glu
   1               5                   10                  15
<210> 171
   <211> 15
   <212> PRT
   <213> Peanut peptide
<400> 171
   Glu Gln Val Glu Lys Leu Ile Lys Asn Gln Lys Glu Ser His Phe
   1               5                   10                  15
<210> 172
   <211> 15
   <212> PRT
   <213> Peanut peptide
<400> 172
   Glu Lys Leu Ile Lys Asn Gln Lys Glu Ser His Phe Val Ser Ala
   1               5                   10                  15
<210> 173
   <211> 15
   <212> PRT
   <213> Peanut peptide
<400> 173
   Ile Lys Asn Gln Lys Glu Ser His Phe Val Ser Ala Arg Pro Gln
   1               5                   10                  15
<210> 174
   <211> 15
   <212> PRT
   <213> Peanut peptide
<400> 174
   Gln Lys Glu Ser His Phe Val Ser Ala Arg Pro Gln Ser Gln Ser
   1               5                   10                  15
<210> 175
   <211> 15
   <212> PRT
   <213> Peanut peptide
<400> 175
   Ser His Phe Val Ser Ala Arg Pro Gln Ser Gln Ser Gln Ser Pro
   1               5                   10                  15
<210> 176
   <211> 15
   <212> PRT
   <213> Peanut peptide
<400> 176
   Val Ser Ala Arg Pro Gln Ser Gln Ser Gln Ser Pro Ser Ser Pro
   1               5                   10                  15
<210> 177
   <211> 15
   <212> PRT
   <213> Peanut peptide
<400> 177
   Arg Pro Gln Ser Gln Ser Gln Ser Pro Ser Ser Pro Glu Lys Glu
   1               5                   10                  15
<210> 178
   <211> 15
   <212> PRT
   <213> Peanut peptide
<400> 178
   Ser Gln Ser Gln Ser Pro Ser Ser Pro Glu Lys Glu Ser Pro Glu
   1               5                   10                  15
<210> 179
   <211> 15
   <212> PRT
   <213> Peanut peptide
<400> 179
   Gln Ser Pro Ser Ser Pro Glu Lys Glu Ser Pro Glu Lys Glu Asp
   1               5                   10                  15
<210> 180
   <211> 15
   <212> PRT
   <213> Peanut peptide
<400> 180
   Ser Ser Pro Glu Lys Glu Ser Pro Glu Lys Glu Asp Gln Glu Glu
   1               5                   10                  15
<210> 181
   <211> 15
   <212> PRT
   <213> Peanut peptide
<400> 181
   Glu Lys Glu Ser Pro Glu Lys Glu Asp Gln Glu Glu Glu Asn Gln
   1               5                   10                  15
<210> 182
   <211> 15
   <212> PRT
   <213> Peanut peptide
<400> 182
   Ser Pro Glu Lys Glu Asp Gln Glu Glu Glu Asn Gln Gly Gly Lys
   1               5                   10                  15
<210> 183
   <211> 15
   <212> PRT
   <213> Peanut peptide
<400> 183
   Lys Glu Asp Gln Glu Glu Glu Asn Gln Gly Gly Lys Gly Pro Leu
   1               5                   10                  15
<210> 184
   <211> 15
   <212> PRT
   <213> Peanut peptide
<400> 184
   Gln Glu Glu Glu Asn Gln Gly Gly Lys Gly Pro Leu Leu Ser Ile
   1               5                   10                  15
<210> 185
   <211> 15
   <212> PRT
   <213> Peanut peptide
<400> 185
   Ala Ala His Ala Ser Ala Arg Gln Gln Trp Glu Leu Gln Gly Asp
   1               5                   10                  15
<210> 186
   <211> 15
   <212> PRT
   <213> Peanut peptide
<400> 186
   Ala Ser Ala Arg Gln Gln Trp Glu Leu Gln Gly Asp Arg Arg Cys
   1               5                   10                  15
<210> 187
   <211> 15
   <212> PRT
   <213> Peanut peptide
<400> 187
   Arg Gln Gln Trp Glu Leu Gln Gly Asp Arg Arg Cys Gln Ser Gln
   1 5 10 15
<210> 188
   <211> 15
   <212> PRT
   <213> Peanut peptide
<400> 188
   Trp Glu Leu Gln Gly Asp Arg Arg Cys Gln Ser Gln Leu Glu Arg
   1 5 10 15
<210> 189
   <211> 15
   <212> PRT
   <213> Peanut peptide
<400> 189
   Gln Gly Asp Arg Arg Cys Gln Ser Gln Leu Glu Arg Ala Asn Leu
   1 5 10 15
<210> 190
   <211> 15
   <212> PRT
   <213> Peanut peptide
<400> 190
   Arg Arg Cys Gln Ser Gln Leu Glu Arg Ala Asn Leu Arg Pro Cys
   1 5 10 15
<210> 191
   <211> 15
   <212> PRT
   <213> Peanut peptide
<400> 191
   Gln Ser Gln Leu Glu Arg Ala Asn Leu Arg Pro Cys Glu Gln His
   1 5 10 15
<210> 192
   <211> 15
   <212> PRT
   <213> Peanut peptide
<400> 192
   Leu Glu Arg Ala Asn Leu Arg Pro Cys Glu Gln His Leu Met Gln
   1 5 10 15
<210> 193
   <211> 15
   <212> PRT
   <213> Peanut peptide
<400> 193
   Ala Asn Leu Arg Pro Cys Glu Gln His Leu Met Gln Lys Ile Gln
   1 5 10 15
<210> 194
   <211> 15
   <212> PRT
   <213> Peanut peptide
<400> 194
   Arg Pro Cys Glu Gln His Leu Met Gln Lys Ile Gln Arg Asp Glu
   1 5 10 15
<210> 195
   <211> 15
   <212> PRT
   <213> Peanut peptide
<400> 195
   Glu Gln His Leu Met Gln Lys Ile Gln Arg Asp Glu Asp Ser Tyr
   1 5 10 15
<210> 196
   <211> 15
   <212> PRT
   <213> Peanut peptide
<400> 196
   Leu Met Gln Lys Ile Gln Arg Asp Glu Asp Ser Tyr Glu Arg Asp
   1 5 10 15
<210> 197
   <211> 15
   <212> PRT
   <213> Peanut peptide
<400> 197
   Lys Ile Gln Arg Asp Glu Asp Ser Tyr Glu Arg Asp Pro Tyr Ser
   1 5 10 15
<210> 198
   <211> 15
   <212> PRT
   <213> Peanut peptide
<400> 198
   Arg Asp Glu Asp Ser Tyr Glu Arg Asp Pro Tyr Ser Pro Ser Gln
   1               5                   10                  15
<210> 199
   <211> 15
   <212> PRT
   <213> Peanut peptide
<400> 199
   Asp Ser Tyr Glu Arg Asp Pro Tyr Ser Pro Ser Gln Asp Pro Tyr
   1               5                   10                  15
<210> 200
   <211> 15
   <212> PRT
   <213> Peanut peptide
<400> 200
   Glu Arg Asp Pro Tyr Ser Pro Ser Gln Asp Pro Tyr Ser Pro Ser
   1               5                   10                  15
<210> 201
   <211> 15
   <212> PRT
   <213> Peanut peptide
<400> 201
   Pro Tyr Ser Pro Ser Gln Asp Pro Tyr Ser Pro Ser Pro Tyr Asp
   1               5                   10                  15
<210> 202
   <211> 15
   <212> PRT
   <213> Peanut peptide
<400> 202
   Pro Ser Gln Asp Pro Tyr Ser Pro Ser Pro Tyr Asp Arg Arg Gly
   1               5                   10                  15
<210> 203
   <211> 15
   <212> PRT
   <213> Peanut peptide
<400> 203
   Asp Pro Tyr Ser Pro Ser Pro Tyr Asp Arg Arg Gly Ala Gly Ser
   1               5                   10                  15
<210> 204
   <211> 15
   <212> PRT
   <213> Peanut peptide
<400> 204
   Ser Pro Ser Pro Tyr Asp Arg Arg Gly Ala Gly Ser Ser Gln His
   1 5 10 15
<210> 205
   <211> 15
   <212> PRT
   <213> Peanut peptide
<400> 205
   Pro Tyr Asp Arg Arg Gly Ala Gly Ser Ser Gln His Gln Glu Arg
   1 5 10 15
<210> 206
   <211> 15
   <212> PRT
   <213> Peanut peptide
<400> 206
   Gln Glu Arg Cys Cys Asn Glu Leu Asn Glu Phe Glu Asn Asn Gln
   1 5 10 15
<210> 207
   <211> 15
   <212> PRT
   <213> Peanut peptide
<400> 207
   Cys Cys Asn Glu Leu Asn Glu Phe Glu Asn Asn Gln Arg Cys Met
   1 5 10 15
<210> 208
   <211> 15
   <212> PRT
   <213> Peanut peptide
<400> 208
   Glu Leu Asn Glu Phe Glu Asn Asn Gln Arg Cys Met Cys Glu Ala
   1 5 10 15
<210> 209
   <211> 15
   <212> PRT
   <213> Peanut peptide
<400> 209
   Glu Phe Glu Asn Asn Gln Arg Cys Met Cys Glu Ala Leu Gln Gln
   1 5 10 15
<210> 210
   <211> 15
   <212> PRT
   <213> Peanut peptide
<400> 210
   Arg Cys Met Cys Glu Ala Leu Gln Gln Ile Met Glu Asn Gln Ser
   1               5                   10                  15
<210> 211
   <211> 15
   <212> PRT
   <213> Peanut peptide
<400> 211
   Cys Glu Ala Leu Gln Gln Ile Met Glu Asn Gln Ser Asp Arg Leu
   1               5                   10                  15
<210> 212
   <211> 15
   <212> PRT
   <213> Peanut peptide
<400> 212
   Leu Gln Gln Ile Met Glu Asn Gln Ser Asp Arg Leu Gln Gly Arg
   1               5                   10                  15
<210> 213
   <211> 15
   <212> PRT
   <213> Peanut peptide
<400> 213
   Ile Met Glu Asn Gln Ser Asp Arg Leu Gln Gly Arg Gln Gln Glu
   1               5                   10                  15
<210> 214
   <211> 15
   <212> PRT
   <213> Peanut peptide
<400> 214
   Asn Gln Ser Asp Arg Leu Gln Gly Arg Gln Gln Glu Gln Gln Phe
   1               5                   10                  15
<210> 215
   <211> 15
   <212> PRT
   <213> Peanut peptide
<400> 215
   Asp Arg Leu Gln Gly Arg Gln Gln Glu Gln Gln Phe Lys Arg Glu
   1               5                   10                  15
<210> 216
   <211> 15
   <212> PRT
   <213> Peanut peptide
<400> 216
   Gln Gly Arg Gln Gln Glu Gln Gln Phe Lys Arg Glu Leu Arg Asn
   1               5                   10                  15
<210> 217
   <211> 15
   <212> PRT
   <213> Peanut peptide
<400> 217
   Gln Gln Glu Gln Gln Phe Lys Arg Glu Leu Arg Asn Leu Pro Gln
   1               5                   10                  15
<210> 218
   <211> 15
   <212> PRT
   <213> Peanut peptide
<400> 218
   Gln Gln Phe Lys Arg Glu Leu Arg Asn Leu Pro Gln Gln Cys Gly
   1               5                   10                  15
<210> 219
   <211> 15
   <212> PRT
   <213> Peanut peptide
<400> 219
   Lys Arg Glu Leu Arg Asn Leu Pro Gln Gln Cys Gly Leu Arg Ala
   1               5                   10                  15
<210> 220
   <211> 15
   <212> PRT
   <213> Peanut peptide
<400> 220
   Leu Pro Gln Gln Cys Gly Leu Arg Ala Pro Gln Arg Cys Asp Leu
   1               5                   10                  15
<210> 221
   <211> 15
   <212> PRT
   <213> Peanut peptide
<400> 221
   Gln Cys Gly Leu Arg Ala Pro Gln Arg Cys Asp Leu Asp Val Glu
   1 5 10 15
<210> 222
   <211> 15
   <212> PRT
   <213> Peanut peptide
<400> 222
   Leu Arg Ala Pro Gln Arg Cys Asp Leu Asp Val Glu Ser Gly Gly
   1 5 10 15
<210> 223
   <211> 15
   <212> PRT
   <213> Peanut peptide
<400> 223
   Arg Ile Glu Ser Glu Gly Gly Tyr Ile Glu Thr Trp Asn Pro Asn
   1 5 10 15
<210> 224
   <211> 15
   <212> PRT
   <213> Peanut peptide
<400> 224
   Asn Gln Glu Phe Glu Cys Ala Gly Val Ala Leu Ser Arg Leu Val
   1 5 10 15
<210> 225
   <211> 15
   <212> PRT
   <213> Peanut peptide
<400> 225
   Ala Gly Val Ala Leu Ser Arg Leu Val Leu Arg Arg Asn Ala Leu
   1 5 10 15
<210> 226
   <211> 15
   <212> PRT
   <213> Peanut peptide
<400> 226
   Ala Leu Ser Arg Leu Val Leu Arg Arg Asn Ala Leu Arg Arg Pro
   1 5 10 15
<210> 227
   <211> 15
   <212> PRT
   <213> Peanut peptide
<400> 227
   Arg Leu Val Leu Arg Arg Asn Ala Leu Arg Arg Pro Phe Tyr Ser
   1 5 10 15
<210> 228
   <211> 15
   <212> PRT
   <213> Peanut peptide
<400> 228
   Leu Arg Arg Asn Ala Leu Arg Arg Pro Phe Tyr Ser Asn Ala Pro
   1 5 10 15
<210> 229
   <211> 15
   <212> PRT
   <213> Peanut peptide
<400> 229
   Asn Ala Leu Arg Arg Pro Phe Tyr Ser Asn Ala Pro Gln Glu Ile
   1 5 10 15
<210> 230
   <211> 15
   <212> PRT
   <213> Peanut peptide
<400> 230
   His Tyr Glu Glu Pro His Thr Gln Gly Arg Arg Ser Gln Ser Gln
   1 5 10 15
<210> 231
   <211> 15
   <212> PRT
   <213> Peanut peptide
<400> 231
   Glu Pro His Thr Gln Gly Arg Arg Ser Gln Ser Gln Arg Pro Pro
   1 5 10 15
<210> 232
   <211> 15
   <212> PRT
   <213> Peanut peptide
<400> 232
   Thr Gln Gly Arg Arg Ser Gln Ser Gln Arg Pro Pro Arg Arg Leu
   1 5 10 15
<210> 233
   <211> 15
   <212> PRT
   <213> Peanut peptide
<400> 233
   Arg Arg Ser Gln Ser Gln Arg Pro Pro Arg Arg Leu Gln Gly Glu
   1 5 10 15
<210> 234
   <211> 15
   <212> PRT
   <213> Peanut peptide
<400> 234
   Arg Arg Leu Gln Gly Glu Asp Gln Ser Gln Gln Gln Arg Asp Ser
   1 5 10 15
<210> 235
   <211> 15
   <212> PRT
   <213> Peanut peptide
<400> 235
   Gln Gly Glu Asp Gln Ser Gln Gln Gln Arg Asp Ser His Gln Lys
   1 5 10 15
<210> 236
   <211> 15
   <212> PRT
   <213> Peanut peptide
<400> 236
   Asn Thr Glu Gln Glu Phe Leu Arg Tyr Gln Gln Gln Ser Arg Gln
   1 5 10 15
<210> 237
   <211> 15
   <212> PRT
   <213> Peanut peptide
<400> 237
   Gln Glu Phe Leu Arg Tyr Gln Gln Gln Ser Arg Gln Ser Arg Arg
   1 5 10 15
<210> 238
   <211> 15
   <212> PRT
   <213> Peanut peptide
<400> 238
   Pro Tyr Ser Pro Gln Ser Gln Pro Arg Gln Glu Glu Arg Glu Phe
   1 5 10 15
<210> 239
   <211> 15
   <212> PRT
   <213> Peanut peptide
<400> 239
   Pro Gln Ser Gln Pro Arg Gln Glu Glu Arg Glu Phe Ser Pro Arg
   1 5 10 15
<210> 240
   <211> 15
   <212> PRT
   <213> Peanut peptide
<400> 240
   Gln Pro Arg Gln Glu Glu Arg Glu Phe Ser Pro Arg Gly Gln His
   1 5 10 15
<210> 241
   <211> 15
   <212> PRT
   <213> Peanut peptide
<400> 241
   Gln Glu Glu Arg Glu Phe Ser Pro Arg Gly Gln His Ser Arg Arg
   1 5 10 15
<210> 242
   <211> 15
   <212> PRT
   <213> Peanut peptide
<400> 242
   Ser Pro Arg Gly Gln His Ser Arg Arg Glu Arg Ala Gly Gln Glu
   1 5 10 15
<210> 243
   <211> 15
   <212> PRT
   <213> Peanut peptide
<400> 243
   Gly Gln His Ser Arg Arg Glu Arg Ala Gly Gln Glu Glu Glu Asn
   1 5 10 15
<210> 244
   <211> 15
   <212> PRT
   <213> Peanut peptide
<400> 244
   Ser Arg Arg Glu Arg Ala Gly Gln Glu Glu Glu Asn Glu Gly Gly
   1 5 10 15
<210> 245
   <211> 15
   <212> PRT
   <213> Peanut peptide
<400> 245
   Gly Gln Glu Glu Glu Asn Glu Gly Gly Asn Ile Phe Ser Gly Phe
   1               5                   10                  15
<210> 246
   <211> 15
   <212> PRT
   <213> Peanut peptide
<400> 246
   Glu Glu Asn Glu Gly Gly Asn Ile Phe Ser Gly Phe Thr Pro Glu
   1               5                   10                  15
<210> 247
   <211> 15
   <212> PRT
   <213> Peanut peptide
<400> 247
   Glu Gly Gly Asn Ile Phe Ser Gly Phe Thr Pro Glu Phe Leu Glu
   1               5                   10                  15
<210> 248
   <211> 15
   <212> PRT
   <213> Peanut peptide
<400> 248
   Asn Ile Phe Ser Gly Phe Thr Pro Glu Phe Leu Glu Gln Ala Phe
   1               5                   10                  15
<210> 249
   <211> 15
   <212> PRT
   <213> Peanut peptide
<400> 249
   Ser Gly Phe Thr Pro Glu Phe Leu Glu Gln Ala Phe Gln Val Asp
   1               5                   10                  15
<210> 250
   <211> 15
   <212> PRT
   <213> Peanut peptide
<400> 250
   Thr Pro Glu Phe Leu Glu Gln Ala Phe Gln Val Asp Asp Arg Gln
   1 5 10 15
<210> 251
   <211> 15
   <212> PRT
   <213> Peanut peptide
<400> 251
   Glu Ser Glu Glu Glu Gly Ala Ile Val Thr Val Arg Gly Gly Leu
   1 5 10 15
<210> 252
   <211> 15
   <212> PRT
   <213> Peanut peptide
<400> 252
   Glu Glu Gly Ala Ile Val Thr Val Arg Gly Gly Leu Arg Ile Leu
   1 5 10 15
<210> 253
   <211> 15
   <212> PRT
   <213> Peanut peptide
<400> 253
   Ala Ile Val Thr Val Arg Gly Gly Leu Arg Ile Leu Ser Pro Asp
   1 5 10 15
<210> 254
   <211> 15
   <212> PRT
   <213> Peanut peptide
<400> 254
   Thr Val Arg Gly Gly Leu Arg Ile Leu Ser Pro Asp Arg Lys Arg
   1 5 10 15
<210> 255
   <211> 15
   <212> PRT
   <213> Peanut peptide
<400> 255
   Gly Gly Leu Arg Ile Leu Ser Pro Asp Arg Lys Arg Arg Ala Asp
   1 5 10 15
<210> 256
   <211> 15
   <212> PRT
   <213> Peanut peptide
<400> 256
   Arg Ile Leu Ser Pro Asp Arg Lys Arg Arg Ala Asp Glu Glu Glu
   1 5 10 15
<210> 257
   <211> 15
   <212> PRT
   <213> Peanut peptide
<400> 257
   Arg Lys Arg Arg Ala Asp Glu Glu Glu Glu Tyr Asp Glu Asp Glu
   1 5 10 15
<210> 258
   <211> 15
   <212> PRT
   <213> Peanut peptide
<400> 258
   Arg Ala Asp Glu Glu Glu Glu Tyr Asp Glu Asp Glu Tyr Glu Tyr
   1 5 10 15
<210> 259
   <211> 15
   <212> PRT
   <213> Peanut peptide
<400> 259
   Glu Glu Glu Glu Tyr Asp Glu Asp Glu Tyr Glu Tyr Asp Glu Glu
   1 5 10 15
<210> 260
   <211> 15
   <212> PRT
   <213> Peanut peptide
<400> 260
   Glu Tyr Asp Glu Asp Glu Tyr Glu Tyr Asp Glu Glu Asp Arg Arg
   1 5 10 15
<210> 261
   <211> 15
   <212> PRT
   <213> Peanut peptide
<400> 261
   Glu Asp Glu Tyr Glu Tyr Asp Glu Glu Asp Arg Arg Arg Gly Arg
   1 5 10 15
<210> 262
   <211> 15
   <212> PRT
   <213> Peanut peptide
<400> 262
   Tyr Glu Tyr Asp Glu Glu Asp Arg Arg Arg Gly Arg Gly Ser Arg
   1 5 10 15
<210> 263
   <211> 15
   <212> PRT
   <213> Peanut peptide
<400> 263
   Asp Glu Glu Asp Arg Arg Arg Gly Arg Gly Ser Arg Gly Arg Gly
   1 5 10 15
<210> 264
   <211> 15
   <212> PRT
   <213> Peanut peptide
<400> 264
   Asp Arg Arg Arg Gly Arg Gly Ser Arg Gly Arg Gly Asn Gly Ile
   1 5 10 15
<210> 265
   <211> 15
   <212> PRT
   <213> Peanut peptide
<400> 265
   Arg Gly Arg Gly Ser Arg Gly Arg Gly Asn Gly Ile Glu Glu Thr
   1 5 10 15
<210> 266
   <211> 15
   <212> PRT
   <213> Peanut peptide
<400> 266
   Gly Ser Arg Gly Arg Gly Asn Gly Ile Glu Glu Thr Ile Cys Thr
   1 5 10 15
<210> 267
   <211> 15
   <212> PRT
   <213> Peanut peptide
<400> 267
   Gly Arg Gly Asn Gly Ile Glu Glu Thr Ile Cys Thr Ala Ser Ala
   1 5 10 15
<210> 268
   <211> 15
   <212> PRT
   <213> Peanut peptide
<400> 268
   Asn Gly Ile Glu Glu Thr Ile Cys Thr Ala Ser Ala Lys Lys Asn
   1 5 10 15
<210> 269
   <211> 15
   <212> PRT
   <213> Peanut peptide
<400> 269
   Ile Ala Asn Leu Ala Gly Glu Asn Ser Val Ile Asp Asn Leu Pro
   1 5 10 15
<210> 270
   <211> 15
   <212> PRT
   <213> Peanut peptide
<400> 270
   Leu Ala Gly Glu Asn Ser Val Ile Asp Asn Leu Pro Glu Glu Val
   1 5 10 15
<210> 271
   <211> 15
   <212> PRT
   <213> Peanut peptide
<400> 271
   Glu Asn Ser Val Ile Asp Asn Leu Pro Glu Glu Val Val Ala Asn
   1 5 10 15
<210> 272
   <211> 15
   <212> PRT
   <213> Peanut peptide
<400> 272
   Val Ile Asp Asn Leu Pro Glu Glu Val Val Ala Asn Ser Tyr Gly
   1 5 10 15
<210> 273
   <211> 15
   <212> PRT
   <213> Peanut peptide
<400> 273
   Glu Gln Ala Arg Gln Leu Lys Asn Asn Asn Pro Phe Lys Phe Phe
   1 5 10 15
<210> 274
   <211> 15
   <212> PRT
   <213> Peanut peptide
<400> 274
   Arg Gln Leu Lys Asn Asn Asn Pro Phe Lys Phe Phe Val Pro Pro
   1               5                   10                  15
<210> 275
   <211> 15
   <212> PRT
   <213> Peanut peptide
<400> 275
   Lys Asn Asn Asn Pro Phe Lys Phe Phe Val Pro Pro Ser Gln Gln
   1               5                   10                  15
<210> 276
   <211> 15
   <212> PRT
   <213> Peanut peptide
<400> 276
   Asn Pro Phe Lys Phe Phe Val Pro Pro Ser Gln Gln Ser Pro Arg
   1               5                   10                  15
<210> 277
   <211> 15
   <212> PRT
   <213> Peanut peptide
<400> 277
   Lys Phe Phe Val Pro Pro Ser Gln Gln Ser Pro Arg Ala Val Ala
   1               5                   10                  15
<210> 278
   <211> 15
   <212> PRT
   <213> Egg peptide
<400> 278
   Met Gly Ser Ile Gly Ala Ala Ser Met Glu Phe Cys Phe Asp Val
   1               5                   10                  15
<210> 279
   <211> 15
   <212> PRT
   <213> Egg peptide
<400> 279
   Ile Gly Ala Ala Ser Met Glu Phe Cys Phe Asp Val Phe Lys Glu
   1 5 10 15
<210> 280
   <211> 15
   <212> PRT
   <213> Egg peptide
<400> 280
   Ala Ser Met Glu Phe Cys Phe Asp Val Phe Lys Glu Leu Lys Val
   1 5 10 15
<210> 281
   <211> 15
   <212> PRT
   <213> Egg peptide
<400> 281
   Glu Phe Cys Phe Asp Val Phe Lys Glu Leu Lys Val His His Ala
   1 5 10 15
<210> 282
   <211> 15
   <212> PRT
   <213> Egg peptide
<400> 282
   Phe Asp Val Phe Lys Glu Leu Lys Val His His Ala Asn Glu Asn
   1 5 10 15
<210> 283
   <211> 15
   <212> PRT
   <213> Egg peptide
<400> 283
   Phe Lys Glu Leu Lys Val His His Ala Asn Glu Asn Ile Phe Tyr
   1 5 10 15
<210> 284
   <211> 15
   <212> PRT
   <213> Egg peptide
<400> 284
   Leu Lys Val His His Ala Asn Glu Asn Ile Phe Tyr Cys Pro Ile
   1 5 10 15
<210> 285
   <211> 15
   <212> PRT
   <213> Egg peptide
<400> 285
   His His Ala Asn Glu Asn Ile Phe Tyr Cys Pro Ile Ala Ile Met
   1 5 10 15
<210> 286
   <211> 15
   <212> PRT
   <213> Egg peptide
<400> 286
   Asn Glu Asn Ile Phe Tyr Cys Pro Ile Ala Ile Met Ser Ala Leu
   1 5 10 15
<210> 287
   <211> 15
   <212> PRT
   <213> Egg peptide
<400> 287
   Ile Phe Tyr Cys Pro Ile Ala Ile Met Ser Ala Leu Ala Met Val
   1 5 10 15
<210> 288
   <211> 15
   <212> PRT
   <213> Egg peptide
<400> 288
   Cys Pro Ile Ala Ile Met Ser Ala Leu Ala Met Val Tyr Leu Gly
   1 5 10 15
<210> 289
   <211> 15
   <212> PRT
   <213> Egg peptide
<400> 289
   Ala Ile Met Ser Ala Leu Ala Met Val Tyr Leu Gly Ala Lys Asp
   1 5 10 15
<210> 290
   <211> 15
   <212> PRT
   <213> Egg peptide
<400> 290
   Ser Ala Leu Ala Met Val Tyr Leu Gly Ala Lys Asp Ser Thr Arg
   1 5 10 15
<210> 291
   <211> 15
   <212> PRT
   <213> Egg peptide
<400> 291
   Ala Met Val Tyr Leu Gly Ala Lys Asp Ser Thr Arg Thr Gln Ile
   1 5 10 15
<210> 292
   <211> 15
   <212> PRT
   <213> Egg peptide
<400> 292
   Tyr Leu Gly Ala Lys Asp Ser Thr Arg Thr Gln Ile Asn Lys Val
   1 5 10 15
<210> 293
   <211> 15
   <212> PRT
   <213> Egg peptide
<400> 293
   Ala Lys Asp Ser Thr Arg Thr Gln Ile Asn Lys Val Val Arg Phe
   1 5 10 15
<210> 294
   <211> 15
   <212> PRT
   <213> Egg peptide
<400> 294
   Ser Thr Arg Thr Gln Ile Asn Lys Val Val Arg Phe Asp Lys Leu
   1 5 10 15
<210> 295
   <211> 15
   <212> PRT
   <213> Egg peptide
<400> 295
   Thr Gln Ile Asn Lys Val Val Arg Phe Asp Lys Leu Pro Gly Phe
   1 5 10 15
<210> 296
   <211> 15
   <212> PRT
   <213> Egg peptide
<400> 296
   Asn Lys Val Val Arg Phe Asp Lys Leu Pro Gly Phe Gly Asp Ser
   1 5 10 15
<210> 297
   <211> 15
   <212> PRT
   <213> Egg peptide
<400> 297
   Val Arg Phe Asp Lys Leu Pro Gly Phe Gly Asp Ser Ile Glu Ala
   1 5 10 15
<210> 298
   <211> 15
   <212> PRT
   <213> Egg peptide
<400> 298
   Asp Lys Leu Pro Gly Phe Gly Asp Ser Ile Glu Ala Gln Cys Gly
   1 5 10 15
<210> 299
   <211> 15
   <212> PRT
   <213> Egg peptide
<400> 299
   Pro Gly Phe Gly Asp Ser Ile Glu Ala Gln Cys Gly Thr Ser Val
   1 5 10 15
<210> 300
   <211> 15
   <212> PRT
   <213> Egg peptide
<400> 300
   Gly Asp Ser Ile Glu Ala Gln Cys Gly Thr Ser Val Asn Val His
   1 5 10 15
<210> 301
   <211> 15
   <212> PRT
   <213> Egg peptide
<400> 301
   Ile Glu Ala Gln Cys Gly Thr Ser Val Asn Val His Ser Ser Leu
   1 5 10 15
<210> 302
   <211> 15
   <212> PRT
   <213> Egg peptide
<400> 302
   Gln Cys Gly Thr Ser Val Asn Val His Ser Ser Leu Arg Asp Ile
   1               5                   10                  15
<210> 303
   <211> 15
   <212> PRT
   <213> Egg peptide
<400> 303
   Thr Ser Val Asn Val His Ser Ser Leu Arg Asp Ile Leu Asn Gln
   1               5                   10                  15
<210> 304
   <211> 15
   <212> PRT
   <213> Egg peptide
<400> 304
   Asn Val His Ser Ser Leu Arg Asp Ile Leu Asn Gln Ile Thr Lys
   1               5                   10                  15
<210> 305
   <211> 15
   <212> PRT
   <213> Egg peptide
<400> 305
   Ser Ser Leu Arg Asp Ile Leu Asn Gln Ile Thr Lys Pro Asn Asp
   1               5                   10                  15
<210> 306
   <211> 15
   <212> PRT
   <213> Egg peptide
<400> 306
   Arg Asp Ile Leu Asn Gln Ile Thr Lys Pro Asn Asp Val Tyr Ser
   1               5                   10                  15
<210> 307
   <211> 15
   <212> PRT
   <213> Egg peptide
<400> 307
   Leu Asn Gln Ile Thr Lys Pro Asn Asp Val Tyr Ser Phe Ser Leu
   1               5                   10                  15
<210> 308
   <211> 15
   <212> PRT
   <213> Egg peptide
<400> 308
   Ile Thr Lys Pro Asn Asp Val Tyr Ser Phe Ser Leu Ala Ser Arg
   1 5 10 15
<210> 309
   <211> 15
   <212> PRT
   <213> Egg peptide
<400> 309
   Pro Asn Asp Val Tyr Ser Phe Ser Leu Ala Ser Arg Leu Tyr Ala
   1 5 10 15
<210> 310
   <211> 15
   <212> PRT
   <213> Egg peptide
<400> 310
   Val Tyr Ser Phe Ser Leu Ala Ser Arg Leu Tyr Ala Glu Glu Arg
   1 5 10 15
<210> 311
   <211> 15
   <212> PRT
   <213> Egg peptide
<400> 311
   Phe Ser Leu Ala Ser Arg Leu Tyr Ala Glu Glu Arg Tyr Pro Ile
   1 5 10 15
<210> 312
   <211> 15
   <212> PRT
   <213> Egg peptide
<400> 312
   Ala Ser Arg Leu Tyr Ala Glu Glu Arg Tyr Pro Ile Leu Pro Glu
   1 5 10 15
<210> 313
   <211> 15
   <212> PRT
   <213> Egg peptide
<400> 313
   Leu Tyr Ala Glu Glu Arg Tyr Pro Ile Leu Pro Glu Tyr Leu Gln
   1 5 10 15
<210> 314
   <211> 15
   <212> PRT
   <213> Egg peptide
<400> 314
   Glu Glu Arg Tyr Pro Ile Leu Pro Glu Tyr Leu Gln Cys Val Lys
   1 5 10 15
<210> 315
   <211> 15
   <212> PRT
   <213> Egg peptide
<400> 315
   Tyr Pro Ile Leu Pro Glu Tyr Leu Gln Cys Val Lys Glu Leu Tyr
   1 5 10 15
<210> 316
   <211> 15
   <212> PRT
   <213> Egg peptide
<400> 316
   Leu Pro Glu Tyr Leu Gln Cys Val Lys Glu Leu Tyr Arg Gly Gly
   1 5 10 15
<210> 317
   <211> 15
   <212> PRT
   <213> Egg peptide
<400> 317
   Tyr Leu Gln Cys Val Lys Glu Leu Tyr Arg Gly Gly Leu Glu Pro
   1 5 10 15
<210> 318
   <211> 15
   <212> PRT
   <213> Egg peptide
<400> 318
   Cys Val Lys Glu Leu Tyr Arg Gly Gly Leu Glu Pro Ile Asn Phe
   1 5 10 15
<210> 319
   <211> 15
   <212> PRT
   <213> Egg peptide
<400> 319
   Glu Leu Tyr Arg Gly Gly Leu Glu Pro Ile Asn Phe Gln Thr Ala
   1 5 10 15
<210> 320
   <211> 15
   <212> PRT
   <213> Egg peptide
<400> 320
   Arg Gly Gly Leu Glu Pro Ile Asn Phe Gln Thr Ala Ala Asp Gln
   1 5 10 15
<210> 321
   <211> 15
   <212> PRT
   <213> Egg peptide
<400> 321
   Leu Glu Pro Ile Asn Phe Gln Thr Ala Ala Asp Gln Ala Arg Glu
   1 5 10 15
<210> 322
   <211> 15
   <212> PRT
   <213> Egg peptide
<400> 322
   Ile Asn Phe Gln Thr Ala Ala Asp Gln Ala Arg Glu Leu Ile Asn
   1 5 10 15
<210> 323
   <211> 15
   <212> PRT
   <213> Egg peptide
<400> 323
   Gln Thr Ala Ala Asp Gln Ala Arg Glu Leu Ile Asn Ser Trp Val
   1 5 10 15
<210> 324
   <211> 15
   <212> PRT
   <213> Egg peptide
<400> 324
   Ala Asp Gln Ala Arg Glu Leu Ile Asn Ser Trp Val Glu Ser Gln
   1 5 10 15
<210> 325
   <211> 15
   <212> PRT
   <213> Egg peptide
<400> 325
   Ala Arg Glu Leu Ile Asn Ser Trp Val Glu Ser Gln Thr Asn Gly
   1 5 10 15
<210> 326
   <211> 15
   <212> PRT
   <213> Egg peptide
<400> 326
   Leu Ile Asn Ser Trp Val Glu Ser Gln Thr Asn Gly Ile Ile Arg
   1 5 10 15
<210> 327
   <211> 15
   <212> PRT
   <213> Egg peptide
<400> 327
   Ser Trp Val Glu Ser Gln Thr Asn Gly Ile Ile Arg Asn Val Leu
   1 5 10 15
<210> 328
   <211> 15
   <212> PRT
   <213> Egg peptide
<400> 328
   Glu Ser Gln Thr Asn Gly Ile Ile Arg Asn Val Leu Gln Pro Ser
   1 5 10 15
<210> 329
   <211> 15
   <212> PRT
   <213> Egg peptide
<400> 329
   Thr Asn Gly Ile Ile Arg Asn Val Leu Gln Pro Ser Ser Val Asp
   1 5 10 15
<210> 330
   <211> 15
   <212> PRT
   <213> Egg peptide
<400> 330
   Ile Ile Arg Asn Val Leu Gln Pro Ser Ser Val Asp Ser Gln Thr
   1 5 10 15
<210> 331
   <211> 15
   <212> PRT
   <213> Egg peptide
<400> 331
   Asn Val Leu Gln Pro Ser Ser Val Asp Ser Gln Thr Ala Met Val
   1 5 10 15
<210> 332
   <211> 15
   <212> PRT
   <213> Egg peptide
<400> 332
   Gln Pro Ser Ser Val Asp Ser Gln Thr Ala Met Val Leu Val Asn
   1 5 10 15
<210> 333
   <211> 15
   <212> PRT
   <213> Egg peptide
<400> 333
   Ser Val Asp Ser Gln Thr Ala Met Val Leu Val Asn Ala Ile Val
   1 5 10 15
<210> 334
   <211> 15
   <212> PRT
   <213> Egg peptide
<400> 334
   Ser Gln Thr Ala Met Val Leu Val Asn Ala Ile Val Phe Lys Gly
   1 5 10 15
<210> 335
   <211> 15
   <212> PRT
   <213> Egg peptide
<400> 335
   Ala Met Val Leu Val Asn Ala Ile Val Phe Lys Gly Leu Trp Glu
   1 5 10 15
<210> 336
   <211> 15
   <212> PRT
   <213> Egg peptide
<400> 336
   Leu Val Asn Ala Ile Val Phe Lys Gly Leu Trp Glu Lys Ala Phe
   1 5 10 15
<210> 337
   <211> 15
   <212> PRT
   <213> Egg peptide
<400> 337
   Ala Ile Val Phe Lys Gly Leu Trp Glu Lys Ala Phe Lys Asp Glu
   1               5                   10                  15
<210> 338
   <211> 15
   <212> PRT
   <213> Egg peptide
<400> 338
   Phe Lys Gly Leu Trp Glu Lys Ala Phe Lys Asp Glu Asp Thr Gln
   1               5                   10                  15
<210> 339
   <211> 15
   <212> PRT
   <213> Egg peptide
<400> 339
   Leu Trp Glu Lys Ala Phe Lys Asp Glu Asp Thr Gln Ala Met Pro
   1               5                   10                  15
<210> 340
   <211> 15
   <212> PRT
   <213> Egg peptide
<400> 340
   Lys Ala Phe Lys Asp Glu Asp Thr Gln Ala Met Pro Phe Arg Val
   1               5                   10                  15
<210> 341
   <211> 15
   <212> PRT
   <213> Egg peptide
<400> 341
   Lys Asp Glu Asp Thr Gln Ala Met Pro Phe Arg Val Thr Glu Gln
   1               5                   10                  15
<210> 342
   <211> 15
   <212> PRT
   <213> Egg peptide
<400> 342
   Asp Thr Gln Ala Met Pro Phe Arg Val Thr Glu Gln Glu Ser Lys
   1               5                   10                  15
<210> 343
   <211> 15
   <212> PRT
   <213> Egg peptide
<400> 343
   Ala Met Pro Phe Arg Val Thr Glu Gln Glu Ser Lys Pro Val Gln
   1               5                   10                  15
<210> 344
   <211> 15
   <212> PRT
   <213> Egg peptide
<400> 344
   Phe Arg Val Thr Glu Gln Glu Ser Lys Pro Val Gln Met Met Tyr
   1               5                   10                  15
<210> 345
   <211> 15
   <212> PRT
   <213> Egg peptide
<400> 345
   Thr Glu Gln Glu Ser Lys Pro Val Gln Met Met Tyr Gln Ile Gly
   1               5                   10                  15
<210> 346
   <211> 15
   <212> PRT
   <213> Egg peptide
<400> 346
   Glu Ser Lys Pro Val Gln Met Met Tyr Gln Ile Gly Leu Phe Arg
   1               5                   10                  15
<210> 347
   <211> 15
   <212> PRT
   <213> Egg peptide
<400> 347
   Pro Val Gln Met Met Tyr Gln Ile Gly Leu Phe Arg Val Ala Ser
   1               5                   10                  15
<210> 348
   <211> 15
   <212> PRT
   <213> Egg peptide
<400> 348
   Met Met Tyr Gln Ile Gly Leu Phe Arg Val Ala Ser Met Ala Ser
   1 5 10 15
<210> 349
   <211> 15
   <212> PRT
   <213> Egg peptide
<400> 349
   Gln Ile Gly Leu Phe Arg Val Ala Ser Met Ala Ser Glu Lys Met
   1 5 10 15
<210> 350
   <211> 15
   <212> PRT
   <213> Egg peptide
<400> 350
   Leu Phe Arg Val Ala Ser Met Ala Ser Glu Lys Met Lys Ile Leu
   1 5 10 15
<210> 351
   <211> 15
   <212> PRT
   <213> Egg peptide
<400> 351
   Val Ala Ser Met Ala Ser Glu Lys Met Lys Ile Leu Glu Leu Pro
   1 5 10 15
<210> 352
   <211> 15
   <212> PRT
   <213> Egg peptide
<400> 352
   Met Ala Ser Glu Lys Met Lys Ile Leu Glu Leu Pro Phe Ala Ser
   1 5 10 15
<210> 353
   <211> 15
   <212> PRT
   <213> Egg peptide
<400> 353
   Glu Lys Met Lys Ile Leu Glu Leu Pro Phe Ala Ser Gly Thr Met
   1 5 10 15
<210> 354
   <211> 15
   <212> PRT
   <213> Egg peptide
<400> 354
   Lys Ile Leu Glu Leu Pro Phe Ala Ser Gly Thr Met Ser Met Leu
   1 5 10 15
<210> 355
   <211> 15
   <212> PRT
   <213> Egg peptide
<400> 355
   Glu Leu Pro Phe Ala Ser Gly Thr Met Ser Met Leu Val Leu Leu
   1 5 10 15
<210> 356
   <211> 15
   <212> PRT
   <213> Egg peptide
<400> 356
   Phe Ala Ser Gly Thr Met Ser Met Leu Val Leu Leu Pro Asp Glu
   1 5 10 15
<210> 357
   <211> 15
   <212> PRT
   <213> Egg peptide
<400> 357
   Gly Thr Met Ser Met Leu Val Leu Leu Pro Asp Glu Val Ser Gly
   1 5 10 15
<210> 358
   <211> 15
   <212> PRT
   <213> Egg peptide
<400> 358
   Ser Met Leu Val Leu Leu Pro Asp Glu Val Ser Gly Leu Glu Gln
   1 5 10 15
<210> 359
   <211> 15
   <212> PRT
   <213> Egg peptide
<400> 359
   Val Leu Leu Pro Asp Glu Val Ser Gly Leu Glu Gln Leu Glu Ser
   1 5 10 15
<210> 360
   <211> 15
   <212> PRT
   <213> Egg peptide
<400> 360
   Pro Asp Glu Val Ser Gly Leu Glu Gln Leu Glu Ser Ile Ile Asn
   1 5 10 15
<210> 361
   <211> 15
   <212> PRT
   <213> Egg peptide
<400> 361
   Val Ser Gly Leu Glu Gln Leu Glu Ser Ile Ile Asn Phe Glu Lys
   1 5 10 15
<210> 362
   <211> 15
   <212> PRT
   <213> Egg peptide
<400> 362
   Leu Glu Gln Leu Glu Ser Ile Ile Asn Phe Glu Lys Leu Thr Glu
   1 5 10 15
<210> 363
   <211> 15
   <212> PRT
   <213> Egg peptide
<400> 363
   Leu Glu Ser Ile Ile Asn Phe Glu Lys Leu Thr Glu Trp Thr Ser
   1 5 10 15
<210> 364
   <211> 15
   <212> PRT
   <213> Egg peptide
<400> 364
   Ile Ile Asn Phe Glu Lys Leu Thr Glu Trp Thr Ser Ser Asn Val
   1 5 10 15
<210> 365
   <211> 15
   <212> PRT
   <213> Egg peptide
<400> 365
   Phe Glu Lys Leu Thr Glu Trp Thr Ser Ser Asn Val Met Glu Glu
   1 5 10 15
<210> 366
   <211> 15
   <212> PRT
   <213> Egg peptide
<400> 366
   Leu Thr Glu Trp Thr Ser Ser Asn Val Met Glu Glu Arg Lys Ile
   1 5 10 15
<210> 367
   <211> 15
   <212> PRT
   <213> Egg peptide
<400> 367
   Trp Thr Ser Ser Asn Val Met Glu Glu Arg Lys Ile Lys Val Tyr
   1 5 10 15
<210> 368
   <211> 15
   <212> PRT
   <213> Egg peptide
<400> 368
   Ser Asn Val Met Glu Glu Arg Lys Ile Lys Val Tyr Leu Pro Arg
   1 5 10 15
<210> 369
   <211> 15
   <212> PRT
   <213> Egg peptide
<400> 369
   Met Glu Glu Arg Lys Ile Lys Val Tyr Leu Pro Arg Met Lys Met
   1 5 10 15
<210> 370
   <211> 15
   <212> PRT
   <213> Egg peptide
<400> 370
   Arg Lys Ile Lys Val Tyr Leu Pro Arg Met Lys Met Glu Glu Lys
   1 5 10 15
<210> 371
   <211> 15
   <212> PRT
   <213> Egg peptide
<400> 371
   Lys Val Tyr Leu Pro Arg Met Lys Met Glu Glu Lys Tyr Asn Leu
   1 5 10 15
<210> 372
   <211> 15
   <212> PRT
   <213> Egg peptide
<400> 372
   Leu Pro Arg Met Lys Met Glu Glu Lys Tyr Asn Leu Thr Ser Val
   1 5 10 15
<210> 373
   <211> 15
   <212> PRT
   <213> Egg peptide
<400> 373
   Met Lys Met Glu Glu Lys Tyr Asn Leu Thr Ser Val Leu Met Ala
   1 5 10 15
<210> 374
   <211> 15
   <212> PRT
   <213> Egg peptide
<400> 374
   Glu Glu Lys Tyr Asn Leu Thr Ser Val Leu Met Ala Met Gly Ile
   1 5 10 15
<210> 375
   <211> 15
   <212> PRT
   <213> Egg peptide
<400> 375
   Tyr Asn Leu Thr Ser Val Leu Met Ala Met Gly Ile Thr Asp Val
   1 5 10 15
<210> 376
   <211> 15
   <212> PRT
   <213> Egg peptide
<400> 376
   Thr Ser Val Leu Met Ala Met Gly Ile Thr Asp Val Phe Ser Ser
   1 5 10 15
<210> 377
   <211> 15
   <212> PRT
   <213> Egg peptide
<400> 377
   Leu Met Ala Met Gly Ile Thr Asp Val Phe Ser Ser Ser Ala Asn
   1 5 10 15
<210> 378
   <211> 15
   <212> PRT
   <213> Egg peptide
<400> 378
   Met Gly Ile Thr Asp Val Phe Ser Ser Ser Ala Asn Leu Ser Gly
   1 5 10 15
<210> 379
   <211> 15
   <212> PRT
   <213> Egg peptide
<400> 379
   Thr Asp Val Phe Ser Ser Ser Ala Asn Leu Ser Gly Ile Ser Ser
   1 5 10 15
<210> 380
   <211> 15
   <212> PRT
   <213> Egg peptide
<400> 380
   Phe Ser Ser Ser Ala Asn Leu Ser Gly Ile Ser Ser Ala Glu Ser
   1 5 10 15
<210> 381
   <211> 15
   <212> PRT
   <213> Egg peptide
<400> 381
   Ser Ala Asn Leu Ser Gly Ile Ser Ser Ala Glu Ser Leu Lys Ile
   1 5 10 15
<210> 382
   <211> 15
   <212> PRT
   <213> Egg peptide
<400> 382
   Leu Ser Gly Ile Ser Ser Ala Glu Ser Leu Lys Ile Ser Gln Ala
   1 5 10 15
<210> 383
   <211> 15
   <212> PRT
   <213> Egg peptide
<400> 383
   Ile Ser Ser Ala Glu Ser Leu Lys Ile Ser Gln Ala Val His Ala
   1 5 10 15
<210> 384
   <211> 15
   <212> PRT
   <213> Egg peptide
<400> 384
   Ala Glu Ser Leu Lys Ile Ser Gln Ala Val His Ala Ala His Ala
   1 5 10 15
<210> 385
   <211> 15
   <212> PRT
   <213> Egg peptide
<400> 385
   Leu Lys Ile Ser Gln Ala Val His Ala Ala His Ala Glu Ile Asn
   1 5 10 15
<210> 386
   <211> 15
   <212> PRT
   <213> Egg peptide
<400> 386
   Ser Gln Ala Val His Ala Ala His Ala Glu Ile Asn Glu Ala Gly
   1 5 10 15
<210> 387
   <211> 15
   <212> PRT
   <213> Egg peptide
<400> 387
   Val His Ala Ala His Ala Glu Ile Asn Glu Ala Gly Arg Glu Val
   1 5 10 15
<210> 388
   <211> 15
   <212> PRT
   <213> Egg peptide
<400> 388
   Ala His Ala Glu Ile Asn Glu Ala Gly Arg Glu Val Val Gly Ser
   1 5 10 15
<210> 389
   <211> 15
   <212> PRT
   <213> Egg peptide
<400> 389
   Glu Ile Asn Glu Ala Gly Arg Glu Val Val Gly Ser Ala Glu Ala
   1               5                   10                  15
<210> 390
   <211> 15
   <212> PRT
   <213> Egg peptide
<400> 390
   Glu Ala Gly Arg Glu Val Val Gly Ser Ala Glu Ala Gly Val Asp
   1               5                   10                  15
<210> 391
   <211> 15
   <212> PRT
   <213> Egg peptide
<400> 391
   Arg Glu Val Val Gly Ser Ala Glu Ala Gly Val Asp Ala Ala Ser
   1               5                   10                  15
<210> 392
   <211> 15
   <212> PRT
   <213> Egg peptide
<400> 392
   Val Gly Ser Ala Glu Ala Gly Val Asp Ala Ala Ser Val Ser Glu
   1               5                   10                  15
<210> 393
   <211> 15
   <212> PRT
   <213> Egg peptide
<400> 393
   Ala Glu Ala Gly Val Asp Ala Ala Ser Val Ser Glu Glu Phe Arg
   1               5                   10                  15
<210> 394
   <211> 15
   <212> PRT
   <213> Egg peptide
<400> 394
   Gly Val Asp Ala Ala Ser Val Ser Glu Glu Phe Arg Ala Asp His
   1               5                   10                  15
<210> 395
   <211> 15
   <212> PRT
   <213> Egg peptide
<400> 395
   Ala Ala Ser Val Ser Glu Glu Phe Arg Ala Asp His Pro Phe Leu
   1               5                   10                  15
<210> 396
   <211> 15
   <212> PRT
   <213> Egg peptide
<400> 396
   Val Ser Glu Glu Phe Arg Ala Asp His Pro Phe Leu Phe Cys Ile
   1               5                   10                  15
<210> 397
   <211> 15
   <212> PRT
   <213> Egg peptide
<400> 397
   Glu Phe Arg Ala Asp His Pro Phe Leu Phe Cys Ile Lys His Ile
   1               5                   10                  15
<210> 398
   <211> 15
   <212> PRT
   <213> Egg peptide
<400> 398
   Ala Asp His Pro Phe Leu Phe Cys Ile Lys His Ile Ala Thr Asn
   1               5                   10                  15
<210> 399
   <211> 15
   <212> PRT
   <213> Egg peptide
<400> 399
   Pro Phe Leu Phe Cys Ile Lys His Ile Ala Thr Asn Ala Val Leu
   1               5                   10                  15
<210> 400
   <211> 15
   <212> PRT
   <213> Egg peptide
<400> 400
   Phe Cys Ile Lys His Ile Ala Thr Asn Ala Val Leu Phe Phe Gly
   1               5                   10                  15
<210> 401
   <211> 15
   <212> PRT
   <213> Egg peptide
<400> 401
   Lys His Ile Ala Thr Asn Ala Val Leu Phe Phe Gly Arg Cys Val
   1 5 10 15
<210> 402
   <211> 14
   <212> PRT
   <213> Egg peptide
<400> 402
   Ala Thr Asn Ala Val Leu Phe Phe Gly Arg Cys Val Ser Pro
   1 5 10
<210> 403
   <211> 15
   <212> PRT
   <213> Egg peptide
<400> 403
   Ala Glu Val Asp Cys Ser Arg Phe Pro Asn Ala Thr Asp Lys Glu
   1 5 10 15
<210> 404
   <211> 15
   <212> PRT
   <213> Egg peptide
<400> 404
   Asp Cys Ser Arg Phe Pro Asn Ala Thr Asp Lys Glu Gly Lys Asp
   1 5 10 15
<210> 405
   <211> 15
   <212> PRT
   <213> Egg peptide
<400> 405
   Arg Phe Pro Asn Ala Thr Asp Lys Glu Gly Lys Asp Val Leu Val
   1 5 10 15
<210> 406
   <211> 15
   <212> PRT
   <213> Egg peptide
<400> 406
   Asn Ala Thr Asp Lys Glu Gly Lys Asp Val Leu Val Cys Asn Lys
   1               5                   10                  15
<210> 407
   <211> 15
   <212> PRT
   <213> Egg peptide
<400> 407
   Asp Lys Glu Gly Lys Asp Val Leu Val Cys Asn Lys Asp Leu Arg
   1               5                   10                  15
<210> 408
   <211> 15
   <212> PRT
   <213> Egg peptide
<400> 408
   Gly Lys Asp Val Leu Val Cys Asn Lys Asp Leu Arg Pro Ile Cys
   1               5                   10                  15
<210> 409
   <211> 15
   <212> PRT
   <213> Egg peptide
<400> 409
   Val Leu Val Cys Asn Lys Asp Leu Arg Pro Ile Cys Gly Thr Asp
   1               5                   10                  15
<210> 410
   <211> 15
   <212> PRT
   <213> Egg peptide
<400> 410
   Cys Asn Lys Asp Leu Arg Pro Ile Cys Gly Thr Asp Gly Val Thr
   1               5                   10                  15
<210> 411
   <211> 15
   <212> PRT
   <213> Egg peptide
<400> 411
   Asp Leu Arg Pro Ile Cys Gly Thr Asp Gly Val Thr Tyr Thr Asn
   1               5                   10                  15
<210> 412
   <211> 15
   <212> PRT
   <213> Egg peptide
<400> 412
   Pro Ile Cys Gly Thr Asp Gly Val Thr Tyr Thr Asn Asp Cys Leu
   1               5                   10                  15
<210> 413
   <211> 15
   <212> PRT
   <213> Egg peptide
<400> 413
   Gly Thr Asp Gly Val Thr Tyr Thr Asn Asp Cys Leu Leu Cys Ala
   1               5                   10                  15
<210> **414**
   <211> 15
   <212> PRT
   <213> Egg peptide
<400> 414
   Gly Val Thr Tyr Thr Asn Asp Cys Leu Leu Cys Ala Tyr Ser Ile
   1               5                   10                  15
<210> 415
   <211> 15
   <212> PRT
   <213> Egg peptide
<400> 415
   Tyr Thr Asn Asp Cys Leu Leu Cys Ala Tyr Ser Ile Glu Phe Gly
   1               5                   10                  15
<210> 416
   <211> 15
   <212> PRT
   <213> Egg peptide
<400> 416
   Asp Cys Leu Leu Cys Ala Tyr Ser Ile Glu Phe Gly Thr Asn Ile
   1               5                   10                  15
<210> 417
   <211> 15
   <212> PRT
   <213> Egg peptide
<400> 417
   Leu Cys Ala Tyr Ser Ile Glu Phe Gly Thr Asn Ile Ser Lys Glu
   1               5                   10                  15
<210> 418
   <211> 15
   <212> PRT
   <213> Egg peptide
<400> 418
   Tyr Ser Ile Glu Phe Gly Thr Asn Ile Ser Lys Glu His Asp Gly
   1 5 10 15
<210> 419
   <211> 15
   <212> PRT
   <213> Egg peptide
<400> 419
   Glu Phe Gly Thr Asn Ile Ser Lys Glu His Asp Gly Glu Cys Lys
   1 5 10 15
<210> 420
   <211> 15
   <212> PRT
   <213> Egg peptide
<400> 420
   Thr Asn Ile Ser Lys Glu His Asp Gly Glu Cys Lys Glu Thr Val
   1 5 10 15
<210> 421
   <211> 15
   <212> PRT
   <213> Egg peptide
<400> 421
   Ser Lys Glu His Asp Gly Glu Cys Lys Glu Thr Val Pro Met Asn
   1 5 10 15
<210> 422
   <211> 15
   <212> PRT
   <213> Egg peptide
<400> 422
   His Asp Gly Glu Cys Lys Glu Thr Val Pro Met Asn Cys Ser Ser
   1 5 10 15
<210> 423
   <211> 15
   <212> PRT
   <213> Egg peptide
<400> 423
   Glu Cys Lys Glu Thr Val Pro Met Asn Cys Ser Ser Tyr Ala Asn
   1 5 10 15
<210> 424
   <211> 15
   <212> PRT
   <213> Egg peptide
<400> 424
   Glu Thr Val Pro Met Asn Cys Ser Ser Tyr Ala Asn Thr Thr Ser
   1 5 10 15
<210> 425
   <211> 15
   <212> PRT
   <213> Egg peptide
<400> 425
   Pro Met Asn Cys Ser Ser Tyr Ala Asn Thr Thr Ser Glu Asp Gly
   1 5 10 15
<210> 426
   <211> 15
   <212> PRT
   <213> Egg peptide
<400> 426
   Cys Ser Ser Tyr Ala Asn Thr Thr Ser Glu Asp Gly Lys Val Met
   1 5 10 15
<210> 427
   <211> 15
   <212> PRT
   <213> Egg peptide
<400> 427
   Tyr Ala Asn Thr Thr Ser Glu Asp Gly Lys Val Met Val Leu Cys
   1 5 10 15
<210> 428
   <211> 15
   <212> PRT
   <213> Egg peptide
<400> 428
   Thr Thr Ser Glu Asp Gly Lys Val Met Val Leu Cys Asn Arg Ala
   1 5 10 15
<210> 429
   <211> 15
   <212> PRT
   <213> Egg peptide
<400> 429
   Glu Asp Gly Lys Val Met Val Leu Cys Asn Arg Ala Phe Asn Pro
   1 5 10 15
<210> 430
   <211> 15
   <212> PRT
   <213> Egg peptide
<400> 430
   Lys Val Met Val Leu Cys Asn Arg Ala Phe Asn Pro Val Cys Gly
   1 5 10 15
<210> 431
   <211> 15
   <212> PRT
   <213> Egg peptide
<400> 431
   Val Leu Cys Asn Arg Ala Phe Asn Pro Val Cys Gly Thr Asp Gly
   1 5 10 15
<210> 432
   <211> 15
   <212> PRT
   <213> Egg peptide
<400> 432
   Asn Arg Ala Phe Asn Pro Val Cys Gly Thr Asp Gly Val Thr Tyr
   1 5 10 15
<210> 433
   <211> 15
   <212> PRT
   <213> Egg peptide
<400> 433
   Phe Asn Pro Val Cys Gly Thr Asp Gly Val Thr Tyr Asp Asn Glu
   1 5 10 15
<210> 434
   <211> 15
   <212> PRT
   <213> Egg peptide
<400> 434
   Val Cys Gly Thr Asp Gly Val Thr Tyr Asp Asn Glu Cys Leu Leu
   1 5 10 15
<210> 435
   <211> 15
   <212> PRT
   <213> Egg peptide
<400> 435
   Thr Asp Gly Val Thr Tyr Asp Asn Glu Cys Leu Leu Cys Ala His
   1 5 10 15
<210> 436
   <211> 15
   <212> PRT
   <213> Egg peptide
<400> 436
   Val Thr Tyr Asp Asn Glu Cys Leu Leu Cys Ala His Lys Val Glu
   1 5 10 15
<210> 437
   <211> 15
   <212> PRT
   <213> Egg peptide
<400> 437
   Asp Asn Glu Cys Leu Leu Cys Ala His Lys Val Glu Gln Gly Ala
   1 5 10 15
<210> 438
   <211> 15
   <212> PRT
   <213> Egg peptide
<400> 438
   Cys Leu Leu Cys Ala His Lys Val Glu Gln Gly Ala Ser Val Asp
   1 5 10 15
<210> 439
   <211> 15
   <212> PRT
   <213> Egg peptide
<400> 439
   Cys Ala His Lys Val Glu Gln Gly Ala Ser Val Asp Lys Arg His
   1 5 10 15
<210> 440
   <211> 15
   <212> PRT
   <213> Egg peptide
<400> 440
   Lys Val Glu Gln Gly Ala Ser Val Asp Lys Arg His Asp Gly Gly
   1 5 10 15
<210> 441
   <211> 15
   <212> PRT
   <213> Egg peptide
<400> 441
   Gln Gly Ala Ser Val Asp Lys Arg His Asp Gly Gly Cys Arg Lys
   1 5 10 15
<210> 442
   <211> 15
   <212> PRT
   <213> Egg peptide
<400> 442
   Ser Val Asp Lys Arg His Asp Gly Gly Cys Arg Lys Glu Leu Ala
   1 5 10 15
<210> 443
   <211> 15
   <212> PRT
   <213> Egg peptide
<400> 443
   Lys Arg His Asp Gly Gly Cys Arg Lys Glu Leu Ala Ala Val Ser
   1 5 10 15
<210> 444
   <211> 15
   <212> PRT
   <213> Egg peptide
<400> 444
   Asp Gly Gly Cys Arg Lys Glu Leu Ala Ala Val Ser Val Asp Cys
   1 5 10 15
<210> 445
   <211> 15
   <212> PRT
   <213> Egg peptide
<400> 445
   Cys Arg Lys Glu Leu Ala Ala Val Ser Val Asp Cys Ser Glu Tyr
   1 5 10 15
<210> 446
   <211> 15
   <212> PRT
   <213> Egg peptide
<400> 446
   Glu Leu Ala Ala Val Ser Val Asp Cys Ser Glu Tyr Pro Lys Pro
   1 5 10 15
<210> 447
   <211> 15
   <212> PRT
   <213> Egg peptide
<400> 447
   Ala Val Ser Val Asp Cys Ser Glu Tyr Pro Lys Pro Asp Cys Thr
   1 5 10 15
<210> 448
   <211> 15
   <212> PRT
   <213> Egg peptide
<400> 448
   Val Asp Cys Ser Glu Tyr Pro Lys Pro Asp Cys Thr Ala Glu Asp
   1 5 10 15
<210> 449
   <211> 15
   <212> PRT
   <213> Egg peptide
<400> 449
   Ser Glu Tyr Pro Lys Pro Asp Cys Thr Ala Glu Asp Arg Pro Leu
   1 5 10 15
<210> 450
   <211> 15
   <212> PRT
   <213> Egg peptide
<400> 450
   Pro Lys Pro Asp Cys Thr Ala Glu Asp Arg Pro Leu Cys Gly Ser
   1 5 10 15
<210> 451
   <211> 15
   <212> PRT
   <213> Egg peptide
<400> 451
   Asp Cys Thr Ala Glu Asp Arg Pro Leu Cys Gly Ser Asp Asn Lys
   1 5 10 15
<210> 452
   <211> 15
   <212> PRT
   <213> Egg peptide
<400> 452
   Ala Glu Asp Arg Pro Leu Cys Gly Ser Asp Asn Lys Thr Tyr Gly
   1               5                   10                  15
<210> 453
   <211> 15
   <212> PRT
   <213> Egg peptide
<400> 453
   Arg Pro Leu Cys Gly Ser Asp Asn Lys Thr Tyr Gly Asn Lys Cys
   1               5                   10                  15
<210> 454
   <211> 15
   <212> PRT
   <213> Egg peptide
<400> 454
   Cys Gly Ser Asp Asn Lys Thr Tyr Gly Asn Lys Cys Asn Phe Cys
   1               5                   10                  15
<210> 455
   <211> 15
   <212> PRT
   <213> Egg peptide
<400> 455
   Asp Asn Lys Thr Tyr Gly Asn Lys Cys Asn Phe Cys Asn Ala Val
   1               5                   10                  15
<210> 456
   <211> 15
   <212> PRT
   <213> Egg peptide
<400> 456
   Thr Tyr Gly Asn Lys Cys Asn Phe Cys Asn Ala Val Val Glu Ser
   1               5                   10                  15
<210> 457
   <211> 15
   <212> PRT
   <213> Egg peptide
<400> 457
   Asn Lys Cys Asn Phe Cys Asn Ala Val Val Glu Ser Asn Gly Thr
   1               5                   10                  15
<210> 458
   <211> 15
   <212> PRT
   <213> Egg peptide
<400> 458
   Asn Phe Cys Asn Ala Val Val Glu Ser Asn Gly Thr Leu Thr Leu
   1 5 10 15
<210> 459
   <211> 15
   <212> PRT
   <213> Egg peptide
<400> 459
   Asn Ala Val Val Glu Ser Asn Gly Thr Leu Thr Leu Ser His Phe
   1 5 10 15
<210> 460
   <211> 15
   <212> PRT
   <213> Egg peptide
<400> 460
   Val Glu Ser Asn Gly Thr Leu Thr Leu Ser His Phe Gly Lys Cys
   1 5 10 15
<210> 461
   <211> 15
   <212> PRT
   <213> Shrimp peptide
<400> 461
   Met Ala Asp Ala Ala Val Ile Glu Lys Leu Glu Ala Gly Phe Lys
   1 5 10 15
<210> 462
   <211> 15
   <212> PRT
   <213> Shrimp peptide
<400> 462
   Val Ile Glu Lys Leu Glu Ala Gly Phe Lys Lys Leu Glu Ala Ala
   1 5 10 15
<210> 463
   <211> 15
   <212> PRT
   <213> Shrimp peptide
<400> 463
   Glu Ala Gly Phe Lys Lys Leu Glu Ala Ala Thr Asp Cys Lys Ser
   1 5 10 15
<210> 464
   <211> 15
   <212> PRT
   <213> Shrimp peptide
<400> 464
   Lys Leu Glu Ala Ala Thr Asp Cys Lys Ser Leu Leu Lys Lys Tyr
   1 5 10 15
<210> 465
   <211> 15
   <212> PRT
   <213> Shrimp peptide
<400> 465
   Thr Asp Cys Lys Ser Leu Leu Lys Lys Tyr Leu Thr Lys Glu Val
   1 5 10 15
<210> 466
   <211> 15
   <212> PRT
   <213> Shrimp peptide
<400> 466
   Leu Leu Lys Lys Tyr Leu Thr Lys Glu Val Phe Asp Lys Leu Lys
   1 5 10 15
<210> 467
   <211> 15
   <212> PRT
   <213> Shrimp peptide
<400> 467
   Leu Leu Lys Lys Tyr Leu Thr Lys Glu Val Phe Asp Lys Leu Lys
   1 5 10 15
<210> 468
   <211> 15
   <212> PRT
   <213> Shrimp peptide
<400> 468
   Phe Asp Lys Leu Lys Asp Lys Lys Thr Ser Leu Gly Ala Thr Leu
   1 5 10 15
<210> 469
   <211> 15
   <212> PRT
   <213> Shrimp peptide
<400> 469
   Asp Lys Lys Thr Ser Leu Gly Ala Thr Leu Leu Asp Val Ile Gln
   1 5 10 15
<210> 470
   <211> 15
   <212> PRT
   <213> Shrimp peptide
<400> 470
   Leu Gly Ala Thr Leu Leu Asp Val Ile Gln Ser Gly Val Glu Asn
   1               5                   10                  15
<210> 471
   <211> 15
   <212> PRT
   <213> Shrimp peptide
<400> 471
   Leu Asp Val Ile Gln Ser Gly Val Glu Asn Leu Asp Ser Gly Val
   1               5                   10                  15
<210> 472
   <211> 15
   <212> PRT
   <213> Shrimp peptide
<400> 472
   Ser Gly Val Glu Asn Leu Asp Ser Gly Val Gly Ile Tyr Ala Pro
   1               5                   10                  15
<210> 473
   <211> 15
   <212> PRT
   <213> Shrimp peptide
<400> 473
   Leu Asp Ser Gly Val Gly Ile Tyr Ala Pro Asp Ala Glu Ala Tyr
   1               5                   10                  15
<210> 474
   <211> 15
   <212> PRT
   <213> Shrimp peptide
<400> 474
   Gly Ile Tyr Ala Pro Asp Ala Glu Ala Tyr Thr Leu Phe Ala Pro
   1               5                   10                  15
<210> 475
   <211> 15
   <212> PRT
   <213> Shrimp peptide
<400> 475
   Asp Ala Glu Ala Tyr Thr Leu Phe Ala Pro Leu Phe Asp Pro Ile
   1 5 10 15
<210> 476
   <211> 15
   <212> PRT
   <213> Shrimp peptide
<400> 476
   Thr Leu Phe Ala Pro Leu Phe Asp Pro Ile Ile Glu Asp Tyr His
   1 5 10 15
<210> 477
   <211> 15
   <212> PRT
   <213> Shrimp peptide
<400> 477
   Leu Phe Asp Pro Ile Ile Glu Asp Tyr His Val Gly Phe Lys Gln
   1 5 10 15
<210> 478
   <211> 15
   <212> PRT
   <213> Shrimp peptide
<400> 478
   Ile Glu Asp Tyr His Val Gly Phe Lys Gln Thr Asp Lys His Pro
   1 5 10 15
<210> 479
   <211> 15
   <212> PRT
   <213> Shrimp peptide
<400> 479
   Val Gly Phe Lys Gln Thr Asp Lys His Pro Asn Lys Asp Phe Gly
   1 5 10 15
<210> 480
   <211> 15
   <212> PRT
   <213> Shrimp peptide
<400> 480
   Thr Asp Lys His Pro Asn Lys Asp Phe Gly Asp Val Asn Ser Phe
   1 5 10 15
<210> 481
   <211> 15
   <212> PRT
   <213> Shrimp peptide
<400> 481
   Asn Lys Asp Phe Gly Asp Val Asn Ser Phe Val Asn Val Asp Pro
   1 5 10 15
<210> 482
   <211> 15
   <212> PRT
   <213> Shrimp peptide
<400> 482
   Asp Val Asn Ser Phe Val Asn Val Asp Pro Glu Gly Lys Phe Val
   1 5 10 15
<210> 483
   <211> 15
   <212> PRT
   <213> Shrimp peptide
<400> 483
   Val Asn Val Asp Pro Glu Gly Lys Phe Val Ile Ser Thr Arg Val
   1 5 10 15
<210> 484
   <211> 15
   <212> PRT
   <213> Shrimp peptide
<400> 484
   Glu Gly Lys Phe Val Ile Ser Thr Arg Val Arg Cys Gly Arg Ser
   1 5 10 15
<210> 485
   <211> 15
   <212> PRT
   <213> Shrimp peptide
<400> 485
   Ile Ser Thr Arg Val Arg Cys Gly Arg Ser Met Gln Gly Tyr Pro
   1 5 10 15
<210> 486
   <211> 15
   <212> PRT
   <213> Shrimp peptide
<400> 486
   Arg Cys Gly Arg Ser Met Gln Gly Tyr Pro Phe Asn Pro Cys Leu
   1 5 10 15
<210> 487
   <211> 15
   <212> PRT
   <213> Shrimp peptide
<400> 487
   Met Gln Gly Tyr Pro Phe Asn Pro Cys Leu Thr Glu Ser Gln Tyr
   1 5 10 15
<210> 488
   <211> 15
   <212> PRT
   <213> Shrimp peptide
<400> 488
   Phe Asn Pro Cys Leu Thr Glu Ser Gln Tyr Lys Glu Met Glu Ala
   1 5 10 15
<210> 489
   <211> 15
   <212> PRT
   <213> Shrimp peptide
<400> 489
   Thr Glu Ser Gln Tyr Lys Glu Met Glu Ala Lys Val Ser Ser Thr
   1 5 10 15
<210> 490
   <211> 15
   <212> PRT
   <213> Shrimp peptide
<400> 490
   Lys Glu Met Glu Ala Lys Val Ser Ser Thr Leu Ser Ser Leu Glu
   1 5 10 15
<210> 491
   <211> 15
   <212> PRT
   <213> Shrimp peptide
<400> 491
   Lys Val Ser Ser Thr Leu Ser Ser Leu Glu Gly Glu Leu Lys Gly
   1 5 10 15
<210> 492
   <211> 15
   <212> PRT
   <213> Shrimp peptide
<400> 492
   Leu Ser Ser Leu Glu Gly Glu Leu Lys Gly Thr Tyr Tyr Pro Leu
   1 5 10 15
<210> 493
   <211> 15
   <212> PRT
   <213> Shrimp peptide
<400> 493
   Gly Glu Leu Lys Gly Thr Tyr Tyr Pro Leu Thr Gly Met Ser Lys
   1 5 10 15
<210> 494
   <211> 15
   <212> PRT
   <213> Shrimp peptide
<400> 494
   Thr Tyr Tyr Pro Leu Thr Gly Met Ser Lys Glu Val Gln Gln Lys
   1 5 10 15
<210> 495
   <211> 15
   <212> PRT
   <213> Shrimp peptide
<400> 495
   Thr Gly Met Ser Lys Glu Val Gln Gln Lys Leu Ile Asp Asp His
   1 5 10 15
<210> 496
   <211> 15
   <212> PRT
   <213> Shrimp peptide
<400> 496
   Glu Val Gln Gln Lys Leu Ile Asp Asp His Phe Leu Phe Lys Glu
   1 5 10 15
<210> 497
   <211> 15
   <212> PRT
   <213> Shrimp peptide
<400> 497
   Leu Ile Asp Asp His Phe Leu Phe Lys Glu Gly Asp Arg Phe Leu
   1 5 10 15
<210> 498
   <211> 15
   <212> PRT
   <213> Shrimp peptide
<400> 498
   Phe Leu Phe Lys Glu Gly Asp Arg Phe Leu Gln Ala Ala Asn Ala
   1 5 10 15
<210> 499
   <211> 15
   <212> PRT
   <213> Shrimp peptide
<400> 499
   Gly Asp Arg Phe Leu Gln Ala Ala Asn Ala Cys Arg Tyr Trp Pro
   1               5                   10                  15
<210> 500
   <211> 15
   <212> PRT
   <213> Shrimp peptide
<400> 500
   Gln Ala Ala Asn Ala Cys Arg Tyr Trp Pro Ala Gly Arg Gly Ile
   1               5                   10                  15
<210> 501
   <211> 15
   <212> PRT
   <213> Shrimp peptide
<400> 501
   Cys Arg Tyr Trp Pro Ala Gly Arg Gly Ile Tyr His Asn Asp Asn
   1               5                   10                  15
<210> 502
   <211> 15
   <212> PRT
   <213> Shrimp peptide
<400> 502
   Ala Gly Arg Gly Ile Tyr His Asn Asp Asn Lys Thr Phe Leu Val
   1               5                   10                  15
<210> 503
   <211> 15
   <212> PRT
   <213> Shrimp peptide
<400> 503
   Tyr His Asn Asp Asn Lys Thr Phe Leu Val Trp Val Asn Glu Glu
   1               5                   10                  15
<210> 504
   <211> 15
   <212> PRT
   <213> Shrimp peptide
<400> 504
   Lys Thr Phe Leu Val Trp Val Asn Glu Glu Asp His Leu Arg Ile
   1               5                   10                  15
<210> 505
   <211> 15
   <212> PRT
   <213> Shrimp peptide
<400> 505
   Trp Val Asn Glu Glu Asp His Leu Arg Ile Ile Ser Met Gln Met
   1               5                   10                  15
<210> 506
   <211> 15
   <212> PRT
   <213> Shrimp peptide
<400> 506
   Asp His Leu Arg Ile Ile Ser Met Gln Met Gly Gly Asp Leu Gly
   1               5                   10                  15
<210> 507
   <211> 15
   <212> PRT
   <213> Shrimp peptide
<400> 507
   Ile Ser Met Gln Met Gly Gly Asp Leu Gly Gln Val Phe Arg Arg
   1               5                   10                  15
<210> 508
   <211> 15
   <212> PRT
   <213> Shrimp peptide
<400> 508
   Gly Gly Asp Leu Gly Gln Val Phe Arg Arg Leu Thr Ser Ala Val
   1               5                   10                  15
<210> 509
   <211> 15
   <212> PRT
   <213> Shrimp peptide
<400> 509
   Gln Val Phe Arg Arg Leu Thr Ser Ala Val Asn Glu Ile Glu Lys
   1               5                   10                  15
<210> 510
   <211> 15
   <212> PRT
   <213> Shrimp peptide
<400> 510
   Leu Thr Ser Ala Val Asn Glu Ile Glu Lys Arg Ile Pro Phe Ser
   1 5 10 15
<210> 511
   <211> 15
   <212> PRT
   <213> Shrimp peptide
<400> 511
   Asn Glu Ile Glu Lys Arg Ile Pro Phe Ser His His Asp Arg Leu
   1 5 10 15
<210> 512
   <211> 15
   <212> PRT
   <213> Shrimp peptide
<400> 512
   Arg Ile Pro Phe Ser His His Asp Arg Leu Gly Phe Leu Thr Phe
   1 5 10 15
<210> 513
   <211> 15
   <212> PRT
   <213> Shrimp peptide
<400> 513
   His His Asp Arg Leu Gly Phe Leu Thr Phe Cys Pro Thr Asn Leu
   1 5 10 15
<210> 514
   <211> 15
   <212> PRT
   <213> Shrimp peptide
<400> 514
   Gly Phe Leu Thr Phe Cys Pro Thr Asn Leu Gly Thr Thr Val Arg
   1 5 10 15
<210> 515
   <211> 15
   <212> PRT
   <213> Shrimp peptide
<400> 515
   Cys Pro Thr Asn Leu Gly Thr Thr Val Arg Ala Ser Val His Ile
   1 5 10 15
<210> 516
   <211> 15
   <212> PRT
   <213> Shrimp peptide
<400> 516
   Gly Thr Thr Val Arg Ala Ser Val His Ile Lys Leu Pro Lys Leu
   1 5 10 15
<210> 517
   <211> 15
   <212> PRT
   <213> Shrimp peptide
<400> 517
   Ala Ser Val His Ile Lys Leu Pro Lys Leu Ala Ala Asn Arg Glu
   1 5 10 15
<210> 518
   <211> 15
   <212> PRT
   <213> Shrimp peptide
<400> 518
   Lys Leu Pro Lys Leu Ala Ala Asn Arg Glu Lys Leu Glu Glu Val
   1 5 10 15
<210> 519
   <211> 15
   <212> PRT
   <213> Shrimp peptide
<400> 519
   Ala Ala Asn Arg Glu Lys Leu Glu Glu Val Ala Gly Lys Tyr Asn
   1 5 10 15
<210> 520
   <211> 15
   <212> PRT
   <213> Shrimp peptide
<400> 520
   Lys Leu Glu Glu Val Ala Gly Lys Tyr Asn Leu Gln Val Arg Gly
   1 5 10 15
<210> 521
   <211> 15
   <212> PRT
   <213> Shrimp peptide
<400> 521
   Ala Gly Lys Tyr Asn Leu Gln Val Arg Gly Thr Arg Gly Glu His
   1 5 10 15
<210> 522
   <211> 15
   <212> PRT
   <213> Shrimp peptide
<400> 522
   Leu Gln Val Arg Gly Thr Arg Gly Glu His Thr Glu Ala Glu Gly
   1 5 10 15
<210> 523
   <211> 15
   <212> PRT
   <213> Shrimp peptide
<400> 523
   Thr Arg Gly Glu His Thr Glu Ala Glu Gly Gly Ile Tyr Asp Ile
   1 5 10 15
<210> 524
   <211> 15
   <212> PRT
   <213> Shrimp peptide
<400> 524
   Thr Glu Ala Glu Gly Gly Ile Tyr Asp Ile Ser Asn Lys Arg Arg
   1 5 10 15
<210> 525
   <211> 15
   <212> PRT
   <213> Shrimp peptide
<400> 525
   Gly Ile Tyr Asp Ile Ser Asn Lys Arg Arg Met Gly Leu Thr Glu
   1 5 10 15
<210> 526
   <211> 15
   <212> PRT
   <213> Shrimp peptide
<400> 526
   Ser Asn Lys Arg Arg Met Gly Leu Thr Glu Phe Gln Ala Val Lys
   1 5 10 15
<210> 527
   <211> 15
   <212> PRT
   <213> Shrimp peptide
<400> 527
   Met Gly Leu Thr Glu Phe Gln Ala Val Lys Glu Met Gln Asp Gly
   1 5 10 15
<210> 528
   <211> 15
   <212> PRT
   <213> Shrimp peptide
<400> 528
   Phe Gln Ala Val Lys Glu Met Gln Asp Gly Ile Leu Glu Leu Ile
   1               5                   10                  15
<210> 529
   <211> 15
   <212> PRT
   <213> Shrimp peptide
<400> 529
   Glu Met Gln Asp Gly Ile Leu Glu Leu Ile Lys Ile Glu Lys Glu
   1               5                   10                  15
<210> 530
   <211> 15
   <212> PRT
   <213> Shrimp peptide
<400> 530
   Met Ser Arg Lys Ser Gly Ser Arg Ser Ser Ser Lys Arg Ser Lys
   1               5                   10                  15
<210> 531
   <211> 15
   <212> PRT
   <213> Shrimp peptide
<400> 531
   Gly Ser Arg Ser Ser Ser Lys Arg Ser Lys Lys Ser Gly Gly Gly
   1               5                   10                  15
<210> 532
   <211> 15
   <212> PRT
   <213> Shrimp peptide
<400> 532
   Ser Lys Arg Ser Lys Lys Ser Gly Gly Gly Ser Asn Val Phe Asp
   1               5                   10                  15
<210> 533
   <211> 15
   <212> PRT
   <213> Shrimp peptide
<400> 533
   Lys Ser Gly Gly Gly Ser Asn Val Phe Asp Met Phe Thr Gln Arg
   1 5 10 15
<210> 534
   <211> 15
   <212> PRT
   <213> Shrimp peptide
<400> 534
   Ser Asn Val Phe Asp Met Phe Thr Gln Arg Gln Val Ala Glu Phe
   1 5 10 15
<210> 535
   <211> 15
   <212> PRT
   <213> Shrimp peptide
<400> 535
   Met Phe Thr Gln Arg Gln Val Ala Glu Phe Lys Glu Gly Phe Gln
   1 5 10 15
<210> 536
   <211> 15
   <212> PRT
   <213> Shrimp peptide
<400> 536
   Gln Val Ala Glu Phe Lys Glu Gly Phe Gln Leu Met Asp Arg Asp
   1 5 10 15
<210> 537
   <211> 15
   <212> PRT
   <213> Shrimp peptide
<400> 537
   Lys Glu Gly Phe Gln Leu Met Asp Arg Asp Lys Asp Gly Val Ile
   1 5 10 15
<210> 538
   <211> 15
   <212> PRT
   <213> Shrimp peptide
<400> 538
   Leu Met Asp Arg Asp Lys Asp Gly Val Ile Gly Lys Thr Asp Leu
   1 5 10 15
<210> 539
   <211> 15
   <212> PRT
   <213> Shrimp peptide
<400> 539
   Lys Asp Gly Val Ile Gly Lys Thr Asp Leu Arg Gly Thr Phe Asp
   1 5 10 15
<210> 540
   <211> 15
   <212> PRT
   <213> Shrimp peptide
<400> 540
   Gly Lys Thr Asp Leu Arg Gly Thr Phe Asp Glu Ile Gly Arg Ile
   1 5 10 15
<210> 541
   <211> 15
   <212> PRT
   <213> Shrimp peptide
<400> 541
   Arg Gly Thr Phe Asp Glu Ile Gly Arg Ile Ala Thr Asp Gln Glu
   1 5 10 15
<210> 542
   <211> 15
   <212> PRT
   <213> Shrimp peptide
<400> 542
   Glu Ile Gly Arg Ile Ala Thr Asp Gln Glu Leu Asp Glu Met Leu
   1 5 10 15
<210> 543
   <211> 15
   <212> PRT
   <213> Shrimp peptide
<400> 543
   Ala Thr Asp Gln Glu Leu Asp Glu Met Leu Ala Asp Ala Pro Ala
   1 5 10 15
<210> 544
   <211> 15
   <212> PRT
   <213> Shrimp peptide
<400> 544
   Leu Asp Glu Met Leu Ala Asp Ala Pro Ala Pro Ile Asn Phe Thr
   1 5 10 15
<210> 545
   <211> 15
   <212> PRT
   <213> Shrimp peptide
<400> 545
   Ala Asp Ala Pro Ala Pro Ile Asn Phe Thr Met Leu Leu Asn Met
   1               5                   10                  15
<210> 546
   <211> 15
   <212> PRT
   <213> Shrimp peptide
<400> 546
   Pro Ile Asn Phe Thr Met Leu Leu Asn Met Phe Ala Glu Arg Gln
   1               5                   10                  15
<210> 547
   <211> 15
   <212> PRT
   <213> Shrimp peptide
<400> 547
   Met Leu Leu Asn Met Phe Ala Glu Arg Gln Thr Gly Glu Ser Asp
   1               5                   10                  15
<210> 548
   <211> 15
   <212> PRT
   <213> Shrimp peptide
<400> 548
   Phe Ala Glu Arg Gln Thr Gly Glu Ser Asp Asp Asp Asp Val Val
   1               5                   10                  15
<210> 549
   <211> 15
   <212> PRT
   <213> Shrimp peptide
<400> 549
   Thr Gly Glu Ser Asp Asp Asp Asp Val Val Ala Lys Ala Phe Leu
   1               5                   10                  15
<210> 550
   <211> 15
   <212> PRT
   <213> Shrimp peptide
<400> 550
   Asp Asp Asp Val Val Ala Lys Ala Phe Leu Ala Phe Ala Asp Glu
   1 5 10 15
<210> 551
   <211> 15
   <212> PRT
   <213> Shrimp peptide
<400> 551
   Ala Lys Ala Phe Leu Ala Phe Ala Asp Glu Glu Gly Asn Ile Asp
   1 5 10 15
<210> 552
   <211> 15
   <212> PRT
   <213> Shrimp peptide
<400> 552
   Ala Phe Ala Asp Glu Glu Gly Asn Ile Asp Cys Asp Thr Phe Arg
   1 5 10 15
<210> 553
   <211> 15
   <212> PRT
   <213> Shrimp peptide
<400> 553
   Glu Gly Asn Ile Asp Cys Asp Thr Phe Arg His Ala Leu Met Thr
   1 5 10 15
<210> 554
   <211> 15
   <212> PRT
   <213> Shrimp peptide
<400> 554
   Cys Asp Thr Phe Arg His Ala Leu Met Thr Trp Gly Asp Lys Phe
   1 5 10 15
<210> 555
   <211> 15
   <212> PRT
   <213> Shrimp peptide
<400> 555
   His Ala Leu Met Thr Trp Gly Asp Lys Phe Ser Ser Gln Glu Ala
   1 5 10 15
<210> 556
   <211> 15
   <212> PRT
   <213> Shrimp peptide
<400> 556
   Trp Gly Asp Lys Phe Ser Ser Gln Glu Ala Asp Asp Ala Leu Asp
   1               5                   10                  15
<210> 557
   <211> 15
   <212> PRT
   <213> Shrimp peptide
<400> 557
   Ser Ser Gln Glu Ala Asp Asp Ala Leu Asp Gln Met Asp Ile Asp
   1               5                   10                  15
<210> 558
   <211> 15
   <212> PRT
   <213> Shrimp peptide
<400> 558
   Asp Asp Ala Leu Asp Gln Met Asp Ile Asp Asp Gly Gly Lys Ile
   1               5                   10                  15
<210> 559
   <211> 15
   <212> PRT
   <213> Shrimp peptide
<400> 559
   Gln Met Asp Ile Asp Asp Gly Gly Lys Ile Asp Val Gln Gly Val
   1               5                   10                  15
<210> 560
   <211> 15
   <212> PRT
   <213> Shrimp peptide
<400> 560
   Asp Gly Gly Lys Ile Asp Val Gln Gly Val Ile Gln Met Leu Thr
   1               5                   10                  15
<210> 561
   <211> 15
   <212> PRT
   <213> Shrimp peptide
<400> 561
   Asp Val Gln Gly Val Ile Gln Met Leu Thr Ala Gly Gly Gly Asp
   1               5                   10                  15
<210> 562
   <211> 15
   <212> PRT
   <213> Shrimp peptide
<400> 562
   Ile Gln Met Leu Thr Ala Gly Gly Gly Asp Asp Ala Ala Ala Glu
   1 5 10 15
<210> 563
   <211> 12
   <212> PRT
   <213> Shrimp peptide
<400> 563
   Ala Gly Gly Gly Asp Asp Ala Ala Ala Glu Glu Ala
   1 5 10
<210> 564
   <211> 15
   <212> PRT
   <213> Shrimp peptide
<400> 564
   Met Ala Tyr Ser Trp Asp Asn Arg Val Lys Tyr Val Val Arg Tyr
   1 5 10 15
<210> 565
   <211> 15
   <212> PRT
   <213> Shrimp peptide
<400> 565
   Asp Asn Arg Val Lys Tyr Val Val Arg Tyr Met Tyr Asp Ile Asp
   1 5 10 15
<210> 566
   <211> 15
   <212> PRT
   <213> Shrimp peptide
<400> 566
   Tyr Val Val Arg Tyr Met Tyr Asp Ile Asp Asn Asn Gly Phe Leu
   1 5 10 15
<210> 567
   <211> 15
   <212> PRT
   <213> Shrimp peptide
<400> 567
   Met Tyr Asp Ile Asp Asn Asn Gly Phe Leu Asp Lys Asn Asp Phe
   1 5 10 15
<210> 568
   <211> 15
   <212> PRT
   <213> Shrimp peptide
<400> 568
   Asn Asn Gly Phe Leu Asp Lys Asn Asp Phe Glu Cys Leu Ala Val
   1               5                   10                  15
<210> 569
   <211> 15
   <212> PRT
   <213> Shrimp peptide
<400> 569
   Asp Lys Asn Asp Phe Glu Cys Leu Ala Val Arg Asn Thr Leu Ile
   1               5                   10                  15
<210> 570
   <211> 15
   <212> PRT
   <213> Shrimp peptide
<400> 570
   Glu Cys Leu Ala Val Arg Asn Thr Leu Ile Glu Gly Arg Gly Glu
   1               5                   10                  15
<210> 571
   <211> 15
   <212> PRT
   <213> Shrimp peptide
<400> 571
   Arg Asn Thr Leu Ile Glu Gly Arg Gly Glu Phe Ser Ala Asp Ala
   1               5                   10                  15
<210> 572
   <211> 15
   <212> PRT
   <213> Shrimp peptide
<400> 572
   Glu Gly Arg Gly Glu Phe Ser Ala Asp Ala Tyr Ala Asn Asn Gln
   1               5                   10                  15
<210> 573
   <211> 15
   <212> PRT
   <213> Shrimp peptide
<400> 573
   Phe Ser Ala Asp Ala Tyr Ala Asn Asn Gln Lys Ile Met Arg Asn
   1               5                   10                  15
<210> 574
   <211> 15
   <212> PRT
   <213> Shrimp peptide
<400> 574
   Tyr Ala Asn Asn Gln Lys Ile Met Arg Asn Leu Trp Asn Glu Ile
   1 5 10 15
<210> 575
   <211> 15
   <212> PRT
   <213> Shrimp peptide
<400> 575
   Lys Ile Met Arg Asn Leu Trp Asn Glu Ile Ala Glu Leu Ala Asp
   1 5 10 15
<210> 576
   <211> 15
   <212> PRT
   <213> Shrimp peptide
<400> 576
   Leu Trp Asn Glu Ile Ala Glu Leu Ala Asp Phe Asn Lys Asp Gly
   1 5 10 15
<210> 577
   <211> 15
   <212> PRT
   <213> Shrimp peptide
<400> 577
   Ala Glu Leu Ala Asp Phe Asn Lys Asp Gly Glu Val Thr Val Asp
   1 5 10 15
<210> 578
   <211> 15
   <212> PRT
   <213> Shrimp peptide
<400> 578
   Phe Asn Lys Asp Gly Glu Val Thr Val Asp Glu Phe Lys Gln Ala
   1 5 10 15
<210> 579
   <211> 15
   <212> PRT
   <213> Shrimp peptide
<400> 579
   Glu Val Thr Val Asp Glu Phe Lys Gln Ala Val Gln Lys His Cys
   1 5 10 15
<210> 580
   <211> 15
   <212> PRT
   <213> Shrimp peptide
<400> 580
   Glu Phe Lys Gln Ala Val Gln Lys His Cys Gln Gly Lys Lys Tyr
   1 5 10 15
<210> 581
   <211> 15
   <212> PRT
   <213> Shrimp peptide
<400> 581
   Val Gln Lys His Cys Gln Gly Lys Lys Tyr Gly Asp Phe Pro Gly
   1 5 10 15
<210> 582
   <211> 15
   <212> PRT
   <213> Shrimp peptide
<400> 582
   Gln Gly Lys Lys Tyr Gly Asp Phe Pro Gly Ala Phe Lys Val Phe
   1 5 10 15
<210> 583
   <211> 15
   <212> PRT
   <213> Shrimp peptide
<400> 583
   Gly Asp Phe Pro Gly Ala Phe Lys Val Phe Ile Ala Asn Gln Phe
   1 5 10 15
<210> 584
   <211> 15
   <212> PRT
   <213> Shrimp peptide
<400> 584
   Ala Phe Lys Val Phe Ile Ala Asn Gln Phe Lys Ala Ile Asp Val
   1 5 10 15
<210> 585
   <211> 15
   <212> PRT
   <213> Shrimp peptide
<400> 585
   Ile Ala Asn Gln Phe Lys Ala Ile Asp Val Asn Gly Asp Gly Lys
   1 5 10 15
<210> 586
   <211> 15
   <212> PRT
   <213> Shrimp peptide
<400> 586
   Lys Ala Ile Asp Val Asn Gly Asp Gly Lys Val Gly Leu Asp Glu
   1 5 10 15
<210> 587
   <211> 15
   <212> PRT
   <213> Shrimp peptide
<400> 587
   Asn Gly Asp Gly Lys Val Gly Leu Asp Glu Tyr Arg Leu Asp Cys
   1 5 10 15
<210> 588
   <211> 15
   <212> PRT
   <213> Shrimp peptide
<400> 588
   Val Gly Leu Asp Glu Tyr Arg Leu Asp Cys Ile Thr Arg Ser Ala
   1 5 10 15
<210> 589
   <211> 15
   <212> PRT
   <213> Shrimp peptide
<400> 589
   Tyr Arg Leu Asp Cys Ile Thr Arg Ser Ala Phe Ala Glu Val Lys
   1 5 10 15
<210> 590
   <211> 15
   <212> PRT
   <213> Shrimp peptide
<400> 590
   Ile Thr Arg Ser Ala Phe Ala Glu Val Lys Glu Ile Asp Asp Ala
   1 5 10 15
<210> 591
   <211> 15
   <212> PRT
   <213> Shrimp peptide
<400> 591
   Phe Ala Glu Val Lys Glu Ile Asp Asp Ala Tyr Asn Lys Leu Thr
   1               5                   10                  15
<210> 592
   <211> 15
   <212> PRT
   <213> Shrimp peptide
<400> 592
   Glu Ile Asp Asp Ala Tyr Asn Lys Leu Thr Thr Glu Asp Asp Arg
   1               5                   10                  15
<210> 593
   <211> 15
   <212> PRT
   <213> Shrimp peptide
<400> 593
   Tyr Asn Lys Leu Thr Thr Glu Asp Asp Arg Lys Ala Gly Gly Leu
   1               5                   10                  15
<210> 594
   <211> 15
   <212> PRT
   <213> Shrimp peptide
<400> 594
   Thr Glu Asp Asp Arg Lys Ala Gly Gly Leu Thr Leu Glu Arg Tyr
   1               5                   10                  15
<210> 595
   <211> 15
   <212> PRT
   <213> Shrimp peptide
<400> 595
   Lys Ala Gly Gly Leu Thr Leu Glu Arg Tyr Gln Asp Leu Tyr Ala
   1               5                   10                  15
<210> 596
   <211> 15
   <212> PRT
   <213> Shrimp peptide
<400> 596
   Thr Leu Glu Arg Tyr Gln Asp Leu Tyr Ala Gln Phe Ile Ser Asn
   1               5                   10                  15
<210> 597
   <211> 15
   <212> PRT
   <213> Shrimp peptide
<400> 597
   Gln Asp Leu Tyr Ala Gln Phe Ile Ser Asn Pro Asp Glu Ser Cys
   1 5 10 15
<210> 598
   <211> 15
   <212> PRT
   <213> Shrimp peptide
<400> 598
   Gln Phe Ile Ser Asn Pro Asp Glu Ser Cys Ser Ala Cys Tyr Leu
   1 5 10 15
<210> 599
   <211> 15
   <212> PRT
   <213> Shrimp peptide
<400> 599
   Pro Asp Glu Ser Cys Ser Ala Cys Tyr Leu Phe Gly Pro Leu Lys
   1 5 10 15
<210> 600
   <211> 13
   <212> PRT
   <213> Shrimp peptide
<400> 600
   Ser Ala Cys Tyr Leu Phe Gly Pro Leu Lys Val Val Gln
   1 5 10
<210> 601
   <211> 15
   <212> PRT
   <213> Shrimp peptide
<400> 601
   Met Asp Ala Ile Lys Lys Lys Met Gln Ala Met Lys Leu Glu Lys
   1 5 10 15
<210> 602
   <211> 15
   <212> PRT
   <213> Shrimp peptide
<400> 602
   Lys Lys Met Gln Ala Met Lys Leu Glu Lys Asp Asn Ala Met Asp
   1 5 10 15
<210> 603
   <211> 15
   <212> PRT
   <213> Shrimp peptide
<400> 603
   Met Lys Leu Glu Lys Asp Asn Ala Met Asp Arg Ala Asp Thr Leu
   1 5 10 15
<210> 604
   <211> 15
   <212> PRT
   <213> Shrimp peptide
<400> 604
   Asp Asn Ala Met Asp Arg Ala Asp Thr Leu Glu Gln Gln Asn Lys
   1 5 10 15
<210> 605
   <211> 15
   <212> PRT
   <213> Shrimp peptide
<400> 605
   Arg Ala Asp Thr Leu Glu Gln Gln Asn Lys Glu Ala Asn Asn Arg
   1 5 10 15
<210> 606
   <211> 15
   <212> PRT
   <213> Shrimp peptide
<400> 606
   Glu Gln Gln Asn Lys Glu Ala Asn Asn Arg Ala Glu Lys Ser Glu
   1 5 10 15
<210> 607
   <211> 15
   <212> PRT
   <213> Shrimp peptide
<400> 607
   Glu Ala Asn Asn Arg Ala Glu Lys Ser Glu Glu Glu Val His Asn
   1 5 10 15
<210> 608
   <211> 15
   <212> PRT
   <213> Shrimp peptide
<400> 608
   Ala Glu Lys Ser Glu Glu Glu Val His Asn Leu Gln Lys Arg Met
   1 5 10 15
<210> 609
   <211> 15
   <212> PRT
   <213> Shrimp peptide
<400> 609
   Glu Glu Val His Asn Leu Gln Lys Arg Met Gln Gln Leu Glu Asn
   1 5 10 15
<210> 610
   <211> 15
   <212> PRT
   <213> Shrimp peptide
<400> 610
   Leu Gln Lys Arg Met Gln Gln Leu Glu Asn Asp Leu Asp Gln Val
   1 5 10 15
<210> 611
   <211> 15
   <212> PRT
   <213> Shrimp peptide
<400> 611
   Gln Gln Leu Glu Asn Asp Leu Asp Gln Val Gln Glu Ser Leu Leu
   1 5 10 15
<210> 612
   <211> 15
   <212> PRT
   <213> Shrimp peptide
<400> 612
   Asp Leu Asp Gln Val Gln Glu Ser Leu Leu Lys Ala Asn Ile Gln
   1 5 10 15
<210> 613
   <211> 15
   <212> PRT
   <213> Shrimp peptide
<400> 613
   Gln Glu Ser Leu Leu Lys Ala Asn Ile Gln Leu Val Glu Lys Asp
   1 5 10 15
<210> 614
   <211> 15
   <212> PRT
   <213> Shrimp peptide
<400> 614
   Lys Ala Asn Ile Gln Leu Val Glu Lys Asp Lys Ala Leu Ser Asn
   1 5 10 15
<210> 615
   <211> 15
   <212> PRT
   <213> Shrimp peptide
<400> 615
   Leu Val Glu Lys Asp Lys Ala Leu Ser Asn Ala Glu Gly Glu Val
   1 5 10 15
<210> 616
   <211> 15
   <212> PRT
   <213> Shrimp peptide
<400> 616
   Lys Ala Leu Ser Asn Ala Glu Gly Glu Val Ala Ala Leu Asn Arg
   1 5 10 15
<210> 617
   <211> 15
   <212> PRT
   <213> Shrimp peptide
<400> 617
   Ala Glu Gly Glu Val Ala Ala Leu Asn Arg Arg Ile Gln Leu Leu
   1 5 10 15
<210> 618
   <211> 15
   <212> PRT
   <213> Shrimp peptide
<400> 618
   Ala Ala Leu Asn Arg Arg Ile Gln Leu Leu Glu Glu Asp Leu Glu
   1 5 10 15
<210> 619
   <211> 15
   <212> PRT
   <213> Shrimp peptide
<400> 619
   Arg Ile Gln Leu Leu Glu Glu Asp Leu Glu Arg Ser Glu Glu Arg
   1 5 10 15
<210> 620
   <211> 15
   <212> PRT
   <213> Shrimp peptide
<400> 620
   Glu Glu Asp Leu Glu Arg Ser Glu Glu Arg Leu Asn Thr Ala Thr
   1               5                   10                  15
<210> 621
   <211> 15
   <212> PRT
   <213> Shrimp peptide
<400> 621
   Arg Ser Glu Glu Arg Leu Asn Thr Ala Thr Thr Lys Leu Ala Glu
   1               5                   10                  15
<210> 622
   <211> 15
   <212> PRT
   <213> Shrimp peptide
<400> 622
   Leu Asn Thr Ala Thr Thr Lys Leu Ala Glu Ala Ser Gln Ala Ala
   1               5                   10                  15
<210> 623
   <211> 15
   <212> PRT
   <213> Shrimp peptide
<400> 623
   Thr Lys Leu Ala Glu Ala Ser Gln Ala Ala Asp Glu Ser Glu Arg
   1               5                   10                  15
<210> 624
   <211> 15
   <212> PRT
   <213> Shrimp peptide
<400> 624
   Ala Ser Gln Ala Ala Asp Glu Ser Glu Arg Met Arg Lys Val Leu
   1               5                   10                  15
<210> 625
   <211> 15
   <212> PRT
   <213> Shrimp peptide
<400> 625
   Asp Glu Ser Glu Arg Met Arg Lys Val Leu Glu Asn Arg Ser Leu
   1               5                   10                  15
<210> 626
   <211> 15
   <212> PRT
   <213> Shrimp peptide
<400> 626
   Met Arg Lys Val Leu Glu Asn Arg Ser Leu Ser Asp Glu Glu Arg
   1 5 10 15
<210> 627
   <211> 15
   <212> PRT
   <213> Shrimp peptide
<400> 627
   Glu Asn Arg Ser Leu Ser Asp Glu Glu Arg Met Asp Ala Leu Glu
   1 5 10 15
<210> 628
   <211> 15
   <212> PRT
   <213> Shrimp peptide
<400> 628
   Ser Asp Glu Glu Arg Met Asp Ala Leu Glu Asn Gln Leu Lys Glu
   1 5 10 15
<210> 629
   <211> 15
   <212> PRT
   <213> Shrimp peptide
<400> 629
   Met Asp Ala Leu Glu Asn Gln Leu Lys Glu Ala Arg Phe Leu Ala
   1 5 10 15
<210> 630
   <211> 15
   <212> PRT
   <213> Shrimp peptide
<400> 630
   Asn Gln Leu Lys Glu Ala Arg Phe Leu Ala Glu Glu Ala Asp Arg
   1 5 10 15
<210> 631
   <211> 15
   <212> PRT
   <213> Shrimp peptide
<400> 631
   Ala Arg Phe Leu Ala Glu Glu Ala Asp Arg Lys Tyr Asp Glu Val
   1 5 10 15
<210> 632
   <211> 15
   <212> PRT
   <213> Shrimp peptide
<400> 632
   Glu Glu Ala Asp Arg Lys Tyr Asp Glu Val Ala Arg Lys Leu Ala
   1 5 10 15
<210> 633
   <211> 15
   <212> PRT
   <213> Shrimp peptide
<400> 633
   Lys Tyr Asp Glu Val Ala Arg Lys Leu Ala Met Val Glu Ala Asp
   1 5 10 15
<210> 634
   <211> 15
   <212> PRT
   <213> Shrimp peptide
<400> 634
   Ala Arg Lys Leu Ala Met Val Glu Ala Asp Leu Glu Arg Ala Glu
   1 5 10 15
<210> 635
   <211> 15
   <212> PRT
   <213> Shrimp peptide
<400> 635
   Met Val Glu Ala Asp Leu Glu Arg Ala Glu Glu Arg Ala Glu Thr
   1 5 10 15
<210> 636
   <211> 15
   <212> PRT
   <213> Shrimp peptide
<400> 636
   Leu Glu Arg Ala Glu Glu Arg Ala Glu Thr Gly Glu Ser Lys Ile
   1 5 10 15
<210> 637
   <211> 15
   <212> PRT
   <213> Shrimp peptide
<400> 637
   Glu Arg Ala Glu Thr Gly Glu Ser Lys Ile Val Glu Leu Glu Glu
   1               5                   10                  15
<210> 638
   <211> 15
   <212> PRT
   <213> Shrimp peptide
<400> 638
   Gly Glu Ser Lys Ile Val Glu Leu Glu Glu Glu Leu Arg Val Val
   1               5                   10                  15
<210> 639
   <211> 15
   <212> PRT
   <213> Shrimp peptide
<400> 639
   Val Glu Leu Glu Glu Glu Leu Arg Val Val Gly Asn Asn Leu Lys
   1               5                   10                  15
<210> 640
   <211> 15
   <212> PRT
   <213> Shrimp peptide
<400> 640
   Glu Leu Arg Val Val Gly Asn Asn Leu Lys Ser Leu Glu Val Ser
   1               5                   10                  15
<210> 641
   <211> 15
   <212> PRT
   <213> Shrimp peptide
<400> 641
   Gly Asn Asn Leu Lys Ser Leu Glu Val Ser Glu Glu Lys Ala Asn
   1               5                   10                  15
<210> 642
   <211> 15
   <212> PRT
   <213> Shrimp peptide
<400> 642
   Ser Leu Glu Val Ser Glu Glu Lys Ala Asn Gln Arg Glu Glu Ala
   1               5                   10                  15
<210> 643
   <211> 15
   <212> PRT
   <213> Shrimp peptide
<400> 643
   Glu Glu Lys Ala Asn Gln Arg Glu Glu Ala Tyr Lys Glu Gln Ile
   1               5                   10                  15
<210> 644
   <211> 15
   <212> PRT
   <213> Shrimp peptide
<400> 644
   Gln Arg Glu Glu Ala Tyr Lys Glu Gln Ile Lys Thr Leu Thr Asn
   1               5                   10                  15
<210> 645
   <211> 15
   <212> PRT
   <213> Shrimp peptide
<400> 645
   Tyr Lys Glu Gln Ile Lys Thr Leu Thr Asn Lys Leu Lys Ala Ala
   1               5                   10                  15
<210> 646
   <211> 15
   <212> PRT
   <213> Shrimp peptide
<400> 646
   Lys Thr Leu Thr Asn Lys Leu Lys Ala Ala Glu Ala Arg Ala Glu
   1               5                   10                  15
<210> 647
   <211> 15
   <212> PRT
   <213> Shrimp peptide
<400> 647
   Lys Leu Lys Ala Ala Glu Ala Arg Ala Glu Phe Ala Glu Arg Ser
   1               5                   10                  15
<210> 648
   <211> 15
   <212> PRT
   <213> Shrimp peptide
<400> 648
   Glu Ala Arg Ala Glu Phe Ala Glu Arg Ser Val Gln Lys Leu Gln
   1               5                   10                  15
<210> 649
   <211> 15
   <212> PRT
   <213> Shrimp peptide
<400> 649
   Phe Ala Glu Arg Ser Val Gln Lys Leu Gln Lys Glu Val Asp Arg
   1               5                   10                  15
<210> 650
   <211> 15
   <212> PRT
   <213> Shrimp peptide
<400> 650
   Val Gln Lys Leu Gln Lys Glu Val Asp Arg Leu Glu Asp Glu Leu
   1               5                   10                  15
<210> 651
   <211> 15
   <212> PRT
   <213> Shrimp peptide
<400> 651
   Lys Glu Val Asp Arg Leu Glu Asp Glu Leu Val Asn Glu Lys Glu
   1               5                   10                  15
<210> 652
   <211> 15
   <212> PRT
   <213> Shrimp peptide
<400> 652
   Leu Glu Asp Glu Leu Val Asn Glu Lys Glu Lys Tyr Lys Ser Ile
   1               5                   10                  15
<210> 653
   <211> 15
   <212> PRT
   <213> Shrimp peptide
<400> 653
   Val Asn Glu Lys Glu Lys Tyr Lys Ser Ile Thr Asp Glu Leu Asp
   1               5                   10                  15
<210> 654
   <211> 15
   <212> PRT
   <213> Shrimp peptide
<400> 654
   Lys Tyr Lys Ser Ile Thr Asp Glu Leu Asp Gln Thr Phe Ser Glu
   1               5                   10                  15
<210> 655
   <211> 14
   <212> PRT
   <213> Shrimp peptide
<400> 655
   Thr Asp Glu Leu Asp Gln Thr Phe Ser Glu Leu Ser Gly Tyr
   1               5                   10
<210> 656
   <211> 15
   <212> PRT
   <213> Shrimp peptide
<400> 656
   Met Asp Ser Leu Asp Glu Glu Gln Ile Glu Thr Leu Arg Lys Ala
   1               5                   10                  15
<210> 657
   <211> 15
   <212> PRT
   <213> Shrimp peptide
<400> 657
   Glu Glu Gln Ile Glu Thr Leu Arg Lys Ala Phe Asp Ser Phe Asp
   1               5                   10                  15
<210> 658
   <211> 15
   <212> PRT
   <213> Shrimp peptide
<400> 658
   Thr Leu Arg Lys Ala Phe Asp Ser Phe Asp Thr Glu Lys Thr Gly
   1               5                   10                  15
<210> 659
   <211> 15
   <212> PRT
   <213> Shrimp peptide
<400> 659
   Phe Asp Ser Phe Asp Thr Glu Lys Thr Gly Ser Ile Thr Ala Glu
   1               5                   10                  15
<210> 660
   <211> 15
   <212> PRT
   <213> Shrimp peptide
<400> 660
   Thr Glu Lys Thr Gly Ser Ile Thr Ala Glu Thr Ile Ala Thr Ile
   1 5 10 15
<210> 661
   <211> 15
   <212> PRT
   <213> Shrimp peptide
<400> 661
   Ser Ile Thr Ala Glu Thr Ile Ala Thr Ile Met Arg Met Met Gly
   1 5 10 15
<210> 662
   <211> 15
   <212> PRT
   <213> Shrimp peptide
<400> 662
   Thr Ile Ala Thr Ile Met Arg Met Met Gly Val Lys Ile Ser Glu
   1 5 10 15
<210> 663
   <211> 15
   <212> PRT
   <213> Shrimp peptide
<400> 663
   Met Arg Met Met Gly Val Lys Ile Ser Glu Lys Asn Leu Gln Glu
   1 5 10 15
<210> 664
   <211> 15
   <212> PRT
   <213> Shrimp peptide
<400> 664
   Val Lys Ile Ser Glu Lys Asn Leu Gln Glu Ala Ile Ala Glu Thr
   1 5 10 15
<210> 665
   <211> 15
   <212> PRT
   <213> Shrimp peptide
<400> 665
   Lys Asn Leu Gln Glu Ala Ile Ala Glu Thr Asp Glu Asp Gly Ser
   1 5 10 15
<210> 666
   <211> 15
   <212> PRT
   <213> Shrimp peptide
<400> 666
   Ala Ile Ala Glu Thr Asp Glu Asp Gly Ser Gly Leu Leu Glu Phe
   1               5                   10                  15
<210> 667
   <211> 15
   <212> PRT
   <213> Shrimp peptide
<400> 667
   Asp Glu Asp Gly Ser Gly Leu Leu Glu Phe Glu Glu Phe Val Glu
   1               5                   10                  15
<210> 668
   <211> 15
   <212> PRT
   <213> Shrimp peptide
<400> 668
   Gly Leu Leu Glu Phe Glu Glu Phe Val Glu Leu Ser Ala Lys Phe
   1               5                   10                  15
<210> 669
   <211> 15
   <212> PRT
   <213> Shrimp peptide
<400> 669
   Glu Glu Phe Val Glu Leu Ser Ala Lys Phe Leu Ile Glu Glu Asp
   1               5                   10                  15
<210> 670
   <211> 15
   <212> PRT
   <213> Shrimp peptide
<400> 670
   Leu Ser Ala Lys Phe Leu Ile Glu Glu Asp Glu Glu Ala Leu Lys
   1               5                   10                  15
<210> 671
   <211> 15
   <212> PRT
   <213> Shrimp peptide
<400> 671
   Leu Ile Glu Glu Asp Glu Glu Ala Leu Lys Ala Glu Leu Arg Glu
   1               5                   10                  15
<210> 672
   <211> 15
   <212> PRT
   <213> Shrimp peptide
<400> 672
   Glu Glu Ala Leu Lys Ala Glu Leu Arg Glu Ala Phe Arg Ile Tyr
   1 5 10 15
<210> 673
   <211> 15
   <212> PRT
   <213> Shrimp peptide
<400> 673
   Ala Glu Leu Arg Glu Ala Phe Arg Ile Tyr Asp Lys Glu Gly Asn
   1 5 10 15
<210> 674
   <211> 15
   <212> PRT
   <213> Shrimp peptide
<400> 674
   Ala Phe Arg Ile Tyr Asp Lys Glu Gly Asn Gly Phe Ile Thr Thr
   1 5 10 15
<210> 675
   <211> 15
   <212> PRT
   <213> Shrimp peptide
<400> 675
   Asp Lys Glu Gly Asn Gly Phe Ile Thr Thr Asp Val Leu Lys Glu
   1 5 10 15
<210> 676
   <211> 15
   <212> PRT
   <213> Shrimp peptide
<400> 676
   Gly Phe Ile Thr Thr Asp Val Leu Lys Glu Ile Leu Ala Glu Leu
   1 5 10 15
<210> 677
   <211> 15
   <212> PRT
   <213> Shrimp peptide
<400> 677
   Asp Val Leu Lys Glu Ile Leu Ala Glu Leu Asp Pro Arg Leu Thr
   1               5                   10                  15
<210> 678
   <211> 15
   <212> PRT
   <213> Shrimp peptide
<400> 678
   Ile Leu Ala Glu Leu Asp Pro Arg Leu Thr Pro Ala Asp Leu Glu
   1               5                   10                  15
<210> 679
   <211> 15
   <212> PRT
   <213> Shrimp peptide
<400> 679
   Asp Pro Arg Leu Thr Pro Ala Asp Leu Glu Asn Ile Ile Glu Glu
   1               5                   10                  15
<210> 680
   <211> 15
   <212> PRT
   <213> Shrimp peptide
<400> 680
   Pro Ala Asp Leu Glu Asn Ile Ile Glu Glu Val Asp Glu Asp Gly
   1               5                   10                  15
<210> 681
   <211> 15
   <212> PRT
   <213> Shrimp peptide
<400> 681
   Asn Ile Ile Glu Glu Val Asp Glu Asp Gly Ser Gly Thr Leu Asp
   1               5                   10                  15
<210> 682
   <211> 15
   <212> PRT
   <213> Shrimp peptide
<400> 682
   Val Asp Glu Asp Gly Ser Gly Thr Leu Asp Phe Asp Glu Phe Met
   1               5                   10                  15
<210> 683
   <211> 15
   <212> PRT
   <213> Shrimp peptide
<400> 683
   Ser Gly Thr Leu Asp Phe Asp Glu Phe Met Glu Met Met Asn Gly
   1               5                   10                  15

## Description

### TECHNICAL FIELD

The invention relates to peptide biomarkers for diagnosis of allergy and for determining whether an allergic subject is likely to outgrow the allergy. The invention also relates to diagnostic methods and diagnostic kits employing the peptide biomarkers.

### BACKGROUND

Food allergies are a common problem among adults and children, and symptoms may range from mild oral pruritus to potentially life-threatening anaphylactic shock. Food allergies are currently diagnosed by skin prick testing or oral provocation, and measurement of serum levels of specific IgE and in some cases other serum antibodies, such as IgG4. These tests indicate the likelihood of clinical reactivity but do not distinguish the different phenotypes of food allergy or provide prognostic information. They also involve some level of risk to the patient. The relationship between current IgE testing and the actual clinical sensitivity of the patient is a weak one that is usually defined as a combination of reaction severity and the amount of allergen that provokes a reaction. Another limitation of current testing is the inability to determine whether or not pediatric patients will outgrow the allergy during childhood. In this case there is a positive but weak correlation between specific IgE level and the duration of clinical allergy.

More recently, it has been suggested that clinical reactivity to food allergens may correlate better with allergen-specific IgE on the epitope recognition level. It has been reported that patients with persistent or more severe allergic reactions recognize larger numbers of IgE epitopes, suggesting epitope mapping as an additional tool for allergy diagnosis and prediction. Spot membrane-based immunoassays have been used for epitope mapping. In this system, peptides are synthesized on the membrane and incubated with the patient's sera. The process requires a large number of peptides and is therefore error prone, time consuming, labor intensive, and expensive. Immunoassays in this format also require a large volume of patient serum.

Development of multiplex assay technologies, such as microarrays, and advances in peptide synthesis techniques have improved epitope mapping of food allergens. Immunoassays in microarray format can assay thousands of target peptides in parallel using small volumes of diluted serum, greatly reducing the cost and allowing for better replication and statistical approaches to the analysis. Unfortunately, the microarray-based test is not high through-put and it frequently requires multiple replicates to overcome limitations in reproducibility.

High-throughput assay formats have the advantage of rapidly processing multiple patient specimens in an automated fashion, and have been developed for application to multiplex screening methods. Bead-based multiplexing, such as the LUMINEX/xMAP technology, uses 5.6 µm polystyrene beads dyed with red and infrared fluorophores. Using different amounts of each of the two fluorophores, up to 500 different specific spectral signatures can be produced and theoretically up to 500 tests in a single reaction volume is possible. In the typical protein assay, antibodies are conjugated to the surface of the beads to capture the analyte of interest. Biotinylated detection antibodies specific to the analyte of interest are then bound to form an antibody-antigen sandwich. Interaction of biotin with a phycoerythrin-conjugated streptavidin (SA-PE) is used to label the complex. To detect the analyte, the beads are read on a dual-laser flow-based detection instrument. One laser classifies the bead according to the incorporated dyes and determines the analyte that is being detected. The second laser determines the magnitude of the PE-derived signal, which is in direct proportion to the amount of the bound analyte. Such assays reduce the amount of capture antibody and sample required compared to an ELISA plate assay, thus reducing the cost and conserving rare or difficult-to-obtain sample material. The dynamic range and sensitivity of the assay are also generally improved.

Cow's milk allergy (CMA) is one of the most common food allergies in children. It typically involves sensitivity to several of the component proteins of cow's milk. These include proteins in the casein fraction (αₛ₋₁-, αₛ₋₂-, β-, and κ-casein), α-lactalbumin and β-lactoglobulin. Both conformational and sequential epitopes can elicit antibody responses. Although the majority of children eventually outgrow their CMA (i.e., they become clinically tolerant), some retain their sensitivity into later life. The mechanisms contributing to development of clinical tolerance are not well understood, but it is hypothesized that IgE antibodies of those with persistent CMA may recognize certain epitopes of cow's milk proteins that are not recognized by IgE antibodies from patients who are likely to outgrow their allergy.

Analysis of epitopes, such as sequential epitope recognition, can provide useful information concerning persistence of CMA. Peptide microarray results have shown a correlation with clinical features of milk allergy, i.e., patients with milk allergy and milk-tolerant patients evidenced different epitope recognition patterns. It was also demonstrated that changes in the relative binding of IgE and IgG4 to milk peptides correlated with the presence of allergy or with clinical improvement.

MATSUMOTO et al., Peptides, (20090000), vol. 30, no. 10, pages 1840 - 1847 considers peptide array-based analysis of the specific IgE and IgG4 in cow's milk allergens and its use in allergy evaluation,

LIN et al., Journal of Allergy and Clinical Immunology, (20090000), vol. 124, no. 2, pages 315 - 322, relates to development of a novel peptide microarray for large-scale epitope mapping of food allergens,

WANG et al., Journal of Allergy and Clinical Immunology, (20100000), vol. 125, no. 3, pages 695 - 702 considers a correlation of IgE/IgG4 milk epitopes and affinity of milk-specific IgE antibodies with different phenotypes of clinical milk allergy,

WO2015/015043A1 method that makes it possible to form a prognosis concerning the number of reactions that will be produced during oral immunotherapy (OIT) against proteins in cow's milk and/or an estimate of the treatment time required to achieve tolerance or desensitization during OIT to proteins in cow's milk and/or the need for pre-medication during OIT in a human subject.

However, there remains a need to identify informative epitopes that are useful for diagnosing CMA and for predicting the clinical outcome of CMA. There also remains a need for new assay platforms that overcome the deficiencies of microarray immunoassays, and provide high-throughput, increased flexibility, reduced sample volume, and lower cost, with a similar workflow. The present invention addresses these needs.

### SUMMARY

The invention relates to peptides containing allergenic epitopes of cow's milk proteins that are useful for diagnosis of CMA, for detecting development of clinical tolerance to cow's milk proteins, and for monitoring increases and decreases in the intensity of the allergic response.

In a first embodiment, the invention provides a method for diagnosing a milk allergy in a subject comprising:
a) providing a plurality of peptides selected from among SEQ ID NOs:1-33, wherein the plurality of peptides consists of all 33 peptides of SEQ ID NOs:1-33, a subset of 20-25 peptides, a subset of 15-20 peptides, a subset of 10-15 peptides, a subset of 5-10 peptides, or a subset of 2-5 peptides, each peptide conjugated to a separately identifiable solid support;
b) contacting each solid support with serum obtained from the subject under conditions sufficient to permit binding of allergy associated immunoglobulin (AAI) in the serum to the peptide on each solid support to form a peptide-IgE complex;
c) binding an AAI-specific labeling reagent to the peptide-AAI complex; and
d) analyzing binding of the labeling reagent to each peptide-AAI complex to identify peptides recognized by the AAI in the serum of the subject;
wherein recognition of at least one peptide by AAI in the serum of the subject indicates that the subject is allergic to milk.

In one embodiment IgG and IgE are detected.

In one embodiment the IgG is IgG4.

In one embodiment the plurality of peptides is represented by SEQ ID NOs:1-9, SEQ ID NOs:10-15, SEQ ID NOs: 16-23, SEQ ID NOs:24-27, SEQ ID NOs:28-33, and combinations thereof.

The invention also provides a method for detecting development of clinical tolerance or an increase in intensity of allergy to milk in a subject that is allergic to milk comprising:
a) providing an initial profile of allergy associated immunoglobulin (AAI) reactivity of the subject's serum to a plurality of peptides selected from among SEQ ID NOs:1-33, wherein the plurality of peptides consists of all 33 peptides of SEQ ID NOs: 1-33, a subset of 20-25 peptides, a subset of 15-20 peptides, a subset of 10-15 peptides, a subset of 5-10 peptides, or a subset of 2-5 peptides, wherein the initial profile defines an initial number of peptides recognized by AAI in the serum of the subject or an initial concentration of AAI in the serum of the subject that recognizes each peptide;
b) providing the plurality of peptides each conjugated to a separately identifiable solid support;
c) contacting each solid support with serum obtained from the subject at a time-point subsequent to the initial profile under conditions sufficient to permit binding of AAI in the serum to the peptide on each solid support to form a peptide-AAI complex;
d) binding an AAI-specific labeling reagent to the peptide-AAI complex; and
e) analyzing binding of the labeling reagent to each peptide-AAI complex to identify a subsequent number of peptides recognized by AAI in the serum of the subject or a subsequent concentration of AAI in the serum of the subject that recognizes each peptide;
wherein development of clinical tolerance to milk is indicated when the subsequent number of peptides recognized by AAI in the serum of the subject is less than the initial number of peptides recognized by AAI in the serum of the subject, or when the subsequent concentration of AAI in the serum of the subject that recognizes at least one peptide is less than the initial concentration of AAI in the serum of the subject that recognizes the at least one peptide, and
wherein an increased intensity of the allergic response is indicated when the subsequent number of peptides recognized by AAI in the serum of the subject is greater than the initial number of peptides recognized by AAI in the serum of the subject, or when the subsequent concentration of AAI in the serum of the subject that recognizes at least one peptide is greater than the initial concentration of AAI in the serum of the subject that recognizes the at least one_peptide.

In one embodiment a pattern of quantitative reduction in AAI reactivity with selected peptides is used to predict development of clinical tolerance to the allergenic milk in the subject or a pattern of quantitatively increased AAI reactivity with selected peptides is used to predict increasing intensity of allergy to milk over time.

The invention also provides a set of allergenic epitope-containing peptides for detection of milk allergy consisting of a plurality of peptides represented by SEQ ID NOs:1-33, wherein the plurality of peptides consists of all 33 peptides of SEQ ID NOs:1-33, a subset of 15-20 peptides, a subset of 10-15 peptides or a subset of 5-10 peptides.

In a specific aspect of the first embodiment, the allergenic epitope-containing peptides are a plurality of peptides selected from the group consisting of allergenic The allergenic epitope-containing peptides are a plurality of peptides selected from among SEQ ID NOs:1-33:

| AlphaS-1 Casein Peptides: | | |
|---|---|---|
| alphaS 1-03 | KHQGLPQEVLNENLLRFFVA | SEQ ID NO:1 |
| alphaS 1-09 | VAPFPEVFGKEKVNELSKDI | SEQ ID NO:2 |
| alphaS 1-22 | SISSSEEIVPNSVEQKHIQK | SEQ ID NO:3 |
| alphaS 1-27 | KHIQKEDVPSERYLGYLEQL | SEQ ID NO:4 |
| alphaS 1-30 | SERYLGYLEQLLRLKKYKVP | SEQ ID NO:5 |
| alphaS 1-35 | KYKVPQLEIVPNSAEERLHS | SEQ ID NO:6 |
| alphaS 1-44 | QQKEPMIGVNQELAYFYPEL | SEQ ID NO:7 |
| alphaS 1-57 | LGTQYTDAPSFSDIPNPIGS | SEQ ID NO:8 |
| alphaS 1-61 | SDIPNPIGSENSEKTTMPLW | SEQ ID NO:9 |

| AlphaS-2 Casein Peptides: | | |
|---|---|---|
| alphaS2-08 | QEKNMAINPSKENLCSTFCK | SEQ ID NO:10 |
| alphaS2-13 | STFCKEWRNANEEEYSIGS | SEQ ID NO:11 |
| alphaS2-26 | KHYQKALNEINQFYQKFPQY | SEQ ID NO: 12 |
| alphaS2-33 | QYLYQGPIVLNPWDQVKRNA | SEQ ID NO:13 |
| alphaS2-56 | KISQRYQKFALPQYLKTVYQ | SEQ ID NO:14 |
| alphaS2-60 | QYLKTVYQHQKAMKPWIQPK | SEQ ID NO:15 |

| Beta-Casein Peptides: | | |
|---|---|---|
| betacas-01 | RELEELNVPGEIVESLSSSE | SEQ ID NO:16 |
| betacas-16 | QDKIHPFAQTQSLVYPFPGP | SEQ ID NO: 17 |
| betacas-18 | FAQTQSLVYPFPGPIPNSLP | SEQ ID NO:18 |
| betacas-25 | NIPPLTQTPVWPPFLQPEV | SEQ ID NO:19 |
| betacas-33 | KVKEAMAPKHKEMPFPKYPV | SEQ ID NO:20 |
| betacas-42 | SLTLTDVENLHLPLPLLQSW | SEQ ID NO:21 |
| betacas-53 | FPPQSVLSLSQSKVLPVPQK | SEQ ID NO:22 |
| betacas-58 | PVPQKAVPYPQRDMPIQAFL | SEQ ID NO:23 |

| Beta-Lactoalobulin Peptides: | | |
|---|---|---|
| betalac-14 | RVYVEELKPTPEGDLEILLQ | SEQ ID NO:24 |
| betalac-22 | DECAQKKIIAEKTKIPAVFK | SEQ ID NO:25 |
| betalac-41 | CLVRTPEVDDEALEKFDKAL | SEQ ID NO:26 |
| betalac-43 | EVDDEALEKFDKALKALPMH | SEQ ID NO:27 |

| Kappa Casein Peptides: | | |
|---|---|---|
| kappacas-04 | RCEKDERFFSDKIAKYIPIQ | SEQ ID NO:28 |
| kappacas-16 | KPVALINNQFLPYPYYAKPA | SEQ ID NO:29 |
| kappacas-36 | MAIPPKKNQDKTEIPTINTI | SEQ ID NO:30 |
| kappacas-44 | PTSTPTTEAVESTVATLEDS | SEQ ID NO:31 |
| kappacas-49 | TLEDSPEVIESPPEINTVQV | SEQ ID NO:32 |
| kappacas-21 | YAKPAAVRSPAQILQWQVLS | SEQ ID NO:33 |

Peptides useful in methods for diagnosis of CMA, for detecting development of clinical tolerance to cow's milk proteins, and for detecting increases and decreases in the intensity of the allergy may also include peptides containing non-reactive epitopes of cow's milk proteins. These peptides are useful as negative controls. In specific aspects the peptides containing negative control epitopes are one or more peptides selected from the group consisting of non-reactive peptide epitopes of αS2-casein, β-casein, and β-lactoglobulin. In a further specific embodiment, the non-reactive epitope-containing peptides are one or more peptides selected from the group consisting of:

| AlphaS-2 Peptides: | | |
|---|---|---|
| alphas2-42 | NREQLSTSEENSKKTVDMES | SEQ ID NO:34 |

| Beta-Casein Peptides: | | |
|---|---|---|
| betacas-09 | RINKKIEKFQSEEQQQTEDE | SEQ ID NO:35 |

| Beta-Lactoalobulin Peptides. | | |
|---|---|---|
| betalac-31 | KVLVLDTDYKKYLLVCMENS | SEQ ID NO:36 |

### BRIEF DESCRIPTION OF THE DRAWINGS

Fig. 1 illustrates serum reactivity over time to the peptide panel of SEQ ID NOs:1-33 of a cow's milk tolerant individual receiving immunotherapy for CMA.
Fig. 2 illustrates serum reactivity over time to the peptide panel of SEQ ID NOs:1-33 of an individual with CMA who became desensitized in response to immunotherapy for CMA.
Fig. 3 illustrates serum reactivity over time to the peptide panel of SEQ ID NOs:1-33 of an individual with CMA who partially responded to immunotherapy for CMA.

In the drawings, peptides identified beginning with "a" represent "alpha", peptides identified beginning with "b" represent "beta" and peptides identified as beginning with "k" represent "kappa."

### DETAILED DESCRIPTION

Before describing several exemplary embodiments of the invention, it is to be understood that the invention is not limited to the details of construction or process steps set forth in the following description. The invention is capable of other embodiments and of being practiced or being carried out in various ways.

Reference throughout this specification to "one embodiment," "certain embodiments," "one or more embodiments" or "an embodiment" means that a particular feature, structure, material, or characteristic described in connection with the embodiment is included in at least one embodiment of the invention. Thus, the appearances of the phrases such as "in one or more embodiments," "in certain embodiments," "in one embodiment" or "in an embodiment" in various places throughout this specification are not necessarily referring to the same embodiment of the invention. Furthermore, the particular features, structures, materials, or characteristics may be combined in any suitable manner in one or more embodiments.

As used herein, the terms "allergy associated immunoglobulin" and "AAI" refer to immunoglobulins in sera that mediate hypersensitivity to food allergens. These include one or more of IgE, IgA, and IgG (including IgG4).

As used herein, the terms "reactive", "reactivity", "recognize" and the like refer to the ability of an allergy associated immunoglobulin to bind to an allergenic epitope containing peptide. The level of reactivity indicates the concentration of AAI in the serum, with high reactivity associated with higher AAI concentrations and lower reactivity associated with lower AAI concentrations. The relative AAI concentration (i.e., the relative serum reactivity) is determined by the amount of signal detected in the assay. The level of reactivity of AAI to allergenic epitope containing peptides also indicates the intensity of the allergic response, i.e., higher reactivity is associated with a more intense allergic reaction.

As used herein, the term "clinical tolerance" refers to immunological tolerance to a food allergen that is developed by an allergic subject as a result of exposure to the allergen, i.e., tolerance developed as a result of immunotherapy.

As used herein, the term "natural tolerance" refers to immunological tolerance to a food allergen that is developed by an allergic subject as a biochemical process over time, either as a result of natural exposure to the allergen during a lifetime or in the absence of exposure.

It is to be understood that although the allergenic epitope-containing peptides disclosed herein are described as having specific amino acid sequence, one skilled in the art will recognize that each such peptide may be shifted in either the N-terminal or C-terminal direction of the protein from which it is derived to obtain a related peptide sequence that still contains the relevant epitope but in which the relevant epitope is flanked by different amino acids than specified.

The allergenic epitope-containing peptides represented by SEQ ID NOs:1-33 were identified in a library of peptides derived from αS1-casein, αS2-casein, β-casein, β-lactoglobulin, and κ-casein as having a z-score in highly allergic individuals of greater than 10. In highly allergic subjects, all thirty-three peptides of SEQ ID NOs:1-33 are reactive with sera. Conversely, in non-allergic subjects none of the thirty-three peptides of SEQ ID NOs:1-33 are reactive with sera. The individual peptides of SEQ ID NOs:1-33 also provide a continuum of reactivity which is useful for determining the intensity of CMA in an individual, and for monitoring changes in the intensity of CMA over time. Individuals having intensities of allergy to cow's milk that fall between non-reactive and the most highly reactive have sera that are reactive with some, but not all, of the peptides among SEQ ID NOs:1-33. In general, the number of peptides among SEQ ID NOs:1-33 that are reactive with the sera of these individuals is positively correlated with the intensity of the allergy, i.e., the more intense the allergy the more peptides among SEQ ID NOs:1-33 are reactive with the sera. The sera of individuals with mild allergy are reactive with fewer peptides than the sera of individuals with more intense allergy. The invention therefore not only provides methods for diagnosing CMA, it provides methods for determining the intensity of the allergy and methods for determining changes in the intensity of the allergy over time, including detection of development of clinical tolerance to cow's milk proteins.

In certain aspects of the invention, the number of allergenic epitope-containing peptides within the group of SEQ ID NOs:1-33 that are reactive with the sera of a CMA subject has a positive correlation with the intensity of the allergic response, i.e., reactivity with fewer peptides indicates a milder allergic response to cow's milk and reactivity with more peptides indicates the subject is more highly allergic to cow's milk. In another aspect of the invention, the intensity of binding of serum IgE to the peptides represented by SEQ ID NOs:1-33 (a measure of IgE concentration in the sera) correlates with the intensity of the allergic response, i.e., weaker reactivity with all thirty-three peptides, or with a subset of the thirty-three peptides, indicates a more moderate allergic response compared to stronger reactivity with all thirty-three peptides or with the subset of peptides. As used herein, reference to "non-reactive" or "negative" reactivity with an allergenic epitope-containing peptide means a signal-to-noise ratio (S/N) in the assay that is less than about 2. A typical background signal (N) is that generated by a pool of sera from non-allergenic individuals. Alternatively, the invention contemplates use of negative peptides as the basis for establishing the background signal. As used herein, reference to "weak" or "moderate" "moderate" reactivity with an allergenic epitope-containing peptide means a S/N of about 2-10, although this value may vary depending on the peptide and the allergy. As used herein, reference to "high" or "strong" reactivity with an allergenic epitope-containing peptide means a S/N of greater than about 10.

Previously known assays for CMA based on analysis of peptide epitopes in cow's milk proteins are competitive immunoassays which rely on analysis of the relative affinity of binding of IgE and IgG4 to the epitope. The affinity of antibody binding is believed to be related to whether or not the subject will develop clinical tolerance to cow's milk. In contrast, in one aspect, the present invention is based on an analysis of the presence or absence of AAI binding to each individual peptide in a set of key cow's milk protein epitopes that correlates with a diagnosis of CMA, with the intensity of the allergic response, and with the potential of a patient to either develop tolerance or experience an increased allergic response based on the number of epitopes (i.e., peptides) bound by IgE in the serum of the subject. In a second aspect, the invention is based on analysis of the concentration of AAIs in sera that are reactive with each of the allergenic epitope-containing peptides, which also correlates with the intensity of the allergic response.

One embodiment of the invention relates to a method for diagnosing CMA in a subject comprising providing a plurality of peptides selected from among SEQ ID NOs:1-33, each peptide conjugated to a separately identifiable solid support, contacting each solid support with serum obtained from the subject under conditions sufficient to permit binding of AAI in the serum to the peptide on each solid support to form a peptide-AAI complex, binding an AAI-specific labeling reagent to the peptide-AAI complex, and analyzing binding of the labeling reagent to each peptide-AAI complex to identify peptides recognized by the AAI in the serum of the subject. If, following exposure to cow's milk allergens, at least one peptide is moderately or highly reactive with serum AAI (S/N > 2) and reactivity of one or more of the reactive peptides does not decrease at least 2-fold within about six months, the subject is diagnosed as having CMA.

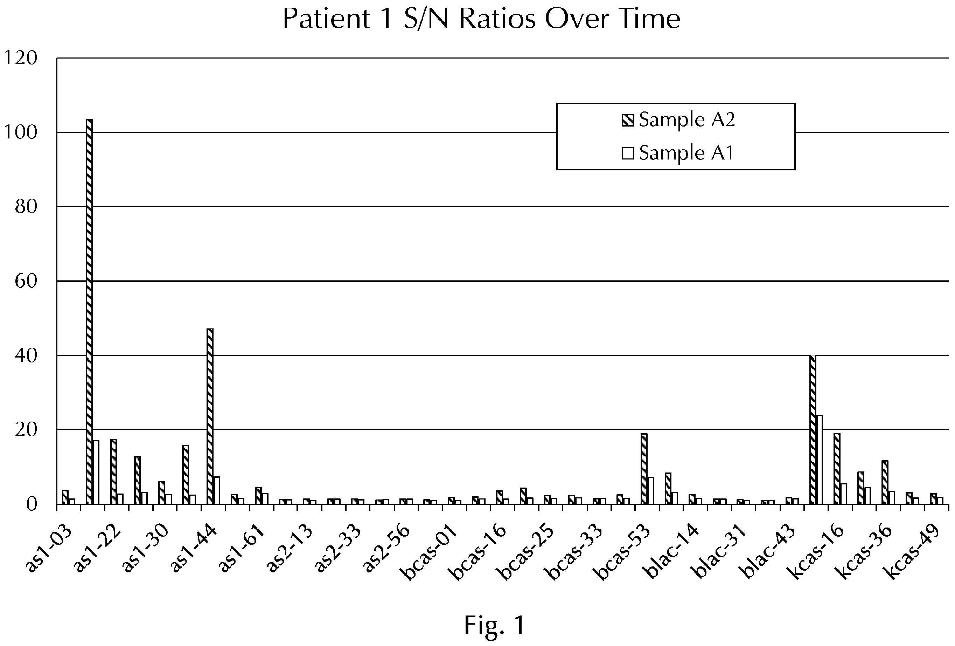

Serum reactivity of a cow's milk tolerant individual following administration of CMA immunotherapy is shown in Fig. 1. In this experiment, a cow's milk tolerant individual was treated with immunotherapy for CMA and serum samples were taken 6-12 months apart. It can be seen that the initial response to immunotherapy resulted in moderate to high reactivity with about eleven of the peptides (blue bars, S/N >2). Within six months (orange bars), there was at least about a 2-fold reduction in reactivity for all of these peptides. Although kcas--04 was in the range of slightly less than a 2-fold reduction in reactivity, the most highly reactive peptides (e.g., as1-09, as1-44) exhibited reductions in reactivity within six months that were substantially larger than 2-fold, in the range of at least 5-fold. In addition, none of the reactive peptides (S/N > 2) failed to diminish in reactivity at the six month time-point.

In another aspect of the method, the analysis of binding of the labeling reagent to each peptide-AAI complex may include analysis of the extent of binding, which indicates a concentration of each peptide-specific AAI in the serum. A low to moderate serum reactivity with all of the peptides of SEQ ID NOs:1-33, or with a subset thereof, indicates a lower concentration of peptide-specific AAI in the serum and mild to moderate CMA, whereas high serum reactivity with all of the peptides, or a subset thereof, indicates a higher concentration of peptide-specific AAI in the serum and more severe CMA. The analysis of binding for diagnosis of CMA may employ either the number of peptides reactive with sera, the extent of binding of serum AAI to the peptides, or both.

In certain aspects, the invention further relates to peptides of cow's milk proteins that contain epitopes that are non-reactive with the sera of subjects that are allergic to cow's milk, even if the subject is phenotypically highly allergic. The sera of non-allergic subjects are also non-reactive with these peptides. These peptides are represented by SEQ ID NOs:34, 35 and 36, and are useful as negative controls in specific embodiments of the assays for diagnosis of CMA. Having a highly reliable negative control available for this purpose reduces the likelihood of false positive diagnoses and falsely high determinations of reactive AAI concentration.

In specific embodiments, of the methods for diagnosing CMA in a subject using a plurality (two or more) of peptides selected from among SEQ ID NOs:1-33 include solid phase assays. The plurality of peptides selected for use in the solid phase assay may represent all 33 peptides of SEQ ID NOs:1-33, a subset of 5-10 peptides, a subset of 10-15 peptides, or a subset of 15-20 peptides. The methods may also employ two or more such subsets of the peptides. Each of the plurality of peptides selected from among SEQ ID NOs:1-33 is provided conjugated to a solid support, which may be a bead, a microtiter plate, a chromatographic material (e.g., a filter), or any other suitable solid support. Each bead, microtiter plate well, or discrete location on the chromatographic material is occupied by a single peptide selected from among SEQ ID NOs:1-33. The solid supports are then contacted with serum obtained from the subject under conditions appropriate for specific binding of anti-peptide AAIE in the serum (if present) to the peptide on each solid support or discrete location on a solid support to form a peptide-AAI complex on the solid support.

Any peptide-AAI complex formed on a solid support is then detected by contacting the complex on each solid support or discrete location on the solid support with a labeling reagent that specifically binds to the complex, typically by binding to the immobilized serum AAI antibody. A single labeling reagent will generally be used for universal detection of all complexes. The specific peptide-AAI complex may then be identified by its position on the microtiter plate or chromatographic support. When the solid support to which each peptide is conjugated has different spectral properties, the specific peptide-AAI complex may also be identified by analysis of the spectral properties of the solid support associated with the peptide-AAI complex, once the presence of a complex is identified via a detectable signal from the labeling reagent bound to the complex. As an example, the presence or absence of a peptide-AAI complex in each well of a microtiter plate can be determined by binding to the complex an anti-human AAI antibody that is conjugated to a reporter moiety, such as a fluorescent dye, a chromogenic dye, an enzyme label or a radioactive label. Alternatively, the anti-human AAI antibody may be conjugated to a reporter moiety that is not directly detectable, so specific binding of a second, directly detectable reporter moiety to the labeling reagent is necessary for analysis of binding.

In certain aspects, the methods for diagnosis of CMA are qualitative methods, i.e., based only on presence or absence of AAI reactive to each selected peptide. Presence of AAI moderately or highly reactive with any selected peptide can be considered to indicate some degree of CMA, provided that the reactivity does not substantially diminish within a short period of time such as about six months. The methods may also be semi-quantitative, i.e., the greater the number of peptides reactive with the serum of the subject the relatively more intense the allergy and, conversely, the fewer the number of reactive peptides the relatively less intense the allergy. Serum reactivity with 5-15 of the peptides of SEQ ID NOs:1-33 may indicate mild to moderate CMA, with reactivity within the lower end of this range generally characterized as mild CMA. Serum reactivity with 16-33, 16-30, 16-25, 16-20, 16-18 or all 33 peptides of SEQ ID NOs:1-33 may indicate moderate to severe CMA, with reactivity within the lower end of this range generally characterized as moderate CMA. In the midrange, serum reactivity with 10-20, 12-18 or 14-16 of the peptides of SEQ ID NOs:1-33 may generally be considered to indicate moderate CMA. It is a particularly useful feature of the peptides of SEQ ID NOs:1-33 that generally no more than about 8-10 are highly reactive (S/N > 10) with the sera of non-allergic individuals and thus provide a higher confidence level in the result of the diagnostic assay than conventional assays.

In other aspects, the methods for diagnosis of CMA are quantitative methods, i.e., based on quantitation of the level of AAI reactivity to each selected peptide. In this example, the level of reactivity correlates with the amount of labeling reagent bound to the peptide-AAI complex, with higher levels of signal from the reporter moiety indicating a higher concentration of a particular peptide-specific AAI in the serum. To obtain the amount or concentration of reporter moiety bound to a particular peptide-AAI complex, the quantity of fluorescence from a fluorescent dye, intensity of color from a colored or chromogenic dye or from an enzyme label, or quantity of radioactivity from a radioactive label is positively correlated with the amount of bound AAI in the complex and therefore its concentration. Methods for measuring these parameters are known in the art. The relative quantities of AAI reactive with any of the peptides can be considered to indicate the degree or intensity of CMA. That is, the higher the level of reactivity of the plurality of selected peptides, or of one or more peptides within the selected peptides, the more intense the allergy. Conversely, the lower the level of reactivity of the plurality of selected peptides, or of one or more peptides within the selected peptides, the less intense the allergy.

A particularly useful quantitative assay for use in any of the methods of the invention is a multiplex peptide-bead assay for flow cytometric analysis, such as the LUMINEX exMAP multiplex bead assay, which is a high-throughput alternative to the ELISA. In this assay, polystyrene beads (microspheres) dyed with distinct proportions of red and near-infrared fluorophores are used as the solid support. The peptides may be chemically linked to the beads or bound thereto through peptide-specific capture antibodies coated on the beads. The proportions of the fluorophores define a "spectral address" for each bead population that can be identified by a flow cytometer using digital signal processing. Detection of a third fluorescence color is used for measurement of the fluorescence intensity of the reporter moiety of the labeling reagent bound to the bead. Multiple analytes can be detected simultaneously by binding each peptide selected from among SEQ ID NOs:1-33 to a bead having a specific "spectral address." Contacting the beads with serum containing AAI that are specific for the peptide bound to it is followed by addition of anti-human AAI antibodies conjugated to a reporter moiety. In one example, the reporter moiety of the anti-human AAI is biotin and binding to phycoerythyrin (PE)-conjugated streptavidin provides the fluorescent signal for detection. Following binding of the labeling reagent, the beads are analyzed on a dual-laser flow-based detection instrument, such as the LUMINEX 200 or Bio-Rad BIO-PLEX analyzer. One laser classifies the bead and identifies the peptide bound to it. The second laser determines the magnitude of the reporter-derived signal, which is in direct proportion to the amount of bound serum AAI.

Because the degree of binding of each peptide-specific AAI to the peptide-AAI complex on the solid support can be quantitated, the plurality of peptides selected from among peptides represented by SEQ ID NOs:1-33 are also useful in methods for detecting an increase in the intensity of CMA over time in a subject diagnosed with CMA or development of CMA over time in a subject initially diagnosed as non-allergic. An initial assay is performed on a plurality of peptides selected from among SEQ ID NOs:1-33 as described above to provide an initial number of reactive peptides or an initial concentration of each peptide-specific AAI. At a time-point subsequent to the initial assay, the analysis is repeated with the same plurality of peptides selected from among SEQ ID NOs:1-33 as the initial profile to obtain a subsequent number of reactive peptides or a subsequent concentration of peptide-specific AAI. This method can be summarized as follows: providing an initial profile of a subject's serum AAI reactivity to a plurality of peptides selected from among SEQ ID NOs:1-33, wherein the initial profile indicates an initial number of peptides recognized (bound) by AAI in the serum of the subject or an initial concentration of AAI in the serum of the subject that recognizes (binds to) each peptide; at a time-point subsequent to the initial profile, contacting each peptide of the same plurality of peptides conjugated to a separately identifiable solid support with serum from the subject under conditions sufficient to permit binding of AAI in the serum to the peptide on each solid support, forming a peptide-AAI complex; binding an AAI-specific labeling reagent to the complex, and; analyzing the binding of the labeling reagent to each peptide-AAI complex to identify a subsequent number of peptides recognized by AAI in the serum of the subject or a subsequent concentration of AAI in the serum of the subject that reacts with each selected peptide.

An alternative assay format useful in the invention is a lateral flow or immunochromatographic assay. In such an assay, the selected allergenic epitope containing peptide(s) are immobilized on the porous support and serum containing the AAI is wicked into contact with the peptide(s) to form immunocomplexes. Further migration of the immunocomplex through the porous support brings it into contact with a specific capture reagent for detection of the immunocomplex using appropriate detection reagents.

The methods for detecting an increase in intensity of the allergy may make use of any appropriate assay format, including those described above. Examples of the types of analyses available for analyzing binding of the labeling reagent are also as described above. An increase in the number of peptides reactive with AAI at the subsequent time-point compared to the initial profile (including an increase compared to no peptides reactive with AAI in the initial profile), or an increase in intensity of binding of AAI to any of the peptides at the subsequent time-point compared to the initial profile (including an increase from no binding to a particular peptide in the initial profile to detectable binding at the subsequent time-point), indicates an increase in the intensity of CMA in a subject previously diagnosed with CMA or development of CMA in the previously non-allergic subject. As discussed above, comparing the initial profile of a subject to that of a subsequent time point may be used to predict the subject's increase in severity or lower tolerance in a particular allergy, or to predict the likelihood of development of clinical or natural tolerance to the allergen.

The plurality of peptides selected from among peptides represented by SEQ ID NOs:1-33 are also useful in methods for detecting development of clinical tolerance to cow's milk proteins in a subject diagnosed with CMA. In these embodiments, the assay generally as described above for detection of an increase in allergy intensity, is performed first at an initial time-point to establish an initial profile of serum AAI reactivity with the plurality of peptides selected from among SEQ ID NOs:1-33. The initial profile is based on semi-quantitative or quantitative analysis of serum reactivity with the selected peptides, as discussed above. The selected peptides conjugated to the solid supports are then contacted with serum from the subject obtained at a time-point subsequent to the initial profile and the assay is conducted as above with semi-quantitation or quantitation of the intensity of CMA at the subsequent time-point. A reduction in the number of peptides reactive with AAI at the subsequent time-point as compared to the initial profile, or a reduction in intensity of binding of AAI to any of the peptides at the subsequent time-point as compared to the initial profile, particularly at least a 2-fold reduction, indicates development of clinical tolerance to cow's milk proteins. It will be appreciated that development of clinical tolerance to cow's milk proteins in a subject previously diagnosed with CMA also indicates a decrease in allergy intensity over the time period between the initial profile and the subsequent time-point, and that the method can also be used to detect and predict such decreases in allergy intensity over time.

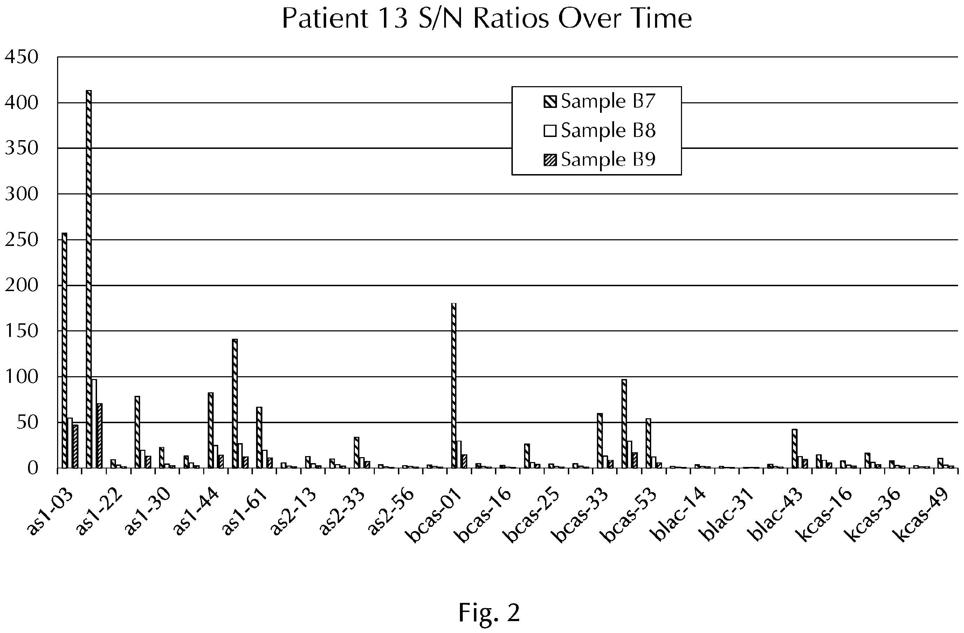

As an example, serum reactivity of a CMA allergic individual is shown in Fig. 2. In this experiment, an individual allergic to cow's milk was treated with immunotherapy for CMA and serum samples were taken 6 months and 12 months later. It can be seen that the initial response to immunotherapy involved moderate to high reactivity with about 15-17 peptides (i.e., S/N >2, blue bars). At six months, serum reactivity had diminished greater than 2-fold for all of the most reactive peptides (orange bars). Reduction in reactivity for certain peptides was in the range of 4-fold to 7-fold (e.g., as1-03, as1-09, as1-57, as1-44, bcas-01). Little or no further reduction in reactivity was observed at 12 months (gray bars), and none of the initially most reactive peptides returned to non-reactive levels (S/N < 2). This individual became desensitized to cow's milk over the course of immunotherapy, showing that the assay successfully detected development of clinical tolerance to cow's milk proteins in a subject diagnosed with CMA

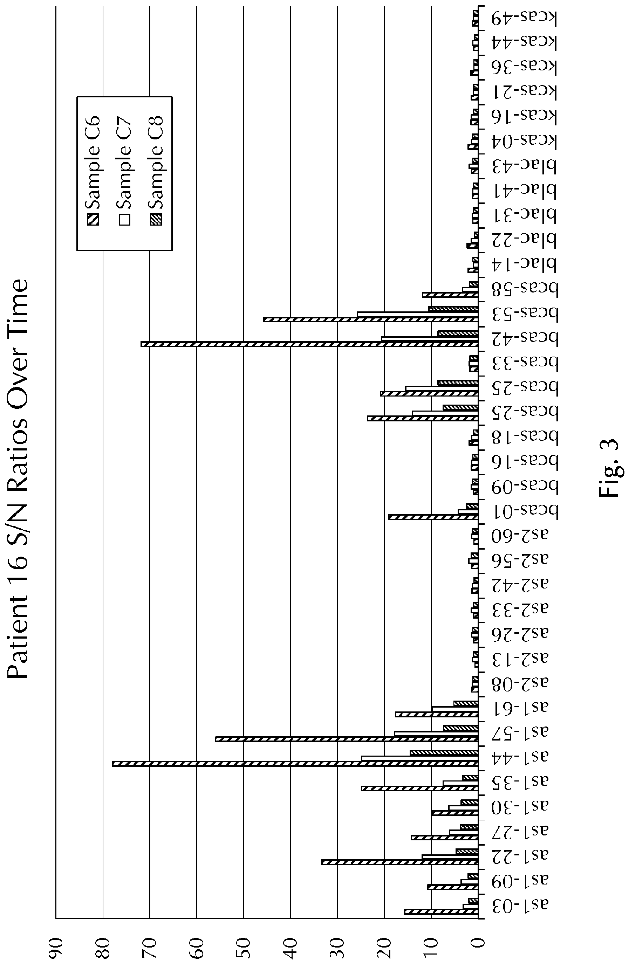

Several peptides in the panel were highly reactive with the sera of the individual shown in Fig. 2 (S/N > 10). Similarly, sera of the individual tested in Fig. 3 were moderately to highly reactive with at least about 15 peptides, indicating allergy to cow's milk. In contrast, however, at six months (orange bars) fewer than all of the initially most reactive peptides exhibited a reduction in reactivity of at least 2-fold. Examples of minimal reduction in reactivity < 2-fold are seen, for example, with as1-61, bcas-25, and bcas-53. The individual shown in Fig. 3 only partially responded to immunotherapy and, although there were greater than 2-fold reductions in reactivity with some of the most highly reactive peptides, the finding that some of the highly reactive peptides did not exhibit a similar reduction in reactivity may be an indication that immunotherapy is less likely to result in complete desensitization or that complete desensitization may require a longer treatment with immunotherapy.

It will also be recognized that analysis of all thirty-three of the peptides represented by SEQ ID NOs:1-33 is not always necessary to obtain useful results in the foregoing methods of the invention. It is only necessary to employ a sufficient number of peptides selected from among the peptides represented by SEQ ID NOs:1-33 to provide a statistically reliable result. For example, if the CMA status of a subject is not known, it is generally desirable to analyze a greater number of allergenic epitope-containing peptides selected from among the peptides represented by SEQ ID NOs:1-33 to ensure that mild to moderate CMA, that may involve reactivity with only a few of the peptides represented by SEQ ID NOs:1-33, is detectable. Conversely, if a subject is known to have high-intensity CMA, fewer allergenic epitope-containing peptides selected from among the peptides represented by SEQ ID NOs:1-33 may be sufficient to detect changes in allergy intensity or development of clinical tolerance, because a larger number of the peptides represented by SEQ ID NOs:1-33 will be initially reactive. However, because changes in allergy intensity and development of clinical tolerance are evidenced by changes in the number of peptides reactive with sera as well as changes in concentration of serum IgE reactive with a particular peptide, it is particularly desirable to include in the assays a large enough set of peptides selected from among the peptides represented by SEQ ID NOs:1-33 to ensure that changes with respect to a peptide that is diagnostic for a particular subject are not missed. Accordingly, the plurality of allergenic epitope-containing peptides selected from among peptides represented by SEQ ID NOs:1-33 for use in any of the foregoing methods may represent all 33 peptides of SEQ ID NOs:1-33, a subset of 20-25 peptides, a subset of 15-20 peptides, a subset of 10-15 peptides, a subset of 5-10 peptides or a subset of 2-5 peptides. By way of example, it has been found that in many cases the betalac peptides (SEQ ID NOs:24-27) are substantially less reactive, or non-reactive, with sera of allergic individuals. Accordingly, it may be desirable to use the SEQ ID NOs:1-23 (the alphaS1, alphaS2, and betacas peptides) alone or with SEQ ID NOs:28-33 (the kappacas peptides) for certain applications. Each of these subgroups may also be used alone in the invention if desired.

For the convenience of the user, the reagents for use in any of the foregoing methods may be packaged together in the form of a kit comprising a plurality of allergenic epitope-containing peptides selected from among the peptides represented by SEQ ID NOs:1-33 or any of the useful subgroups, a labeling reagent comprising an anti-human IgE antibody conjugated to a first reporter moiety and, optionally (if required for indirect detection) a second reporter moiety that specifically binds to the labeling reagent. The kit will typically include instructions for use of these reagents in one or more of the methods of the invention described above.

In certain kit embodiments, as well as in the methods of the invention, the anti-human AAI antibody may be provided conjugated to a reporter moiety that can be directly detected. Directly detectable reporter moieties are those that can be identified and/or quantitated without the need for binding to a specific binding partner. Examples of directly-detectable reporter moieties that may be conjugated to the anti-human AAI antibody include fluorescent dyes, colored dyes, chromogenic dyes and enzyme labels that can be detected by a subsequent chemical reaction, and radiolabels. In other kit embodiments, as in the methods of the invention, the anti-human AAI antibody may be provided conjugated to a reporter moiety that is indirectly detectable, i.e., a reporter moiety that is not itself detectable but which undergoes a reaction or interaction with a second reporter moiety that comprises a directly detectable reporter moiety, such as a specific binding partner for the reporter moiety conjugated to a directly detectable label. Examples of indirectly-detectable reporter moieties include biotin, digoxigenin, and other haptens that are detectable upon subsequent binding of a secondary antibody (e.g., anti-digoxigenin) or other binding partner (e.g., streptavidin) which is labeled for direct detection. It will be understood that any of these labeling reagents and reporter moieties are useful in the appropriate assay format in the foregoing methods of the invention and as components of the kits. In a specific example of a kit for performing the flow cytometry multiplex assay described above, the components of the kit may comprise a plurality of allergenic epitope-containing peptides selected from among the peptides represented by SEQ ID NOs:1-33, a biotinylated anti-human AAI antibody (labeling reagent with first reporter moiety), and streptavidin conjugated to PE (second reporter moiety).

The plurality of allergenic epitope-containing peptides selected from among SEQ ID NOs:1-33 for inclusion in any of the foregoing kits may represent all 33 peptides of SEQ ID NOs:1-33, a subset of 20-25 peptides, a subset of 15-20 peptides, a subset of 10-15 peptides, a subset of 5-10 peptides or a subset of 2-5 peptides. The plurality of allergenic epitope-containing peptides selected from among SEQ ID NOs:1-33 for inclusion in any of the foregoing kits may also represent one or more of the related peptides subgroups (i.e., alphaS1, alphaS2, betacas, betalac and kappacas peptides)

### EXAMPLES

Sera were obtained from CM tolerant and CMA individuals and assayed in the LUMINEX assay to obtain the representative results shown in Figs. 1-3. Wash buffer, sera, beads and antibody dilutions were prepared according to the manufacturer's directions. The filter plate was pre-wet with buffer for 1 min. and the buffer was removed by vacuum. 100 µl of the bead cocktail was added to each well, the buffer was removed by vacuum, and the beads were washed twice with 100µl buffer. 100 µl of sera dilution was added to each well and incubated for 2 hrs. with shaking. Vacuum was applied to remove liquid. Beads were again washed twice with buffer. 50 µl of the antibody dilution was applied to each well and incubated for 30 min. with shaking. After application of vacuum the well was washed three times. 100 µl of buffer was added to the wells and the samples were transferred to a fixed plate. The wells were read on the LUMINEX instrument. Results are shown in Figs. 1-3 and discussed above.

### Additional Applications of the Methods

The concepts of the invention with respect to allergenic epitope-containing peptides derived from cow's milk and their use for diagnosis of CMA, for detecting development of clinical tolerance to cow's milk proteins, and for detecting increases and decreases in the intensity of the allergy can also be applied to development of other allergenic epitope-containing peptide panels and their use in diagnosing, detecting tolerance, and detecting increases and development of tolerance to other allergenic proteins.

In a further aspect, the application provides a method for detecting an increase in intensity of allergy to cow's milk in a subject over time, the method comprising:
a) providing an initial profile of the subject's serum IgE reactivity to a plurality of peptides derived from one or more allergenic proteins found in the food, wherein the initial profile defines an initial number of peptides recognized by IgE in the serum of the subject or an initial concentration of IgE in the serum of the subject that recognizes each peptide;
b) providing the plurality of peptides each conjugated to a separately identifiable solid support
c) contacting each solid support with serum obtained from the subject at a time-point subsequent to the initial profile under conditions sufficient to permit binding of IgE in the serum to the peptide on each solid support to form a peptide-IgE complex;
d) binding an IgE-specific labeling reagent to the peptide-IgE complex; and
e) analyzing the binding of the labeling reagent to each peptide-IgE complex to identify a subsequent number of peptides recognized by IgE in the serum of the subject or a subsequent concentration of IgE in the serum of the subject that recognizes each peptide;
wherein an increase in the subsequent number of peptides recognized by IgE in the serum of the subject compared to the initial number of peptides recognized by IgE in the serum of the subject, or an increase in the subsequent concentration of IgE in the serum of the subject that recognizes at least one peptide compared to the initial concentration of IgE in the serum of the subject that recognizes the at least one peptide, indicates increased intensity in the subject of the allergic response to the food.

The reagents and materials used in any of the foregoing methods may be packaged in the form of a kit in which the plurality of allergenic epitope-containing peptides, a labeling reagent comprising an anti-IgE antibody conjugated to a first reporter moiety and, optionally, a second reporter moiety that specifically binds to the labeling reagent are packaged together.

Although the invention herein has been described with reference to particular embodiments, it is to be understood that these embodiments are merely illustrative of the principles and applications of the present invention. Thus, it is intended that the present invention include modifications and variations that are within the scope of the appended claims.

## Claims

1. A method for diagnosing a milk allergy in a subject comprising:
a) providing a plurality of peptides selected from among SEQ ID NOs:1-33, wherein the plurality of peptides consists of all 33 peptides of SEQ ID NOs:1-33, a subset of 20-25 peptides, a subset of 15-20 peptides, a subset of 10-15 peptides, a subset of 5-10 peptides, or a subset of 2-5 peptides, each peptide conjugated to a separately identifiable solid support;
b) contacting each solid support with serum obtained from the subject under conditions sufficient to permit binding of allergy associated immunoglobulin (AAI) in the serum to the peptide on each solid support to form a peptide-IgE complex;
c) binding an AAI-specific labeling reagent to the peptide-AAI complex; and
d) analyzing binding of the labeling reagent to each peptide-AAI complex to identify peptides recognized by the AAI in the serum of the subject;
wherein recognition of at least one peptide by AAI in the serum of the subject indicates that the subject is allergic to milk.

2. The method of claim 1 wherein IgG and IgE are detected.

3. The method of claim 2 wherein the IgG is IgG4.

4. The method of claim 1, wherein the plurality of peptides is represented by SEQ ID NOs:1-9, SEQ ID NOs:10-15, SEQ ID NOs:16-23, SEQ ID NOs:24-27, SEQ ID NOs:28-33, and combinations thereof.

5. A method for detecting development of clinical tolerance or an increase in intensity of allergy to milk in a subject that is allergic to milk comprising:
a) providing an initial profile of allergy associated immunoglobulin (AAI) reactivity of the subject's serum to a plurality of peptides selected from among SEQ ID NOs:1-33, wherein the plurality of peptides consists of all 33 peptides of SEQ ID NOs:1-33, a subset of 20-25 peptides, a subset of 15-20 peptides, a subset of 10-15 peptides, a subset of 5-10 peptides, or a subset of 2-5 peptides, wherein the initial profile defines an initial number of peptides recognized by AAI in the serum of the subject or an initial concentration of AAI in the serum of the subject that recognizes each peptide;
b) providing the plurality of peptides each conjugated to a separately identifiable solid support;
c) contacting each solid support with serum obtained from the subject at a time-point subsequent to the initial profile under conditions sufficient to permit binding of AAI in the serum to the peptide on each solid support to form a peptide-AAI complex;
d) binding an AAI-specific labeling reagent to the peptide-AAI complex; and
e) analyzing binding of the labeling reagent to each peptide-AAI complex to identify a subsequent number of peptides recognized by AAI in the serum of the subject or a subsequent concentration of AAI in the serum of the subject that recognizes each peptide;
wherein development of clinical tolerance to milk is indicated when the subsequent number of peptides recognized by AAI in the serum of the subject is less than the initial number of peptides recognized by AAI in the serum of the subject, or when the subsequent concentration of AAI in the serum of the subject that recognizes at least one peptide is less than the initial concentration of AAI in the serum of the subject that recognizes the at least one peptide, and
wherein an increased intensity of the allergic response is indicated when the subsequent number of peptides recognized by AAI in the serum of the subject is greater than the initial number of peptides recognized by AAI in the serum of the subject, or when the subsequent concentration of AAI in the serum of the subject that recognizes at least one peptide is greater than the initial concentration of AAI in the serum of the subject that recognizes the at least one peptide.

6. The method of claim 5, wherein a pattern of quantitative reduction in AAI reactivity with selected peptides is used to predict development of clinical tolerance to the allergenic milk in the subject or a pattern of quantitatively increased AAI reactivity with selected peptides is used to predict increasing intensity of allergy to milk over time.

7. A set of allergenic epitope-containing peptides for detection of milk allergy consisting of a plurality of peptides represented by SEQ ID NOs:1-33, wherein the plurality of peptides consists of all 33 peptides of SEQ ID NOs:1-33, a subset of 15-20 peptides, a subset of 10-15 peptides, or a subset of 5-10 peptides.

## Patentansprüche

1. Verfahren zum Diagnostizieren einer Milchallergie bei einem Subjekt, umfassend:
a) Bereitstellen einer Vielzahl von Peptiden, die aus SEQ ID NOs:1-33 ausgewählt sind, wobei die Vielzahl von Peptiden aus allen 33 Peptiden der SEQ ID NOs:1-33, einer Untergruppe von 20-25 Peptiden, einer Untergruppe von 15-20 Peptiden, einer Untergruppe von 10-15 Peptiden, einer Untergruppe von 5-10 Peptiden oder einer Untergruppe von 2-5 Peptiden besteht, wobei jedes Peptid an einen separat identifizierbaren festen Träger konjugiert ist;
b) Inkontaktbringen jedes festen Trägers mit Serum, das von dem Subjekt erhalten wurde, unter Bedingungen, die ausreichen, um ein Binden von allergieassoziiertem Immunglobulin (AAI) in dem Serum an das Peptid auf jedem festen Träger zu ermöglichen, um einen Peptid-IgE-Komplex zu bilden;
c) Binden eines AAI-spezifischen Markierungsreagens an den Peptid-AAI-Komplex; und
d) Analysieren des Bindens des Markierungsreagens an jeden Peptid-AAI-Komplex, um Peptide, die von AAI in dem Serum des Subjekts erkannt wurden, zu identifizieren;
wobei eine Erkennung von mindestens einem Peptid durch AAI in dem Serum des Subjekts anzeigt, dass das Subjekt auf Milch allergisch ist.

2. Verfahren nach Anspruch 1, wobei IgG und IgE nachgewiesen werden.

3. Verfahren nach Anspruch 2, wobei das IgG IgG4 ist.

4. Verfahren nach Anspruch 1, wobei die Vielzahl von Peptiden durch SEQ ID NOs:1-9, SEQ ID NOs:10-15, SEQ ID NOs:16-23, SEQ ID NOs:24-27, SEQ ID NOs:28-33 und Kombinationen davon dargestellt wird.

5. Verfahren zum Nachweisen einer Entwicklung einer klinischen Toleranz oder einer Zunahme der Intensität einer Milchallergie bei einem Subjekt, das auf Milch allergisch ist, umfassend:
a) Bereitstellen eines anfänglichen Profils einer allergieassoziierten Immunglobulin(AAI)-Reaktivität des Serums des Subjekts gegenüber einer Vielzahl von Peptiden, die aus SEQ ID NOs:1-33 ausgewählt sind, wobei die Vielzahl von Peptiden aus allen 33 Peptiden der SEQ ID NOs:1-33, einer Untergruppe von 20-25 Peptiden, einer Untergruppe von 15-20 Peptiden, einer Untergruppe von 10-15 Peptiden, einer Untergruppe von 5-10 Peptiden oder einer Untergruppe von 2-5 Peptiden besteht, wobei das anfängliche Profil eine anfängliche Anzahl von Peptiden, die von AAI in dem Serum des Subjekts erkannt werden, oder eine anfängliche Konzentration von AAI in dem Serum des Subjekts definiert, die jedes Peptid erkennt;
b) Bereitstellen der Vielzahl von Peptiden, die jeweils an einen separat identifizierbaren festen Träger konjugiert sind;
c) Inkontaktbringen jedes festen Trägers mit Serum, das von dem Subjekt zu einem Zeitpunkt nach dem anfänglichen Profil erhalten wurde, unter Bedingungen, die ausreichen, um ein Binden von AAI in dem Serum an das Peptid auf jedem festen Träger zu ermöglichen, um einen Peptid-AAI-Komplex zu bilden;
d) Binden eines AAI-spezifischen Markierungsreagens an den Peptid-AAI-Komplex; und
e) Analysieren des Bindens des Markierungsreagens an jeden Peptid-AAI-Komplex, um eine nachfolgende Anzahl von Peptiden, die von AAI in dem Serum des Subjekts erkannt wurden, oder eine nachfolgende Konzentration von AAI in dem Serum des Subjekts, die jedes Peptid erkennt, zu identifizieren;
wobei eine Entwicklung einer klinischen Toleranz gegenüber Milch angezeigt ist, wenn die nachfolgende Anzahl von Peptiden, die von AAI in dem Serum des Subjekts erkannt wird, niedriger als die anfängliche Anzahl von Peptiden ist, die von AAI in dem Serum des Subjekts erkannt wurde, oder wenn die nachfolgende Konzentration von AAI in dem Serum des Subjekts, die mindestens ein Peptid erkennt, niedriger als die anfängliche Konzentration von AAI in dem Serum des Subjekts ist, die das mindestens eine Peptid erkennt, und
wobei eine erhöhte Intensität der allergischen Reaktion angezeigt ist, wenn die nachfolgende Anzahl von Peptiden, die von AAI in dem Serum des Subjekts erkannt wird, höher als die anfängliche Anzahl von Peptiden ist, die von AAI in dem Serum des Subjekts erkannt wurde, oder wenn die nachfolgende Konzentration von AAI in dem Serum des Subjekts, die mindestens ein Peptid erkennt, höher als die anfängliche Konzentration von AAI in dem Serum des Subjekts ist, die das mindestens eine Peptid erkennt.

6. Verfahren nach Anspruch 5, wobei ein Muster einer quantitativen Reduzierung der AAI-Reaktivität mit ausgewählten Peptiden verwendet wird, um eine Entwicklung einer klinischen Toleranz gegenüber der allergenen Milch bei dem Subjekt vorherzusagen, oder ein Muster einer quantitativ erhöhten AAI-Reaktivität mit ausgewählten Peptiden verwendet wird, um eine zunehmende Intensität einer Milchallergie im Laufe der Zeit vorherzusagen.

7. Satz allergene Epitope enthaltender Peptide zum Nachweis einer Milchallergie, bestehend aus einer Vielzahl von Peptiden, dargestellt durch SEQ ID NOs:1-33, wobei die Vielzahl von Peptiden aus allen 33 Peptiden der SEQ ID NOs:1-33, einer Untergruppe von 15-20 Peptiden, einer Untergruppe von 10-15 Peptiden oder einer Untergruppe von 5-10 Peptiden besteht.

## Revendications

1. Procédé de diagnostic de l'allergie au lait chez un sujet comprenant :
a) la fourniture d'une pluralité de peptides choisis parmi les SEQ ID NO : 1-33, dans lequel la pluralité de peptides consiste en l'ensemble des 33 peptides des SEQ ID NO : 1-33, un sous-ensemble de 20-25 peptides, un sous-ensemble de 15-20 peptides, un sous-ensemble de 10-15 peptides, un sous-ensemble de 5-10 peptides, ou un sous-ensemble de 2-5 peptides, chaque peptide étant conjugué à un support solide identifiable séparément ;
b) la mise en contact de chaque support solide avec du sérum obtenu à partir du sujet dans des conditions suffisantes pour permettre la liaison de l'immunoglobuline associée à l'allergie (AAI) dans le sérum au peptide sur chaque support solide pour former un complexe peptide-IgE ;
c) la liaison d'un réactif de marquage spécifique de l'AAI au complexe peptide-AAI ; et
d) l'analyse de la liaison du réactif de marquage à chaque complexe peptide-AAI pour identifier les peptides reconnus par l'AAI dans le sérum du sujet ;
dans lequel la reconnaissance d'au moins un peptide par l'AAI dans le sérum du sujet indique que le sujet est allergique au lait.

2. Procédé selon la revendication 1, dans lequel les IgG et les IgE sont détectés.

3. Procédé selon la revendication 2, dans lequel l'IgG est l'IgG4.

4. Procédé selon la revendication 1, dans lequel la pluralité de peptides est représentée par les SEQ ID NO : 1-9, SEQ ID NO : 10-15, SEQ ID NO : 16-23, SEQ ID NO : 24-27, SEQ ID NO : 28-33, et leurs combinaisons.

5. Procédé de détection du développement d'une tolérance clinique ou d'une augmentation de l'intensité de l'allergie au lait chez un sujet qui est allergique au lait, comprenant :
a) la fourniture d'un profil initial de réactivité des immunoglobulines associées à l'allergie (AAI) du sérum du sujet à une pluralité de peptides choisis parmi les SEQ ID NO : 1-33, dans lequel la pluralité de peptides consiste en l'ensemble des 33 peptides des SEQ ID NO : 1-33, un sous-ensemble de 20-25 peptides, un sous-ensemble de 15-20 peptides, un sous-ensemble de 10-15 peptides, un sous-ensemble de 5-10 peptides, ou un sous-ensemble de 2-5 peptides, dans lequel le profil initial définit un nombre initial de peptides reconnus par l'AAI dans le sérum du sujet ou une concentration initiale d'AAI dans le sérum du sujet qui reconnaît chaque peptide ;
b) la fourniture d'une pluralité de peptides, chacun étant conjugué à un support solide identifiable séparément ;
c) la mise en contact de chaque support solide avec le sérum obtenu du sujet à un moment ultérieur au profil initial dans des conditions suffisantes pour permettre la liaison de l'AAI dans le sérum au peptide sur chaque support solide pour former un complexe peptide-AAI ;
d) la liaison d'un réactif de marquage spécifique de l'AAI au complexe peptide-AAI ; et
e) l'analyse de la liaison du réactif de marquage à chaque complexe peptide-AAI pour identifier un nombre ultérieur de peptides reconnus par l'AAI dans le sérum du sujet ou une concentration ultérieure d'AAI dans le sérum du sujet qui reconnaît chaque peptide ;
dans lequel le développement d'une tolérance clinique au lait est indiqué lorsque le nombre ultérieur de peptides reconnus par l'AAI dans le sérum du sujet est inférieur au nombre initial de peptides reconnus par l'AAI dans le sérum du sujet, ou lorsque la concentration ultérieure d'AAI dans le sérum du sujet qui reconnaît au moins un peptide est inférieure à la concentration initiale d'AAI dans le sérum du sujet qui reconnaît l'au moins un peptide, et
dans lequel une intensité accrue de la réponse allergique est indiquée lorsque le nombre ultérieur de peptides reconnus par l'AAI dans le sérum du sujet est supérieur au nombre initial de peptides reconnus par l'AAI dans le sérum du sujet, ou lorsque la concentration ultérieure d'AAI dans le sérum du sujet qui reconnaît au moins un peptide est supérieure à la concentration initiale d'AAI dans le sérum du sujet qui reconnaît l'au moins un peptide.

6. Procédé selon la revendication 5, dans lequel un modèle de réduction quantitative de la réactivité de l'AAI avec des peptides sélectionnés est utilisé pour prédire le développement d'une tolérance clinique au lait allergène chez le sujet ou un modèle de réactivité de l'AAI quantitativement accrue avec des peptides sélectionnés est utilisé pour prédire l'intensité croissante de l'allergie au lait au fil du temps.

7. Ensemble de peptides contenant des épitopes allergènes pour la détection de l'allergie au lait, constitué d'une pluralité de peptides représentés par les SEQ ID NO : 1 à 33, dans lequel la pluralité de peptides consiste en la totalité des 33 peptides des SEQ ID NO : 1 à 33, un sous-ensemble de 15 à 20 peptides, un sous-ensemble de 10 à 15 peptides, ou un sous-ensemble de 5 à 10 peptides.
